# EUROPEAN PATENT APPLICATION

(11) **EP 3 187 047 A1**
(43) Date of publication of application: **05.07.2017**
(21) Application number: 16001875.0
(22) Date of filing: 26.08.2016
(51) Int. Cl.: A01N 25/34, A01N 25/24, A01N 47/44, A01N 59/16, A01N 33/12, D06M 11/13, A61L 2/16, D06M 13/256, D06M 13/352, D06M 16/00, A62B 23/02, A41D 13/11, A61F 13/49, A61F 13/56, A61F 13/84, A61F 13/47, A61F 13/472, A61F 13/00

(54) **WASH-DURABLE, ABSORBENT TEXTILE WITH ANTIMICROBIAL PROPERTIES AND/OR IMPROVED WASHABILITY, IN PARTICULAR FOR REUSABLE SANITARY NAPKIN**

(30) Priority: 30.12.2015 EP 15203186; 29.02.2016 WO PCT/EP2016/054245
(71) Applicant: Green Impact Holding AG, 6330 Cham/Zug (CH)
(72) Inventor: Swamy, Sanjeev, 6442 Gersau (CH); Kurien, Ashok, 400006 Mumbai (IN)
(74) Representative: Karl, Christof

(57) **Abstract**

FIELD OF THE INVENTION

The present invention is directed to a textile material to which one or more antimicrobial and/or hydrophilic and/or stain release agents are adhered. The agent(s) is/are adhered to the textile material in such a manner that they are not released from the textile even if the textile is wetted or washed, so that the textile is reusable. Washability and/or usability of the textile are improved where one or more hydrophilic and/or stain release agents are adhered to the textile. The textile material can be used, e.g., in a reusable sanitary napkin or other hygiene product. The structure of the sanitary napkin is disclosed, together with a process of attaching the different layers of the sanitary napkin together by ultrasonic welding. The invention further relates to a method of finishing a textile material by applying and binding antimicrobial and/or hydrophilic and/or stain release agents to the textile material so that the agents are essentially irreversibly adhered to the finished textile material.

## Description

### FIELD OF THE INVENTION

The present invention is directed to a textile material to which one or more antimicrobial and/or hydrophilic and/or stain release agents are adhered. The agent(s) is/are adhered to the textile material in such a manner that they are not released from the textile even if the textile is wetted or washed, so that the textile is reusable. Washability and/or usability of the textile are improved where one or more hydrophilic and/or stain release agents are adhered to the textile. The textile material can be used, e.g., in a reusable sanitary napkin or other hygiene product. The invention further relates to a method of finishing a textile material by applying and binding antimicrobial and/or hydrophilic and/or stain release agents to the textile material so that the agents are essentially irreversibly adhered to the finished textile material.

### BACKGROUND OF THE INVENTION

Disposable sanitary napkins are well-known and often used. However, they produce great amounts of waste. Furthermore, the price of disposable sanitary napkins at a minimum of $ 3.50/month is too high in particular for women in developing countries. For example, in 2015, two thirds of Indian women (about 400 million) could not afford them. Instead of sanitary napkins, they oftentimes use pieces of old cotton clothes which are folded into a pad. However, use of such alternative sanitary care measures make women susceptible to yeast and fungal infections, and they also run a higher risk of cervical cancer, reproductive tract and urinary tract diseases. It is desirable to overcome the waste problem and further to provide products for female hygiene, e.g. sanitary napkins, which prevent microbiological contamination.

Reusable sanitary napkins or sanitary towels which have some disinfecting or antibacterial properties are known in the art. For example, it is known to make sanitary napkins antibacterial by using tea (CN 201283041 Y), tetraammonium salt (CN 106559 A), or hexachlorophene (US 3,732,867 A). EP 2 829 256 A1 relates to a sanitary napkin comprising a contact layer (to the skin) being permeable to fluid passage, a pad for absorbing fluids, and an outer layer being impermeable to fluid passage. The pad comprises a percentage of antimicrobial fibers made from polymer material with silver particles added. However, all these solutions have disadvantages in that either the antimicrobial efficacy is low and/or the textiles are expensive to manufacture because of the ingredients used and/or because the ingredients are applied to the fiber or the yarn, not to the fabric. Furthermore, none of the prior art reusable napkins addresses the problem of staining in an efficient manner, so that in general a full wash cycle in a laundering machine is required before they can be reused.

From US 3,489,149, A, reusable sanitary napkins built into underwear are known, which use stain resistant and washable textiles. However, the stain resistant textile is said to be essentially hydrophobic, and to have no great ability to retain moisture, which is highly disadvantageous because a sanitary napkin should absorb fluids as quickly as possible, and be able to retain as much fluid as possible. Reusable water-absorbing pads e.g. for diapers are known from US 2001/0034510 A1. However, for washing them in an efficient manner, an aqueous electrolytic solution (water and salts, e.g. magnesium hydroxide, sodium hypochlorite, and detergent) needs to be used. Furthermore, none of these products exhibits antimicrobial activity.

Textiles used in hospitals or kitchens have a high exposure to staining materials like blood or food. It is important that they are not contaminated by microbes when they are worn or otherwise used. Therefore, these textiles are typically laundered in long wash cycles using large amounts of resources such as energy, water, and detergent. Water or oil repellent textile finishes can help to avoid staining, but some of these textiles, like kitchen towels or bed pads need to have good fluid absorbing capabilities, and therefor they should not be water or oil repellent.

For all of the above-mentioned applications, it would be desirable to have wash-durable textiles with antimicrobial properties, which are easily washable and/or absorb water and oily substances well.

### SUMMARY OF THE INVENTION

It is an object of the invention to provide textile materials that overcome some or all problems of the above-mentioned prior art documents. In particular, one object of the invention is to provide textile materials textile materials exhibiting antibacterial properties even after numerous washes. Furthermore, it is an object of the invention that the textile materials can prohibit growth of bacteria, smells, odors etc. as completely as possible. It is another object that the textile materials are easily washable. It is a further object of the invention that the textile materials have fluid absorbing properties. It is another object that the textile materials exhibit properties rendering them suitable as hygiene products, e.g. as sanitary napkins.

Some or all of these objects are solved by the subject matter of the independent claims. Preferred embodiments are subject of the dependent claims.

In accordance with the present invention, a textile material is provided to which one or more chemical agents are adhered, which chemical agents convey antimicrobial and/or hydrophilic and/or stain release properties to the textile material, in a wash-durable manner. The textile material can be used for hygiene products, e.g. for a sanitary napkin. The invention further provides finishing methods for manufacturing the textile materials, by applying liquor application processes such as padding and exhaust processes, followed by heat treatment.

Unless otherwise stated, all percentages hereinafter refer to the ratio of the uptaken weight of antimicrobial agent on a textile and the weight of that fabric without the uptaken antimicrobial agent. The term "on weight fabric" refers to this ratio. Abbreviations are "owf" or "o.w.f". The term "gpl" means "grams per liter" and is typically used to define the concentration of a substance in a liquor.

In the context of the present invention the terms "textile" and "textile material" relate to a flexible material consisting of fibres, or a network of natural and/or artificial fibres, such as a yarn or a fabric. The material may be in its natural or processed or even finished form. The term "starting textile material" refers to a textile material which has not yet been treated by the finishing processes described in the present disclosure.

The term "antimicrobial" as used in the context of the present invention relates to the ability to kill at least some types of microorganisms, or to inhibit the growth or reproduction of at least some types of microorganisms. Said term relates to any compound, agent, product or process that is harmful to one or more "microorganism" as used in the context of the present invention. Preferably, the one or more "microorganisms" get killed by the "antimicrobial" product or process. By "antimicrobial agent" is meant any substance or combination of substances that kills or prevents the growth of a microorganism. The terms "microorganism" and "microbe", which are used interchangeably in the context of the present invention, are defined to comprise any organism too small to be seen by the unaided eye, such as, especially, single-celled organisms. In particular, the terms "microorganism" and "microbe" cover prokaryotes including bacteria and archaea, eukaryotes including protists, animals like dust mites or spider mites, fungi, and plants like green algae, as well as viruses.

The term "hydrophilic" essentially relates to the attraction of water. In the context of the present invention, the term "hydrophilic" commonly implies attachment or adhering of aqueous liquids at the surface and in the interior of a textile material. Thus, the term "hydrophilic" or "hydrophilic properties" as used herein includes the attachment or adhering of aqueous liquids at or near the surface of a textile material and/or in the interior of a textile material. When the term "hydrophilic" or "hydrophilic properties" is used herein, it means the attachment or adhering of liquid at or near the surface of a textile material. A hydrophilic textile is typically not water-repellent and to the contrary absorbs water quickly. It typically also exhibits good wicking properties. A "hydrophilic agent" is an agent which when applied to a substrate like a textile material, noticeably improves the hydrophilic properties of the substrate.

The term "stain release" relates to easy removal of stains. In the context of the present invention, the term "stain release properties" of a textile commonly refers to its ability of both adsorbing in particular oily stains on the outside surface or on internal surfaces within a textile material and easily removing stains from these surfaces, for instance during laundry. The term "stain release properties" is not to be confused with the term "stain repellent properties" of a textile. Whereas textiles with good stain repellent properties do not adsorb or absorb oily stains in the first place, textiles with good stain release properties may adsorb them but subsequently release them easily. A "stain release agent" is an agent which, when applied to a substrate like a textile material, noticeably improves the stain release properties of that substrate.

Whenever a temperature is mentioned in the present specification, the temperature refers to a temperature applied at normal pressure (101.325 Pa). If in an implementation of the invention higher or lower pressure is applied, the temperatures are understood to be adapted accordingly.

According to a 1st embodiment, the invention is a textile material to which one or more stain release agents and/or one or more hydrophilic agents and/or one or more antimicrobial agents are adhered.

According to a 2nd embodiment, the invention is a textile material to which one or more stain release agents and one or more hydrophilic agents and preferably one or more antimicrobial agents are adhered

According to a 3rd embodiment, the invention is a textile material to which one or more stain release agents and one or more antimicrobial agents and preferably one or more hydrophilic agents are adhered.

According to a 4th embodiment, the invention is a textile material to which one or more antimicrobial agents and one or more hydrophilic agents and preferably one or more stain release agents are adhered.

### Antimicrobial agents

### Types

According to a 5th embodiment, in the textile material of any of the proceeding embodiments, the one or more antimicrobial agents comprise
- at least one, preferably at least two, more preferably at least three, even more preferably at least four, or all five selected from the group consisting of a quaternary ammonium organosilane compound, metal, an azole-based compound, polyglucosamine, and polyhexamethylene biguanide; or
- at least one, preferably at least two, more preferably at least three, and most preferably all four selected from the group consisting of metal, an azole-based compound, polyglucosamine, and polyhexamethylene biguanide; or
- at least one, preferably at least two, more preferably all three selected from the group consisting of metal, an azole-based compound, and a quaternary ammonium organosilane compound; or
- at least metal and at least one selected from the group consisting of an azole-based compound, a quaternary ammonium organosilane compound, polyglucosamine, and polyhexamethylene biguanide; or at least an azole-based compound and at least one selected from the group consisting of a quaternary ammonium organosilane compound, polyglucosamine, and polyhexamethylene biguanide; or at least a quaternary ammonium organosilane compound and at least one selected from the group consisting of polyglucosamine and polyhexamethylene biguanide; or at least polyglucosamine and polyhexamethylene biguanide.

According to a 6^{th} embodiment, in the textile material of any one of embodiments 1 to 4, the one or more antimicrobial agents comprise
- metal and at least one, or at least two, or all three selected from the group consisting of polyhexamethylene biguanide, polyglucosamine, and an azole-based compound; or
- an azole-based compound and at least one, or at least two, or all three selected from the group consisting of polyhexamethylene biguanide, polyglucosamine, and metal; or
- polyhexamethylene biguanide and at least one, or at least two, or all three selected from the group consisting of metal, polyglucosamine, and an azole-based compound; or
- polyglucosamine and at least one, or at least two, or all three selected from the group consisting of silver cations, polyhexamethylene biguanide, and an azole-based compound; or
- quaternary ammonium organosilane and at least one, preferably both selected from the group consisting of metal and an azole-based compound; or
- metal and at least one, preferably both selected from the group consisting of a quaternary ammonium organosilane compound and an azole-based compound; or
- an azole-based compound and at least one, preferably both selected from the group consisting of quaternary ammonium organosilane and metal.

According to a 7^{th} embodiment, In the textile material of any one of the embodiments 5 or 6, the metal is copper, zinc, or preferably silver, more preferably silver cations.

According to a 8^{th} embodiment, in the textile material of any one of the embodiments 5 or 6, the azole-based compound is thiabendazole or a triazole-based compound.

According to a 9^{th} embodiment, in the textile material of embodiment 8, the triazole-based compound is propiconazole.

According to a 10^{th} embodiment, in the textile material of any one of embodiments 1 to 9, the one or more antimicrobial agents are not in the form of nanoparticles.

According to a 11^{th} embodiment, in the textile material of any one of embodiments 1 to 10, the one or more antimicrobial agents have a particle size, in all dimensions (length, width, height), of at least 250 nanometers, preferably at least 500 nanometers, more preferably at least 750 nanometers, and most preferably at least 1,000 nanometers.

According to a 12^{th} embodiment, in the textile material of any one of embodiments 1 to 11, the one or more antimicrobial agents are nonionic or cationic.

According to a 13^{th} embodiment, in the textile material of any one of embodiments 1 to 12, the one or more antimicrobial agents are bound to the textile material either directly, in particular if the agent is a quaternary ammonium organosilane compound, polyglucosamine, or polyhexamethylene biguanide, or by means of an inorganic or organic matrix directly bound to the textile material, in particular if the agent is metal, or via cross linking, in particular if the agent is an azole-based compound.

According to a 14^{th} embodiment in the textile material of any one of embodiments 1 to 13, one or more of the one or more antimicrobial agents are bound to the textile material without the cyclodextrin and/or an inclusion complex, in particular without an inclusion complex of fiber-reactive cyclodextrin derivatives and antimicrobial agents.

### Concentrations

According to a 15^{th} embodiment, in the textile material of any one of the preceding embodiments, the total amount of the one or more antimicrobial agents adhered to the textile material is at most 4.0%, preferably at most 3.0%, more preferably at most 2.5%, and most preferably at most about 2.0% on weight fabric of the textile material.

According to a 16^{th} embodiment, in the textile material of any one of the preceding embodiments, the total amount of the one or more antimicrobial agents adhered to the textile material is at least 0.1%, preferably at least 0.2%, more preferably at least 0.3%, and most preferably at least 0.4% on weight fabric of the textile material.

According to a 17^{th} embodiment, in the textile material of any one of embodiments 5 to 16, the polyhexamethylene biguanide is adhered to the textile material in an amount of at most 1.5%, preferably at most 1%, more preferably at most 0.8%, particularly at most 0.6%, and most preferably at most about 0.4% on weight fabric of the textile material.

According to a 18^{th} embodiment in the textile material of any one of embodiments 5 to 17, the polyhexamethylene biguanide is adhered to the textile material in an amount of at least 0.1%, preferably at least 0.2%, more preferably at least 0.3%, and most preferably at least about 0.4% on weight fabric of the textile material.

According to a 19^{th} embodiment, in textile material of any one of embodiments 6 to 18, the metal is adhered to the textile material in an amount of at most 0.1%, preferably at most 0.05%, more preferably at most 0.03%, particularly at most 0.02% and most preferably at most about 0.085% on weight fabric of the textile material.

According to a 20^{th} embodiment, in the textile material of any one of embodiments 5 to 19, the metal is adhered to the textile material in an amount of at least 0.0002%, preferably at least 0.0005%, more preferably at least 0.0010%, and most preferably at least about 0.0017% on weight fabric of the textile material.

According to a 21^{st} embodiment, in the textile material of any one of embodiments 5 to 20, the azole-based compound is adhered to the textile material in an amount of at most 2.5%, preferably at most 2.0%, more preferably at most 1.75%, and most preferably at most about 1.25% on weight fabric of the textile material.

According to a 22^{nd} embodiment, in the textile material of any one of embodiments 5 to 21, the azole-based compound is adhered to the textile material in an amount of at least 0.02%, preferably at least 0.05%, more preferably at least 0.10%, particularly at least 0.15%, and most preferably at least 0.25% on weight fabric of the textile material.

According to a 23^{rd} embodiment, in the textile material of any one of embodiments 5 to 22, the quaternary ammonium organosilane compound is adhered to the textile material in an amount of at most 2%, preferably at most 1.5%, more preferably at most 1.3%, in particular at most 1%, and most preferably at most about 0.72% on weight fabric of the textile material.

According to a 24^{th} embodiment, in the textile material of any one of embodiments 5 to 23, the quaternary ammonium organosilane compound is adhered to the textile material in an amount of at least 0.03%, preferably at least 0.05%, more preferably at least 0.10%, and most preferably at least about 0.14% on weight fabric of the textile material.

According to a 25^{th} embodiment, in the textile material of any one of embodiments 5 to 24, the polyglucosamine is adhered to the textile material in an amount of at most 1.5%, more preferably at most 1.0%, particularly at most 0.6%, most preferably at most about 0.3% on weight fabric of the textile material.

According to a 26^{th} embodiment, in the textile material of any one of embodiments 5 to 25, the polyglucosamine is adhered to the textile material in an amount of at least 0.05%, preferably at least 0.10%, more preferably at least 0.15%, particularly at least 0.20%, and most preferably at least about 0.3% on weight fabric of the textile material.

### Efficiency

According to a 27^{th} embodiment, in the textile material of any one of the preceding embodiments, the one or more antimicrobial agents adhered to the textile material increase the reduction value and/or the textile material exhibits a reduction value of *Escherichia coli* ATCC 25922 and/or *Staphylococcus aureus* ATCC 6538 and/or *Pseudomonas aeroginosa* ATCC 15442 and/or *Salmonella enterica* ATCC 10708 and/or *Candida albicans* ATCC 10231 and/or *Aspergillus niger* 16404 measured in accordance with AATCC test method 100-2012 and/or ASTM E2149-10 by/of at least 90% (1 log), preferably at least 99% (2 log), more preferably at least 99.9% (3 log), and most preferably at least 99.99% (4 log), within 1 hour of contact time, preferably within 15 minutes of contact time and/or of at least 99% (2 log), preferably at least 99.9% (3 log), more preferably at least 99.99% (4 log), particularly at least 99.999 (5 log), and most preferably at least 99.9999% (6 log) within 24 hours of contact time.

### Hydrophilic agents/stain release agents

### Types

According to a 28^{th} embodiment, in the textile material of any one of the preceding embodiments, the one or more hydrophilic agents adhered to the textile material comprise at least one selected from the group consisting of carboxylic acid esters, polyester ether copolymers, starch based emulsions, preferably fatty alcohol ethoxylates, and organosilane terpolymers, preferably fatty alcohol ethoxylates or organosilane terpolymers, most preferably organosilane terpolymers.

According to a 29^{th} embodiment, in the textile material of any one of the preceding embodiments, the one or more stain release agents adhered to the textile material comprise at least one selected from the group consisting of fatty alcohol ethoxylates, and organosilane terpolymers, preferably organosilane terpolymers.

According to a 30^{th} embodiment, in the textile material of any one of the preceding embodiments, one or more or all of the hydrophilic agents is/are (a) stain release agent(s).

According to a 31^{st} embodiment, in the textile material of any one of the preceding embodiments, one or more or all of the stain release agents is/are (a) hydrophilic agent(s).

### Concentrations

According to a 32nd embodiment, in the textile material of any one of the preceding embodiments, the hydrophilic and/or stain release agents are adhered to the textile material in an amount of together at most 5%, preferably at most 4%, more preferably at most 3%, even more preferably at most 2%, in particular at most 1.5%, and most preferably at most about 1% on weight fabric of the textile material.

According to a 33^{rd} embodiment, in the textile material of the preceding embodiment, the hydrophilic and/or stain release agents are adhered to the textile material in an amount of together at least 0.05%, preferably at least 0.1%, more preferably at least 0.2%, particularly at least 0.3%, and most preferably at least about 0.4% on weight fabric of the textile material.

According to a 34th embodiment, in the textile material of any one of the embodiments 28 or 29, or any one of embodiments 30 to 33 when dependent from any one of embodiments 28 or 29, the organosilane terpolymers are adhered to the textile material in an amount of at least 0.1%, preferably at least 0.2%, more preferably at least 0.4%, particularly at least 0.7%, and most preferably at least about 1% on weight fabric of the textile material.

According to a 35^{th} embodiment, in the textile material of any one of the embodiments 28 or 29, or any one of embodiments 30 to 34 when dependent from any one of embodiments 28 or 29, the fatty alcohol ethoxylates are adhered to the textile material in an amount of at most 3%, preferably at most 2%, more preferably at most 1.5%, even more preferably at most 1%, and most preferably at most about 0.7% on weight fabric of the textile material.

### Efficiency

According to a 36^{th} embodiment, in the textile material of any one of the preceding embodiments, the one or more hydrophilic agents adhered to the textile material reduce the water absorbency time by at least 20%, preferably at least 40%, more preferably at least 60%, and most preferably at least 80%, preferably when measured in accordance with AATCC test method 79-2014 (Option A).

According to a 37^{th} embodiment, in the textile material of any one of the preceding embodiments, exhibiting a water absorbency time of at most 3 seconds, preferably at most 2 seconds, more preferably at most 1 second, and most preferably at most 0.5 seconds, preferably when measured in accordance with AATCC test method 79-2014 (Option A).

According to a 38^{th} embodiment, in the textile material of any one of the preceding embodiments, the one or more hydrophilic agents adhered to the textile material do not increase the water repellency of the textile material.

According to a 39^{th} embodiment, in the textile material of any one of the preceding embodiments, exhibiting a water repellency rating of at most 50, preferably 0, measured in accordance with AATCC test method 22-2014.

According to a 40^{th} embodiment, in the textile material of any one of the preceding embodiments, the one or more hydrophilic agents adhered to the textile material increase the vertical wicking characteristics by at least a 25%, preferably at least 50%, and most preferably at least 100%, when tested according to AATCC test method 197-2013.

According to a 41^{st} embodiment, in the textile material of any one of the preceding embodiments, exhibiting a vertical wicking rate of at least 0.15 mm/sec, preferably at least 0.2 mm/sec, more preferably at least 0.25 mm/sec, and most preferably at least 0.3 mm/sec, when tested according to AATCC test method 197-2013.

According to a 42^{nd} embodiment, in the textile material of any one of the preceding embodiments, the one or more hydrophilic agents adhered to the textile material increase the horizontal wicking characteristics by at least 20%, preferably at least 40%, more preferably at least 60% and most preferably at least 100% when tested according to AATCC test method 198-2013.

According to a 43^{rd} embodiment, in the textile material of any one of the preceding embodiments, exhibiting a horizontal wicking rate of at least 10 mm²/sec, preferably at least 15 mm²/sec, and most preferably at least 20 mm²/sec, when tested according to AATCC test method 197-2013.

According to a 44^{th} embodiment, in the textile material of any one of the preceding embodiments, the one or more stain release agents adhered to the textile material increase the stain release rating of the textile material by at least 1 grade, more preferably by at least 2 grades, particularly at least 3 grades, and most preferably 4 grades, when tested according to AATCC test method 130-2010.

According to a 45^{th} embodiment, in the textile material of any one of the preceding embodiments, having a stain release rating, measured in accordance with AATCC test method 130-2010, of at least grade 3, preferably at least grade 4, and most preferably grade 5.

According to a 46^{th} embodiment, in the textile material of any one of the preceding embodiments, the one or more stain release agents adhered to the textile material increases the stain/oil repellency rating of the textile material, measured in accordance with AATCC test method 118-2013, by at most 1 grade, preferably does not increase the stain/oil repellency rating.

According to a 37^{th} embodiment, in the textile material of any one of the preceding embodiments, the textile material exhibits a stain/oil repellency rating, measured in accordance with AATCC test method 118-2013, of at most grade 1, preferably grade o.

### Wash durability

According to a 48^{th} embodiment, in the textile material of any one of the preceding embodiments, the embodimented properties of the textile material, such as stain release capability, antimicrobial efficiency, and/or properties related to hydrophilicity are achieved even after at least 25 laundry washes in a laundry washing machine at 85±15 °C for 10-15 minutes, preferably using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

### Starting textile material

According to a 50th embodiment, in the textile material of any one of the preceding embodiments, the starting textile material comprises functional groups having the ability to bond one or more antimicrobial agents to the textile material.

According to a 51st embodiment, in the textile material of any one of embodiments 1 to 50, the starting textile material comprises hydroxyl, peptide and/or carbonyl groups, in particular hydroxyl and/or peptide.

According to a 52^{nd} embodiment, in the textile material of any one of the preceding embodiments, the starting textile material is a cellulosic textile material, a synthetic textile material, or a blend of cellulosic textile material and synthetic textile material.

According to a 53^{rd} embodiment, in the textile material of any one of embodiment 52, the cellulosic textile material comprises cotton, viscose, rayon, linen, hemp, ramie, jute, and combinations (blends) thereof, preferably viscose.

According to a 54^{th} embodiment, in the textile material of any one of embodiments 52 or 53, the synthetic textile material comprises one or more selected from the group consisting of polyester, polyamide (nylon), acrylic polyester, spandex (elastane, Lycra), aramids, modal, sulfar, polylactide (PLA), lyocell, polybutyl tetrachloride (PBT), and combinations (blends) thereof, preferably polyester or polyamide (nylon), most preferably polyester.

According to a 55^{th} embodiment, in the textile material of any one of embodiments 52 to 54, the blend of cellulosic textile material and synthetic textile material comprises between 20% and 80% of cellulosic textile material, preferably between 30% and 75%, more preferably between 50% and 70%, and most preferably about 65%.

According to a 56^{th} embodiment, in the textile material of any one of embodiments 50 to 55, the blend of cellulosic textile material and synthetic textile material comprises between 20 and 80% of synthetic textile material, preferably between 25 and 70%, more preferably between 30 and 50%, and most preferably about 35%.

According to a 57^{th} embodiment, in the textile material of any one of the preceding embodiments, the textile material is a fiber, a yarn, or a fabric, preferably a yarn or a fabric, more preferably a fabric.

According to a 58^{th} embodiment, in the textile material of any one of embodiment 57, the fabric is selected from the group consisting of woven, knitted, warp-knitted, crocheted, and non-woven fabrics.

According to a 59^{th} embodiment, in the textile material of any one of the preceding embodiments, the starting textile material has a water holding capacity of at least 4 times its weight, preferably at least 6 times its weight, more preferably at least 7 times its weight, in particular at least 8 times its weight, most preferably at least 9 times its weight, preferably when tested according to ASTM D7367-14.

### Manufacturing process

According to a 60^{th} embodiment the invention is a process for finishing a textile material comprising the steps of:
- treating the textile material using an antimicrobial liquor application process like an exhaustion or padding process, wherein the liquor comprises one or more antimicrobial agents;
- preferably drying the textile material and treating the textile material using a hydrophilic/stain release liquor application process like an exhaustion or preferably a padding process, wherein the liquor comprises one or more hydrophilic agents and/or stain release agents; and
- subjecting the treated textile material to a heat treatment.

According to a 61^{st} embodiment, in the process for finishing a textile material of embodiment 60, the antimicrobial liquor application process is preferably an exhaust process, and between the step of treating the textile material using an antimicrobial liquor application process and the steps of drying the textile material and treating the textile material using a hydrophilic/stain release liquor application process, one or more process cycles are performed, comprising the steps of drying the textile material and treating the textile material using a further antimicrobial liquor application process, preferably a padding process, the liquor comprises one or more antimicrobial agents.

According to a 62^{nd} embodiment, in the process for finishing a textile material comprising the steps of:
- preferably treating the textile material using an antimicrobial liquor application process like an exhaustion or padding process, wherein the liquor comprises one or more antimicrobial agents and drying the textile material;
- treating the textile material using a hydrophilic/stain release liquor application process like an exhaustion or preferably a padding process, wherein the liquor comprises one or more hydrophilic agents and/or stain release agents; and
- subjecting the treated textile material to a heat treatment.

### Liquor

According to a 63^{rd} embodiment, in the process of any one of embodiments 60 to 62, the liquor of the antimicrobial and/or hydrophilic/stain release liquor application process has a pH-value of at most 6.9, preferably at most 6.5, more preferably at most 6.3, in particular at most 6.0, and most preferably at most about 5.5, and a pH-value of at least 3.0, preferably at least 3.5, more preferably at least 4.0, even more preferably at least 4.5, in particular at least 5.0,and most preferably at least about 5.5.

According to a 64^{th} embodiment, in the process of any one of embodiments 60 or 63, the amount of stain release and/or hydrophilic agents in the liquor of the antimicrobial liquor application process is together at most 10 gpl, preferably at most 5 gpl, more preferably at most 2 gpl, even more preferably at most 1 gpl, and most preferably o.

According to a 65^{th} embodiment, in the process of any one of embodiments 60 or 64, in the antimicrobial and/or the hydrophilic/stain release liquor application process, the agents and preferably all other components in the liquor are dissolved in the liquor, in particular are not dispersed in the liquor, and/or the liquor is substantially free of solids.

According to a 66th embodiment, in the process of any one of embodiments 1 to 65, the liquor of the antimicrobial and/or hydrophilic/stain release liquor application process contains a solvent, preferably water, and the agents and the solvent form a homogenous mixture, and in particular do not form a slurry or dispersion.

According to a 67^{th} embodiment, in the process of any one of embodiments 60 to 66, the value of dynamic viscosity of the liquor of the antimicrobial and/or hydrophilic/stain release liquor application process at 20 °C and/or 80 °C, in centipoise (cP), is at most 20% higher than the dynamic viscosity of water at 20 °C and/or 80 °C, respectively, preferably at most 10%, more preferably at most 5%, particularly at most 2%, and most preferably at most about 0%.

According to a 68^{th} embodiment, in the process of any one of embodiments 60 to 67, the amount of latex in the liquor of the antimicrobial and/or the hydrophilic/stain release liquor application process is at most 10 gpl, preferably at most 5 gpl, more preferably at most 2 gpl, even more preferably at most 1 gpl, and most preferably o.

According to a 69^{th} embodiment, in the process of any one of embodiments 60 to 67, the amount of cyclodextrin and/or inclusion complexes, in particular inclusion complexes of fiber-reactive cyclodextrin derivatives and antimicrobial agents in the liquor of the antimicrobial and/or the hydrophilic/stain release liquor application process is at most 10 gpl, preferably at most 5 gpl, more preferably at most 2 gpl, even more preferably at most 1 gpl, and most preferably o.

According to a 70^{th} embodiment, in the process of any one of embodiments 60 to 69, the amount of dye in the liquor of the antimicrobial and/or main process cycle is at most 10 gpl, preferably at most 5 gpl, more preferably at most 2 gpl, even more preferably at most 1 gpl, and most preferably o.

### Drying and curing

According to a 71^{st} embodiment, in the process of any one of embodiments 60 to 70, the drying before the hydrophilic/stain release liquor application process is conducted at least partially at an ambient temperature of at least 70 °C, preferably at least 100 °C, more preferably at least 110°C, most preferably at least about 120 °C.

According to a 72^{nd} embodiment, in the process of any one of embodiments 60 to 71, in the drying before the hydrophilic/stain release liquor application process is conducted at an ambient temperature of at most 160 °C, preferably of at most 140 °C, more preferably of at most 130 °C, and most preferably of at most about 120 °C.

According to a 73^{th} embodiment, in the process of any one of embodiments 60 to 72, the heat treatment comprises drying conducted at least partially at an ambient temperature of at least 60 °C, in particular at least 100 °C, preferably at least 110 °C, more preferably at least 120 °C.

According to a 74^{th} embodiment, in the process of any one of embodiments 60 to 73, the heat treatment comprises curing of the textile material, wherein the curing is conducted at least partially at a curing ambient temperature of at least 140 °C, preferably at least 160 °C, more preferably at least 170 °C, particularly at least 175 °C, and most preferably at least about 180 °C.

According to a 75^{th} embodiment, in the process of any one of embodiments 60 to 74, the heat treatment is conducted at an ambient temperature of at most 200 °C, preferably at most 190 °C, more preferably at most 185 °C, and most preferably at most about 180 °C.

According to a 76^{th} embodiment, in the process of any one of embodiments 74 to 75, curing takes place at the curing temperature as defined in embodiment 26d over a period of at least 30 seconds, preferably at least 40 seconds, more preferably at least 50 seconds, most preferably at least about 60 seconds, and preferably over a period of at most 120 seconds, preferably at most 90 seconds, more preferably at most 80 seconds, particularly at most 70 seconds, most preferably at most about 60 seconds.

According to a 77^{th} embodiment, in the process of any one of embodiments 74 to 76, the textile material is a fabric of at least 350 grams per m² and curing takes place at the curing temperature as defined in embodiment 24 over a period of at least 45 seconds, preferably at least 60 seconds, more preferably at least 75 seconds, most preferably at least about 90 seconds, and preferably over a period of at most 180 seconds, preferably at most 160 seconds, more preferably at most 140 seconds, particularly at most 120 seconds, most preferably at most about 90 seconds.

According to a 78^{th} embodiment, in the process of any one of embodiments 74 to 77, the textile material is a fabric of at least 500 grams per m² and curing takes place at the curing temperature as defined in embodiment 24 over a period of at least 60 seconds, preferably at least 75 seconds, more preferably at least 90 seconds, most preferably at least about 120 seconds, and preferably over a period of at most 240 seconds, preferably at most 210 seconds, more preferably at most 180 seconds, particularly at most 150 seconds, most preferably at most about 120 seconds.

According to a 79^{th} embodiment, in the process of any one of embodiments 74 to 78, curing immediately follows drying of the textile material without the textile material substantially cooling down between drying of the textile material and curing.

According to an 80^{th} embodiment, in the process of embodiment 79, the textile material is a fabric and drying of the textile material and curing are performed over a period of together at least 45 seconds, preferably at least 50 seconds, more preferably at least 55 seconds, most preferably at least about 60 seconds, per 100 grams of fabric weight per square meter.

According to an 81^{st} embodiment, in the process of any one of embodiments 79 or 80, the textile material is a fabric and drying of the textile material and curing are performed over a period of together at most 75 seconds, preferably at most 70 seconds, more preferably at most 65 seconds, most preferably at most about 60 seconds, per 100 grams of fabric weight per square meter.

### Exhaustion and padding parameters:

According to an 82^{nd} embodiment, in the process of any one of embodiments 60 to 81, where the antimicrobial and/or second liquor application process is an exhaustion process, the liquor has a temperature of at least 45 °C, preferably of at least 50 °C, more preferably of at least 60 °C, particularly of at least 70 °C, and most preferably of at least about 80 °C.

According to an 83^{nd} embodiment, in the process of any one of embodiments 60 to 82, where the antimicrobial and/or hydrophilic/stain release liquor application process is an exhaustion process, the liquor has a temperature below boiling temperature, preferably of at most 95 °C, more preferably of at most 90 °C, particularly of at most 85 °C, and most preferably of at most about 80 °C.

According to an 84^{th} embodiment, in the process of any one of embodiments 60 to 83, the exhaustion process time is at most 90 min, preferably at most 80 min, more preferably at most 70 min, and most preferably at most about 60 min.

According to an 85^{th} embodiment, in the process of any one of embodiments 60 to 84, the exhaustion process time is at least 45 min, preferably at least 50 min, more preferably at least 55 min, and most preferably at least about 60 min.

According to a 86^{th} embodiment, in the process of any one of embodiments 60 to 85, in a padding process of the antimicrobial and/or hydrophilic/stain release liquor application process, the liquor pickup rate is at least 30%, preferably at least 40%, more preferably at least 50%, particularly at least 60% or at least 80%, and most preferably at least about 100%, and/or at most 140%, preferably of at most 130%, more preferably of at most 120%, particularly at most 110%, and most preferably of at most about 100%

According to an 87^{th} embodiment, in the process of any one of embodiments 60 to 86, in a padding process of the antimicrobial and/or hydrophilic/stain release liquor application process, the textile material passes through a multiple trough padding mangle, or a continuous dyeing or bleaching range.

### Agents:

According to an 88^{th} embodiment, in the process of any one of embodiments 60 to 87, the one or more antimicrobial agents are selected and/or applied as defined in any one of embodiments 5 to 12.

According to an 89^{th} embodiment, in the process of any one of embodiments 60 to 88, the liquor of the hydrophilic/stain release liquor application process contains metal, preferably copper, zinc, or silver, more preferably silver cations.

According to a 90^{th} embodiment, in the process of embodiment 89, the liquor of the hydrophilic/stain release liquor application process contains such amounts of metal that in the hydrophilic/stain release liquor application process, the metal is applied to the textile material in an amount of at most 0.05%, preferably at most 0.01%, more preferably at most 0.005%, even more preferably at most 0.003%, and most preferably at most about 0.0017% on weight fabric of the textile material.

According to a 91^{st} embodiment, in the process of any one of embodiments 89 or 90, the liquor of the hydrophilic/stain release liquor application process contains such amounts of metal that in the hydrophilic/stain release liquor application process, the metal is applied to the textile material in an amount of at least 0.0001%, preferably at least 0.0005%, more preferably at least 0.001%, and most preferably at least about 0.0017% on weight fabric of the textile material.

According to a 92^{nd} embodiment, in the process of any one of embodiments 60 to 91, the antimicrobial agents are adhered to the textile material with a total weight as defined in embodiments 15 or 16, and/or an individual weight as defined for the respective antimicrobial agents in any one of embodiments 17 to 26.

According to a 93^{rd} embodiment, in the process of any one of embodiments 60 to 92, the one or more hydrophilic agents are selected and/or applied as defined in embodiment 28.

According to a 94^{th} embodiment, in the process of any one of embodiments 60 to 93, the one or more stain release agents are selected and/or applied as defined in embodiment 29.

According to a 95^{th} embodiment, in the process of any one of embodiments 60 to 94, the hydrophilic and/or stain release agents are adhered to the textile material with a total weight as defined in any one of embodiments 32 to 35.

### Starting textile material:

According to a 96^{th} embodiment, in the process of any one of embodiments 60 to 95, the (starting) textile material is a material as defined in any one of embodiments 50 t059.

### Other references between process and product embodiments:

According to a 97^{th} embodiment, in the process of any one of embodiments 60 to 96, the textile material obtained by the process is a textile material according to any one of embodiments 1 to 59.

According to a 98^{th} embodiment, in the process material obtainable according to the process of any one of embodiments 60 to 97.

### Transportation layer (layer 1):

According to a 99^{th} embodiment, in the textile material of any one of embodiments of embodiments 1 to 59, or embodiment 98, the textile material is an impression fabric, a knitted, warp knitted, and/or a multifilament fabric.

According to a 100^{th} embodiment, in the textile material of embodiment 99, the knitted or warp knitted fabric is a knit pique fabric.

According to a 101^{st} embodiment, in the textile material of any one of embodiments 1 to 59, or embodiment 98, or any one of embodiments 99 or 100, at least 95%, preferably at least 97%, more preferably at least 99% of the starting textile material consists of a synthetic fiber, such as polyamide (nylon) or preferably polyester.

According to a 102^{nd} embodiment, in the textile material of any one of embodiments 1 to 59, or embodiment 98, or any one of embodiments 99 to 101, the textile material has at least 25, preferably at least 30, more preferably at least 35, and most preferably at least about 40 holes, per cm².

According to a 103^{rd} embodiment, in the textile material of any one of embodiments 1 to 59, or embodiment 98, or any one of embodiments 99 to 102, the textile material has at most 625, preferably at most 300, more preferably at most 100, and most preferably at most about 40 holes, per cm².

According to a 104^{th} embodiment, in the textile material of any one of embodiments 1 to 59, or embodiment 98, the size of the holes is at least 0.02 mm, preferably at least 0.04 mm, more preferably at least 0.06 mm, in average diameter.

According to a 105^{th} embodiment, in the textile material of any one of embodiments 1 to 59, or embodiment 98, the size of the holes is at most 2.0 mm, preferably at most 1.5 mm, more preferably at most 1.0 mm, and most preferably at most 0.08 mm, in average diameter.

According to a 106^{th} embodiment, in the textile material of any one of embodiments 1 to 59, or embodiment 98, or any one of embodiments 99 to 105, the starting textile material has a weight of at least 50 GSM, preferably at least 80 GSM, more preferably at least 100 GSM, particularly at least 120 GSM, and most preferably at least about 125 GSM.

According to a 107^{th} embodiment, in the textile material of any one of embodiments 1 to 59, or embodiment 98, or any one of embodiments 99 to 106, the starting textile material has a weight of at most 250 GSM, preferably at most 200 GSM, more preferably at least 170 GSM, particularly at least 150 GSM, and most preferably at most about 125 GSM.

According to a 108^{th} embodiment, in the textile material of any one of embodiments 1 to 59, or embodiment 98, or any one of embodiments 99 to 107,
- the azole-based compound is adhered to the textile material in an amount of at least 0.05%, preferably at least 0.10%, more preferably at least 0.15%, particularly at least 0.20%, and most preferably at least about 0.25%, and/or in an amount of at most 2.0%, preferably at most 1.5%, more preferably at most 1.0%, particularly at most 0.5%, most preferably at most about 0.25% on weight fabric of the textile material; and/or
- the metal is adhered to the textile material in an amount of at least 0.0005%, preferably at least 0.001%, more preferably at least 0.002%, particularly at least 0.005%, most preferably at least about 0.01%, and/or in an amount of at most 0.1%, preferably at most 0.05%, more preferably at most 0.03%, particularly at most 0.02%, most preferably at most about 0.01% on weight fabric of the textile material; and/or
- the quaternary ammonium organosilane compound is adhered to the textile material in an amount of at least 0.01%, preferably at least 0.02%, more preferably at least 0.05%, particularly at least 0.10%, and most preferably at least about 0.14%, and/or in an amount of at most 1.0%, preferably at most 0.75%, more preferably at most 0.5%, particularly at most 0.25%, most preferably at most about 0.14% on weight fabric of the textile material.

### Main absorption layer(s) (layer 2):

According to a 109^{th} embodiment, in the textile material of any one of embodiments 1 to 59, or embodiment 98, the textile material is a heat set or heat bonded, spun bonded, melt blown, or preferably needle punch non-woven fabric, or a polar or preferably terry fleece fabric.

According to a 110^{th} embodiment, in the textile material of embodiment 109, the average diameter of the fibers of the non-woven fabric is at most 10 microns, preferably at most 6 microns, more preferably at most 3 microns, even more preferably at most 2 microns, and most preferably at most 1.5 microns.

According to a 111^{th} embodiment, in the textile material of any one of embodiments 1 to 59, or embodiment 95, or embodiments 109 or 110, the starting textile material comprises at least 30%, preferably at least 45%, more preferably at least 60%, most preferably at least about 65% viscose.

According to a 112^{th} embodiment, in the textile material of any one of embodiments 1 to 59, or embodiment 98, or any one of embodiments 109 or 111, the starting textile material comprises at most 100%, preferably at most 75%, more preferably at most 70%, most preferably at most about 65% viscose.

According to a 113^{th} embodiment, in the textile material of any one of embodiments 1 to 59, or embodiment 98, or any one of embodiments 109 to 112, the starting textile material comprises at least 15%, preferably at least 25%, more preferably at least 30%, most preferably at least about 35% of a synthetic fiber, such as polyamide (nylon) or preferably polyester.

According to a 114^{th} embodiment, in the textile material of any one of embodiments 1 to 59, or embodiment 98, or any one of embodiments 109 to 113, the starting textile material comprises at most 100%, preferably at most 65%, more preferably at most 40%, most preferably at most about 35% of a synthetic fiber, such as polyamide (nylon) or preferably polyester.

According to a 115^{th} embodiment, in the textile material of any one of embodiments 1 to 59, or embodiment 98, or any one of embodiments 109 to 114, the starting textile material has a weight of at least 100 GSM, preferably at least 120 GSM, more preferably at least 150 GSM, and most preferably at least about 200 GSM.

According to a 116^{th} embodiment, in the textile material of any one of embodiments 1 to 59, or embodiment 98, or any one of embodiments 109 to 115, the starting textile material has a weight of at most 700 GSM, preferably at most 500 GSM, more preferably at most 400 GSM, and most preferably at most about 300 GSM.

According to a 117^{th} embodiment, in the textile material of any one of embodiments 1 to 59, or embodiment 98, or any one of embodiments 109 to 116,
- the azole-based compound is adhered to the textile material in an amount of at least 0.10%, preferably at least 0.20%, more preferably at least 0.50%, particularly at least 1.0%, and most preferably at least about 1.25%, and/or in an amount of at most 3.0%, preferably at most 2.5%, more preferably at most 2.0%, particularly at most 1.75%, most preferably at most about 1.25% on weight fabric of the textile material; and/or
- the metal is adhered to the textile material in an amount of at least 0.0001%, preferably at least 0.0002%, more preferably at least 0.0005%, particularly at least 0.0010%, most preferably at least about 0.0017%, and/or in an amount of at most 0.05%, preferably at most 0.02%, more preferably at most 0.01%, particularly at most 0.005%, most preferably at most about 0.0017% on weight fabric of the textile material; and/or
- the polyhexamethylene biguanide is adhered to the textile material in an amount of at least 0.05%, preferably at least 0.1%, more preferably at least 0.2%, particularly at least 0.3%, and most preferably at least about 0.4%, and/or in an amount of at most 2.0%, preferably at most 1.5%, more preferably at most 1.0%, particularly at most 0.8%, most preferably at most about 0.4% on weight fabric of the textile material; and/or
- the polyglucosamine is adhered to the textile material in an amount of the at least 0.05%, preferably at least 0.10%, more preferably at least 0.15%, particularly at least 0.20%, and most preferably at least about 0.3%, and/or in an amount of at most 2.0%, preferably at most 1.5%, more preferably at most 1.0%, particularly at most 0.6%, most preferably at most about 0.3% on weight fabric of the textile material.

### Water repellent layer (layer 4):

According to a 118^{th} embodiment, in the sanitary napkin of any one of embodiments 1 to 59, or embodiment 98, the textile material is woven and/or a microfiber.

According to a 119^{th} embodiment, in the textile material of any one of embodiments 1 to 59, or embodiment 98, or embodiment 118, at least 95%, preferably at least 97%, more preferably at least 99% of the starting textile material consists of polyester, nylon, polyamide, polypropylene, or any other synthetic textile substrate.

According to a 120^{th} embodiment, in the textile material of any one of embodiments 1 to 59, or embodiment 98, or any one of embodiments 118 or 119, the starting textile material has a weight of at least 50 GSM, preferably at least 80 GSM, more preferably at least 110 GSM, and most preferably at least about 125 GSM.

According to a 121^{st} embodiment, in the textile material of any one of embodiments 1 to 59, or embodiment 98, or any one of embodiments 118 to 120, the starting textile material has a weight of at most 250 GSM, preferably at most 200 GSM, more preferably at least 150 GSM, and most preferably at most about 125 GSM.

According to a 122^{nd} embodiment, in the textile material of any one of embodiments 1 to 59, or embodiment 98, or any one of embodiments 118 to 121,
- the azole-based compound is adhered to the textile material in an amount of at least 0.1%, preferably at least 0.2%, more preferably at least 0.3%, particularly at least 0.4%, and most preferably at least about 0.5%, and/or in an amount of at most 2.0%, preferably at most 1.5%, more preferably at most 1.0%, particularly at most 0.75%, most preferably at most about 0.50% on weight fabric of the textile material; and/or
- the metal is adhered to the textile material in an amount of at least 0.0001%, preferably at least 0.0002%, more preferably at least 0.0005%, particularly at least 0.0010%, most preferably at least about 0.0017%, and/or in an amount of at most 0.05%, preferably at most 0.02%, more preferably at most 0.01%, particularly at most 0.005%, most preferably at most about 0.0017% on weight fabric of the textile material; and/or
- the quaternary ammonium organosilane compound is adhered to the textile material in an amount of at least 0.1%, preferably at least 0.2%, more preferably at least 0.3%, particularly at least 0.4%, and most preferably at least about 0.72%, and/or in an amount of at most 2.0%, preferably at most 1.5%, more preferably at most 1.3%, particularly at most 1.0%, most preferably at most about 0.72% on weight fabric of the textile material.

According to a 123^{rd} embodiment, in the textile material of any one of embodiments 1 to 59, or embodiment 98, or any one of embodiments 118 to 122, exhibiting a water repellency rating of at least 80, preferably at least 90, most preferably 100, measured in accordance with AATCC test method 22-2014.

According to a 124^{th} embodiment, in the textile material of any one of embodiments 1 to 59, or embodiment 98, or any one of embodiments 118 to 123, the textile material exhibits a stain/oil repellency rating, measured in accordance with AATCC test method 118-2013, of at least grade 4, preferably at least grade 5, more preferably at least grade 6, and most preferably at least grade 7.

According to a 125^{th} embodiment, in the textile material of any one of embodiments 1 to 59, or embodiment 98, or any one of embodiments 118 to 124, one or more water repellency agents are adhered to the textile material.

According to a 126^{th} embodiment, in the textile material of embodiment 125, the total amount of the one or more water repellency agents adhered to the textile material is at least 0.2%, preferably at least 0.5%, more preferably at least 1.0%, particularly at least 1.5%, and most preferably at least about 2.0%, and/or in an amount of at most 5.0%, preferably at most 4.0%, more preferably at most 3.0%, particularly at most 2.5%, most preferably at most about 2.0% on weight fabric of the textile material.

According to a 127^{th} embodiment, in the textile material of any one of embodiments 125 or 126, the textile material is obtainable by the process of any one of embodiments 60 to 97, the hydrophilic/stain release liquor application process is replaced by a water repellency liquor application process like an exhaustion or preferably a padding process, the liquor comprises the one or more water repellency agents.

According to a 128^{th} embodiment, in the textile material of any one of embodiments 125 to 127, the water repellency agents are C6- and/or C8-fluorocarbon and/or polyurethane (PU).

According to a 129^{th} embodiment, in the textile material of any one of embodiments 125 or 126, the textile material is obtainable by a synthetic rubber is applied to it after the antimicrobial liquor application process, preferably by screen or knife coating, preferably in an amount of at least 10% o.w.f, more preferably at least 20% o.w.f., even more preferably at least 30% o.w.f., particularly at least 40% o.w.f., most preferably at least about 50% o.w.f, and/or preferably in an amount of at most 150% o.w.f., more preferably at most 100% o.w.f., even more preferably at most 80% o.w.f., particularly at most 70% o.w.f., and most preferably at most about 50% o.w.f.

### Different uses of the textile material, in particular hygiene product:

According to a 130^{th} embodiment, the invention is a hygiene product comprising a textile material as defined in any one of embodiments 1 to 59, or embodiment 98, or any one of embodiments 99 to 128.

According to a 131^{st} embodiment, in the hygiene product of embodiment 130, the textile material forms a layer.

According to a 132^{nd} embodiment, in the hygiene product of embodiment 131, the hygiene product comprises at least a second layer formed by a textile material as defined in any one of embodiments 1 to 59, or embodiment 98, or any one of embodiments 99 to 128.

According to a 133^{rd} embodiment, in the hygiene product of any one of embodiments 131 to 132, the hygiene product comprises a further layer of a textile material.

According to a 134^{th} embodiment, in the hygiene product of embodiment 133, the further layer comprises or consists of a synthetic fabric or a natural fabric or a blend of both, with or without the addition of a stretch yarn, in particular lycra or spandex.

According to a 135^{th} embodiment, in the hygiene product of any one of embodiments 133 or 1134, at least one of the surfaces of the further layer is finished with a water-and-oil repellant, optionally including polyurethane (PU), preferably enhanced with perfluoroalkylethylacrylate copolymer with C6 or and C8 carbon chain.

According to a 136^{th} embodiment, in the hygiene product of any one of embodiments 130 to 135, the hygiene product is one selected from the group consisting of sanitary napkins, sanitary lungots or langotes, panty or underwear liners, tampons, nappies, diapers, diaper liners, adult diapers, panties or underwear, bras, bed pads, or nursing pads.

According to a 137^{th} embodiment, the invention is a towel, kitchen towel, napkin, handkerchief, face mask, floor cleaning mop, wipe, or textile used in wound care, comprising or consisting of a textile material as defined in any one of embodiments 1 to 59, or embodiment 98, or any one of embodiments 99 to 128.

### Sanitary napkin:

According to a 138^{th} embodiment, the invention is a sanitary napkin comprising
one or more dispersion layers comprising or consisting of textile material, and a water repellent layer, preferably having a water repellency rating of at least 70, preferably at least 80, more preferably at least 90, most preferably 100, measured in accordance with AATCC test method 22-2014.

According to a 139^{th} embodiment, in the sanitary napkin of embodiments 138, the one or more dispersion layers comprise a transportation layer comprising a textile material as defined in any one of embodiments 99 to 124.

According to a 140^{th} embodiment, in the sanitary napkin of embodiment 139, the transportation layer is the inner layer which lies against the skin of the user when the sanitary napkin is worn by the user.

According to a 141^{st} embodiment, in the sanitary napkin of any one of embodiments 138 to 140, the one or more dispersion layers comprise one or more main absorption layers, each main absorption layer comprising a textile material as defined in any one of embodiments 109 to 117.

According to a 142^{nd} embodiment, in the sanitary napkin of embodiments 141, the sanitary napkin comprises one main absorption layer of at least 50 GSM, preferably at least 100 GSM, more preferably at least 200, and most preferably at least 300 GSM.

According to a 143^{rd} embodiment, in the sanitary napkin of embodiments 141, the sanitary napkin comprises one main absorption layer of at most 700 GSM, preferably at most 500 GSM, more preferably at most 400, and most preferably at most 300 GSM.

According to a 144^{th} embodiment, in the sanitary napkin of any one of embodiments 139 or 140, and any one of embodiments 141 or 142, the one or more main absorption layers are located between the transportation layer and the water repellent layer.

According to a 145^{th} embodiment, in the sanitary napkin of any one of embodiments 141 to 144, the sanitary napkin comprises two main absorption layers.

According to a 146^{th} embodiment, in the sanitary napkin of embodiment 145, the sanitary napkin comprises one absorption layer of at least 50 GSM, preferably at least 100 GSM, more preferably at least 200, and most preferably at least 300 GSM and one absorption layer of at least 50 GSM, preferably at least 100 GSM, more preferably at least 150, and most preferably at least 200 GSM.

According to a 147^{th} embodiment, in the sanitary napkin of any one of embodiments 145 or 156, the sanitary napkin comprises one absorption layer of at most 500 GSM, preferably at most 400 GSM, more preferably at most 350, and most preferably at most 300 GSM and one absorption layer of at most 500 GSM, preferably at most 400 GSM, more preferably at most 300 GSM, most preferably at most 200 GSM.

According to a 148^{th} embodiment, in the sanitary napkin of any of embodiments 141 to 147, the total weight of the one or more main absorption layers is at least 1 gram, preferably at least 2 grams, more preferably at least 2.5 grams, most preferably at least 3 grams.

According to a 149^{th} embodiment, in the sanitary napkin of any of embodiments 141 to 147, the total weight of the one or more main absorption layers is at most 20 grams, preferably at most 15 grams, more preferably at most 10 grams, even more preferably at most 8 grams, and most preferably at most 6 grams.

According to a 150^{th} embodiment, in the sanitary napkin of any one of embodiments 138 to 149, the water repellent layer is a textile material as defined in any one of embodiments 118 to 128.

According to a 151^{th} embodiment, in the sanitary napkin of any one of embodiments 138 to 150, the water repellent layer is the outer layer of the sanitary napkin.

According to a 152^{nd} embodiment, in the sanitary napkin of any one of embodiments 138 to 151, a water impermeable layer is placed between the dispersion layers and the outer layer of the sanitary napkin.

According to a 153^{th} embodiment, in the sanitary napkin of 152, the water impermeable layer comprises a substrate such as bonded, preferably spun bonded non-woven textile fabric or paper, the substrate being coated by a water impermeable film.

According to a 154^{th} embodiment, in the sanitary napkin of 153, the water impermeable film is made of or comprises plastic, preferably polyurethane (PU).

According to a 155^{th} embodiment, in the sanitary napkin of embodiments 153 or 154, the water impermeable film is air permeable.

According to a 156^{th} embodiment, in the sanitary napkin of embodiments 153 or 155, the water impermeable film is located on the inner (upper) side of the water impermeable layer and the substrate is located on the outer (lower) side of the water impermeable layer.

According to a 157^{th} embodiment, in the sanitary napkin of embodiments 153 or 155, the water impermeable film is sandwiched between two layers of substrate.

According to a 158^{th} embodiment, in the sanitary napkin of any one of embodiments 153 to 157, the weight of the water impermeable layer is at least 20 GSM, preferably at least 30 GSM, more preferably at least 40 GSM, most preferably at least 50 GSM.

According to a 159^{th} embodiment, in the sanitary napkin of any one of embodiments 153 to 157, the weight of the water impermeable layer is at most 150 GSM, preferably at most 100 GSM, more preferably at most 75 GSM, most preferably at most 50 GSM.

According to a 160^{th} embodiment, in the sanitary napkin of any one of embodiments 153 to 158, the substrate is brown.

### Fluid dispersion:

According to a 161^{st} embodiment, in the sanitary napkin of any one of embodiments 138 to 160, the one or more dispersion layers in conjunction have fluid dispersion properties such that if 1 ml of water is dropped onto the dispersion layers, the water is dispersed within 1 sec in a dispersion area having an average diameter of at least 3 cm, preferably at least 4 cm, more preferably at least 4.5 cm, and most preferably at least 5 cm.

### Shape and construction of the sanitary napkin:

According to a 162^{rd} embodiment, in the sanitary napkin of any one of embodiments 138 to 161, having a functional zone through which it absorbs fluids.

According to a 163^{rd} embodiment, in the sanitary napkin of embodiment 162, the functional zone has a maximum and/or minimum width of at least 3 cm, preferably at least 4 cm, most preferably at least 6 cm.

According to a 164^{th} embodiment, in the sanitary napkin of embodiment 162 or 163, the functional zone has a maximum and/or minimum width of at most 20 cm, preferably at most 15 cm, more preferably at most 10, most preferably at most 7.5 cm.

According to a 165^{th} embodiment, in the sanitary napkin of any one of embodiments 162 to 164, the functional zone has a maximum and/or minimum length of at least 10 cm, preferably at least 15 cm, more preferably at least 20 cm, most preferably at least 23 cm.

According to a 166^{th} embodiment, in the sanitary napkin of any one of embodiments 162 to 165, the functional zone has a maximum and/or minimum length of at most 50 cm, preferably at most 40 cm, more preferably at most 35cm, most preferably at most 30 cm.

According to a 167^{th} embodiment, in the sanitary napkin of any one of embodiments 162 to 166, the sanitary napkin has a seam which is located at one or more edges of the sanitary napkin, where one or more layers of the sanitary napkin are directly or indirectly attached together.

According to a 168^{th} embodiment, in the sanitary napkin of embodiment 167, the one or more layers are attached together by stitching, gluing, or preferably by ultrasonic welding.

According to a 169^{th} embodiment, in the sanitary napkin of any one of embodiments 167 or 168, the seam is located on one or preferably both longitudinal edges of the sanitary napkin, more preferably on all edges of the sanitary napkin.

According to a 170^{th} embodiment, in the sanitary napkin of any one of embodiments 167 to 169, at least the inner and/or the outer layer are among the layers attached together by the seam.

According to a 171^{th} embodiment, in the sanitary napkin of any one of embodiments 167 to 170, when dependent from embodiment 152, the water impermeable layer is among the layers attached together by the seam.

According to a 172^{nd} embodiment, in the sanitary napkin of any one of embodiments 167 to 171, when dependent from embodiment 141, one or more or preferably all main absorption layers are not among the layers attached together by the seam.

According to a 173^{rd} embodiment, in the sanitary napkin of embodiment 172, the seam is located on both longitudinal edges of the sanitary napkin, and the width of one or more or preferably all of the absorption layers, along at least 50%, preferably at least 75%, more preferably at least 90%, most preferably 100% of the of the length of the respective absorption layer, is substantially equal to the respective distance between the inner edges of the seams on the longitudinal sides, substantially equal means plus/minus 2 centimeters, preferably plus/minus 1.5 centimeters, more preferably plus/minus 1 centimeter, even more preferably plus/minus 5 millimeters, and most preferably plus/minus 3 millimeters.

According to a 174^{th} embodiment, in the sanitary napkin of any of the embodiments 167 to 173 the seam has a width of at least 2 millimeters, preferably at least 4 millimeters, more preferably at least 6 millimeters, even more preferably at least 8 millimeters, most preferably at least 1 centimeter.

According to a 175^{th} embodiment, in the sanitary napkin of any of the embodiments 167 to 174, the seam has a width of at most 2 centimeters, preferably at most 1.5 centimeters, more preferably at most 1.3 centimeters, most preferably at most 1 centimeter.

According to a 176^{th} embodiment, in the sanitary napkin of any of the embodiments 167 to 175, the water repellent layer is folded on one or preferably both longitudinal edges of the sanitary napkin such that the water repellent layer covers the one or more dispersion layers in an area of the seam.

According to a 177^{th} embodiment, in the sanitary napkin of any of the embodiments 167 to 176, when dependent from embodiment 152, the water impermeable layer is folded on one or preferably both longitudinal edges of the sanitary napkin such that the water impermeable layer covers the one or more dispersion layers in an area of the seam.

According to a 178^{th} embodiment, in the sanitary napkin of any one of embodiments 138 to 177, the sanitary napkin comprises fixing means to attach the sanitary napkin to a textile, in particular an undergarment, or to the user.

According to a 179^{th} embodiment, in the sanitary napkin of embodiment 178, the fixing means are wings which can be attached to each other, preferably by a snap fastener.

According to a 180^{th} embodiment, in the sanitary napkin of embodiment 179, the wings are an extension of the outer layer.

According to a 181^{st} embodiment, in the sanitary napkin of embodiment 180, wings are part of the outer layer, and obtained by folding the outer layer on itself at least twice at the level of the seam.

According to a 182^{nd} embodiment, in the sanitary napkin of any one of embodiments 138 to 178, the fixing means are one or more ribbons fixing the sanitary napkin to the user and attached at the edge of at least one or preferably both longitudinal ends of the sanitary napkin, preferably at the outer layer thereof.

According to a 183^{rd} embodiment, in the sanitary napkin of embodiment 182, the one or more ribbons are stretched around the waist of the user.

According to a 184^{th} embodiment, in the sanitary napkin of one of embodiments 182 or 183, the one or more ribbons are made of or comprise an elastic material, preferably rubber.

According to a 185^{th} embodiment, in the sanitary napkin of any one of embodiments 182 to 184, the outer layer is folded at one or preferably both longitudinal ends, and attached to itself, leaving a passing through which the one or more ribbons are disposed.

### Pad:

According to a 186^{th} embodiment, in the sanitary napkin of any one of embodiments 162 to 177, the minimum and/or maximum length of the sanitary napkin is at least 10 cm, preferably at least 20 cm, more preferably at least 25 cm, most preferably at least 28 cm.

According to a 187^{th} embodiment, in the sanitary napkin of any one of embodiments 162 to 177 or 186, the minimum and/or maximum length of the sanitary napkin is at most 60 cm, preferably at most 40 cm, more preferably at most 35 cm, most preferably at most 31,5 cm.

According to a 188^{th} embodiment, in the sanitary napkin of any one of embodiments 162 to 177 or any one of embodiments 186 to 187, the minimum and/or maximum width of the sanitary napkin is at least 4 cm, preferably at least 6 cm, more preferably at least 8 cm, most preferably at least 8,7 cm.

According to a 189^{th} embodiment, in the sanitary napkin of any one of embodiments 162 to 177 or any one of embodiments 186 to 188, the minimum and/or maximum width of the sanitary napkin is at most 20 cm, preferably at most 12 cm, more preferably at most 9 cm, most preferably at most 8,7 cm.

According to a 190^{th} embodiment, in the sanitary napkin of any one of embodiments 162 to 177 or any one of embodiments 186 to 189, the minimum width of functional zone is at least 3 cm, preferably at least 5 cm, more preferably at least 6 cm, most preferably at least 7 cm.

According to a 191^{st} embodiment, in the sanitary napkin of any one of embodiments 162 to 177 or any one of embodiments 186 to 190, the maximum width of functional zone is at most 20 cm, preferably at most 15 cm, more preferably at most 10 cm, most preferably at most 7 cm.

According to a 192^{nd} embodiment, in the sanitary napkin of any one of embodiments 162 to 177 or any one of embodiments 186 to 191, the length of functional zone is at least 10 cm, preferably at least 20 cm, more preferably at least 25 cm, most preferably at least 27 cm.

According to a 193^{rd} embodiment, in the sanitary napkin of any one of embodiments 162 to 177 or any one of embodiments 186 to 192, the length of functional zone is at most 60 cm, preferably at most 40 cm, more preferably at most 35 cm, most preferably at most 30 cm.

According to a 194^{th} embodiment, in the sanitary napkin of embodiment 178, and of any one of embodiments 162 to 177 or any one of embodiments 186 to 193, the fixing means are wings as in embodiments 179 to 181.

### Lungote:

According to a 195^{th} embodiment, in the sanitary napkin of any one of embodiments 162 to 185, the minimum and/or maximum length of the sanitary napkin is at least 20 cm, preferably at least 40cm, more preferably at least 50 cm, most preferably at least 56 cm.

According to a 196^{th} embodiment, in the sanitary napkin of any one of embodiments 162 to 185 or 195, the minimum and/or maximum length of the sanitary napkin is at 100 most cm, preferably at most 80 cm, more preferably at most 70 cm, most preferably at most 61 cm.

According to a 197^{th} embodiment, in the sanitary napkin of any one of embodiments 162 to 185 or any one of embodiments 195 to 196, the minimum and/or maximum width of the sanitary napkin is at least 4 cm, preferably at least 5 cm, more preferably at least 7 cm, most preferably at least 8 cm.

According to a 198^{th} embodiment, in the sanitary napkin of any one of embodiments 162 to 185 or any one of embodiments 195 to 197, the minimum and/or maximum width of the sanitary napkin is at most 25 cm, preferably at most 15 cm, more preferably at most 10 cm, most preferably at most 9 cm.

According to a 199^{th} embodiment, in the sanitary napkin of any one of embodiments 162 to 185 or any one of embodiments 195 to 198, the minimum and/or maximum width of functional zone is at least 3 cm, preferably at least 4 cm, more preferably at least 5 cm, most preferably at least 6 cm.

According to a 200^{th} embodiment, in the sanitary napkin of any one of embodiments 162 to 185 or any one of embodiments 195 to 199, the minimum and/or maximum width of functional zone is at most 24 cm, preferably at most 14 cm, more preferably at most 9 cm, most preferably at most 7 cm.

According to a 201^{st} embodiment, in the sanitary napkin of any one of embodiments 162 to 185 or any one of embodiments 195 to 199, the minimum and/or maximum length of the functional zone is at least 10 cm, preferably at least 15 cm, more preferably at least 20 cm, most preferably at least 23 cm.

According to a 202^{nd} embodiment, in the sanitary napkin of any one of embodiments 162 to 185 or any one of embodiments 195 to 201, the minimum and/or maximum length of the functional zone is at most 60 cm, preferably at most 40 cm, more preferably at most 35 cm, most preferably at most 30 cm.

According to a 203^{rd} embodiment, in the sanitary napkin of any one of embodiments 162 to 185 or any one of embodiments 195 to 202, the outer layer is longer than the functional zone, preferably extends substantially over the entire length of the sanitary napkin.

According to a 204^{th} embodiment, in the sanitary napkin of any one of embodiments 162 to 185 or any one of embodiments 195 to 203, the fixing means are ribbons as embodimented in embodiments 182 to 183.

### Sonotrode:

According to a 205^{th} embodiment, the invention is sonotrode for use in the process of attaching together the various layers of the sanitary napkin of any of the proceeding embodiments at the level of the seam using ultrasonic welding.

According to a 206^{th} embodiment, in the sonotrode of embodiment 205, the sonotrode comprises protrusions arranged in repeating patterns

According to a 207^{th} embodiment, in the sonotrode of embodiment 206, the repeating patterns of the protrusion are lines or preferably grid of lines.

According to a 208^{th} embodiment, in the sonotrode of embodiment 206 or 207, the distance between the lines formed by the protrusions, being calculated between the centers of the tops of two consecutive protrusions, can be of at least of 1 mm, preferably of at least 1,5 mm, more preferably of at least 1,8 mm and most preferably of at least of 2mm.

According to a 209^{th} embodiment, in the sonotrode of any of embodiment 206 to 208, the distance between the lines formed by the protrusions, being calculated between the centers of the tops of two consecutive protrusions, can be of at most of 4 mm, preferably of at most 3 mm, more preferably of at most 2.5 mm and most preferably of at most of 2mm.

According to a 210^{th} embodiment, in the sonotrode of embodiment any of embodiment 206 to 209, one or all the sides of the protrusions form with the vertical direction an angle of at least 35°, preferably at least 40°, more preferably at least 45° and most preferably of at least 48.2°.

According to a 211^{st} embodiment, in the sonotrode of embodiment any of embodiment 206 to 210, one or all the sides of the protrusions form with the vertical direction an angle of at most 60°, preferably of at most 55°, more preferably of at most 50° and most preferably of at most 48.2°.

According to a 212^{nd} embodiment, in the sonotrode of any one of embodiments 206 to 211, the tops of the protrusions have a width of at least 0.1 mm, preferably at least 0.2 mm, more preferably at least 0.25 mm and most preferably of at least 0.3 mm.

According to a 213^{rd} embodiment, in the sonotrode of any one of embodiments 206 to 211, the tops of the protrusions have a width of at most 0.5 mm, preferably at most 0.4 mm, more preferably at most 0.35 mm and most preferably of at most 0.3 mm.

According to a 214^{th} embodiment, in the sonotrode of any one of embodiments 206 to 213, the top of protrusions is rounded.

According to a 215^{th} embodiment, in the sonotrode of embodiments 214, the width of embodiment 212 and 213 is the distance between two points, at which the slope of the sides of the protrusion reaches an angle with the horizontal direction of at most 25°, preferably of at most 20°, more preferably at most 15° and most preferably of at most 10°.

According to a 216^{th} embodiment, in the sonotrode of any one of the embodiments 206 to 215, the protrusions have a height of at most 1,5 mm, preferably at most 1,2 mm, more preferably of at most 0,9 mm, most preferably of at most 0,7 mm.

According to a 217^{th} embodiment, in the sonotrode of any one of the embodiments 206 to 216, the protrusions have a height of at least 0,3 mm, preferably at least 0,5 mm, more preferably of at least 0,6 mm, most preferably of at least 0,7 mm.

According to a 218^{th} embodiment, in the sonotrode of any one of embodiments 206 to 217, the sonotrode can be a flat blade or a roller.

### DETAILED DESCRIPTION OF PREFERRED EMBODIMENTS

In the following, preferred embodiments of the invention are described with reference to the figures, in which:
- Fig. 1: is a flowchart illustrating a process of finishing a textile material according to embodiments of the invention;
- Fig. 2: is a flowchart illustrating a process of finishing a textile material according to a preferred embodiment of the invention;
- Fig. 3: shows an embodiment of a sanitary napkin according to the invention, having the shape of a Pad;
- Fig. 4: shows a cross section of the embodiment of the sanitary napkin along the line A-B shown in Fig. 3;
- Fig. 5: shows a cross section of a modified embodiment of the sanitary napkin along the line A-B shown in Fig. 3;
- Fig. 6: shows an embodiment of the sanitary napkin having the form of a Pad and wings as fixing means;
- Fig. 7: shows a cross section of the embodiment of the sanitary napkin of Fig. 6 along the line A'-B' shown in Fig. 5;
- Fig. 8: shows an embodiment of the sanitary napkin having the shape of a langot;
- Fig. 9: shows a cross section of the embodiment of the sanitary napkin along the line A-B shown in Fig. 8;
- Fig. 10: shows a cross section of the sanitary napkin of Fig. 8 along the line E-F shown in Fig. 8;
- Fig. 11: specifies a textile material according to a working examples of the invention which can be used as a transportation layer of a sanitary napkin;
- Fig. 12: specifies a textile material according to a working examples of the invention which can be used as a main absorption layer of a sanitary napkin;
- Fig. 13: specifies a textile material according to a working examples of the invention which can be used as a water-repellent layer of a sanitary napkin;
- Fig. 14 A to D: show cross sections of the protrusions of a sonotrode for use in the process of attaching the various layers of the sanitary napkin when using ultrasonic welding;
- Fig. 15: show a sonotrode according to an embodiment of the invention;
- Fig. 16: shows the results of absorbency and antimicrobial performance for one embodiment of the invention;
- Fig. 17: shows the results of absorbency and antimicrobial performance for one embodiment of the invention;
- Fig. 18: shows the results of absorbency and antimicrobial performance for one embodiment of the invention;
- Fig. 19AA to 19BC: specify textile materials according to experimental examples of the invention, wherein one single antimicrobial agent was applied;
- Fig. 20AA to: 20BC specify textile materials according to experimental examples of the invention, wherein two antimicrobial agents were applied;
- Fig. 21A and 21B: specify textile materials according to experimental examples of the invention, wherein three antimicrobial agents were applied;
- Fig.22 A and 22B: specify textile materials according to experimental examples of the invention, wherein only antimicrobial agents were applied;
- Fig. 23AA to 23BC: specify textile materials according to experimental examples of the invention, wherein antimicrobial agents and Hydrosil were applied;
- Fig. 24AA to 24BC: specify textile materials according to experimental examples of the invention, wherein antimicrobial agents and Permalose were applied;
- Fig. 25AA to 25BC: specify textile materials according to experimental examples of the invention, wherein different exhaust times were applied;
- Fig. 26AA to 26BD: specify textile materials according to experimental examples of the invention, wherein different curing temperatures were applied;
- Fig. 27: specifies a textile material according to an experimental example of the invention, wherein the antimicrobial agents were applied in a padding process

### Antimicrobial agents

### Preferred antimicrobial agents

A great variety of antimicrobial agents can be fixed to a textile by using the process of the invention described below. The antimicrobial agents are preferably non-ionic or cationic, but not anionic. The inventors found that anionic compounds do not bind well to textiles and can easily be removed, e.g. by salts. Cationic (acid) agents are believed to attack the textile and therefore attach to it. Nanoparticles or antimicrobials in the form of nanoparticles are not preferred. Rather, the one or more antimicrobial agents have a preferred particle size, in all dimensions (length, width, height), of at least 250 nanometers, preferably at least 500 nanometers, more preferably at least 750 nanometers, and most preferably at least 1,000 nanometers.

According to the preferred embodiments of the invention, an antimicrobial agent is selected from quaternary ammonium organosilane compounds, metal, polyglucosamine (chitosan), azole-based compounds, and polyhexamethylene biguanide (PHMB).

As will be described for the preferred antimicrobial agents in detail below, the antimicrobial agents are bound to the textile material preferably either directly, in particular if the agent is a quaternary ammonium organosilane compound, polyglucosamine, a silver, copper, or zinc cation, which can be trapped in an inorganic or organic matrix, or PHMB, or via cross linking, in particular if the agent is an azole-based compound. The use of cyclodextrin and/or an inclusion complex, in particular an inclusion complex of fiber-reactive cyclodextrin derivatives and antimicrobial agents is not preferred for binding the antimicrobial agents, in particular because cyclodextrin is prohibitively expensive for most applications.

Suitable quaternary ammonium organosilane compounds have the formula 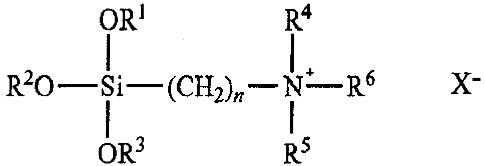 wherein the radicals have, independently of each other, the following meanings: R¹, R², and R³ are a C₁-C₁₂-alkyl group, in particular a C₁-C₆-alkyl group, preferably a methyl group; R⁴, and R⁵ are a C₁-C₁₈-alkyl group, a C₁-C₁₈-hydroxyalkyl group, a C₃-C₇-cycloalkyl group, a phenyl group, or a C₇-C₁₀-aralkyl group, in particular a C₁-C₁₈-alkyl group, preferably a methyl group; R⁶ is a C₁-C₁₈-alkyl group, in particular a C₈-C₁₈-alkyl group; X- is the counterion and an anion, for example, chloride, bromide, fluoride, iodide, acetate, or a sulfonate group, preferably X⁻ is chloride or bromide; and n is an integer of 1 to 6, in particular an integer of 1 to 4, preferably 3. The term "alkyl group" as used herein means a branched or unbranched alkyl group.

Quaternary ammonium organosilane compounds are known in the art and commercially available. Such compounds possess specific functional groups which enable their bonding to functional groups of the textile material. Under the reaction conditions disclosed herein, the quaternary ammonium organosilane compounds are bonded to the textile material via a covalent bond between the organosilane moiety and functional groups of the textile. Furthermore, organosilane moieties polymerize with each other, resulting in -O-Si-O- bonds. A possible reaction mechanism of the ammonium organosilane with a textile material having hydroxyl groups is shown hereinafter.

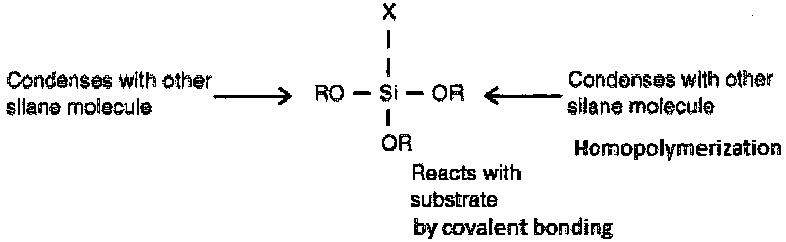

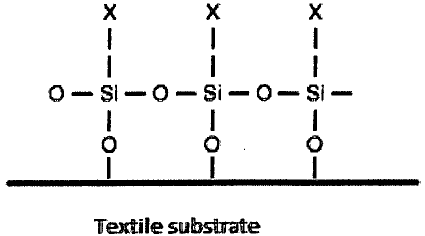

A possible reaction mechanism of the ammonium organosilane with silk having peptide groups (-CO-NH-) is shown hereinafter.

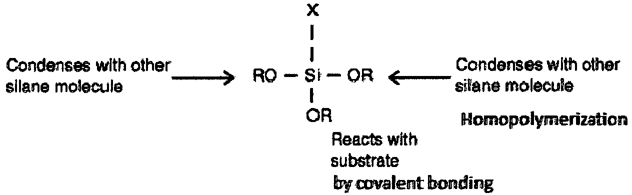

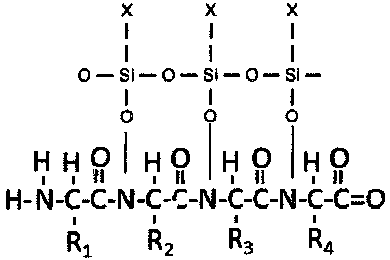

The quaternary ammonium organosilane compound can comprise dimethyloctadecyl[3-(trimethoxysilyl)propyl] ammonium chloride or dimethyltetradecyl[3-(trimethoxysilyl)propyl] ammonium chloride, most preferably dimethyloctadecyl[3-(trimethoxysilyl)propyl]ammonium chloride. The structure of dimethyloctadecyl[3-(trimethoxysilyl)propyl]ammonium is as follows (shown without counterion), wherein further the function of the silane moiety and the ammonium moiety are indicated:

### Organositane quaternary amine

### Molecular Structure:

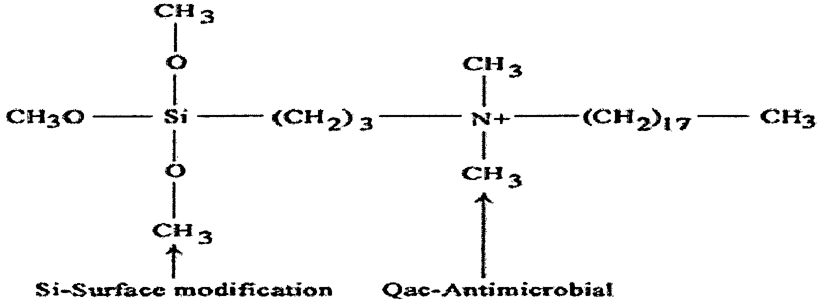

Dimethyloctadecyl[3-(trimethoxysilyl)propyl] ammonium chloride is available on the market, e.g. in AEM 5772 (manufactured by Aegis, USA). Dimethyltetradecyl[3-(trimethoxysilyl)propyl] ammonium chloride is available on the market, e.g. in Sanitized T 99-19 (manufactured by Sanitized AG, Switzerland). Other suitable ammonium silane compounds are described, e.g., in patent applications US 2011/0271873 A1 and US 2006/0193816 A1, and in US patent 8,906,115.

The metal may be copper, zinc, or preferably silver, more preferably silver cations. Tests made by the inventors, whose results are not reproduced herein, showed that copper nitrate and zinc nitrate have similar antimicrobial properties as silver cations, and they can be adhered to textile materials in similar ways. However, the antimicrobial performance of silver is considerably higher than that of zinc, and even much higher than that of copper. Furthermore, treating textile materials with copper or zinc tends to lead more easily to a change of the shade of textile than a treatment with silver.

In particular embodiments, the silver cations are trapped in an inorganic or organic matrix. Preferably, the inorganic matrix is an aluminosilicate. Preferably, the organic matrix is a polymeric matrix. Such silver-containing microbial agents are known in the art and available on the market.

A silver cation in form of its acrylate salt is shown hereinafter.

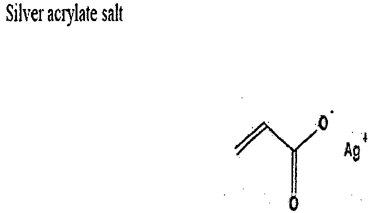

In an exemplary embodiment of the invention, the aluminosilicate is a sodium-poly(sialate-disiloxo) compound. Examples of an aluminosilicate and sialate structures as well as how bonding to a textile material can occur under the reaction conditions disclosed herein, are shown hereinafter.

| | |
|---|---|
| **Si:Al = 1** | 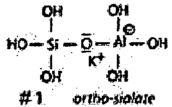 |
| **Si:Al = 2** | 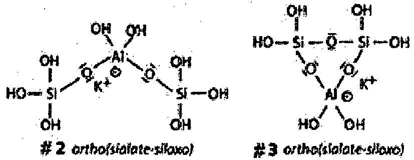 |
| **Si:Al = 3** | 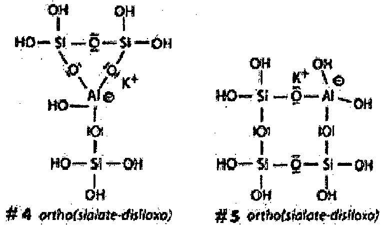 |

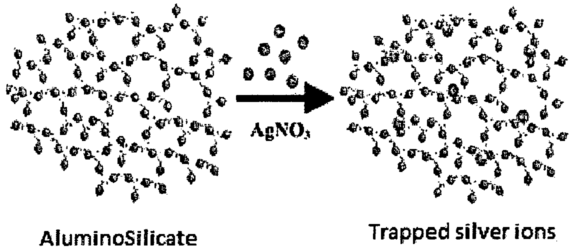

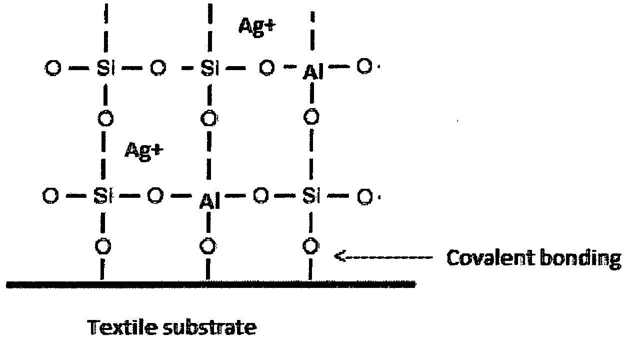

In an exemplary embodiment of the invention, the polymeric matrix into which silver cations are trapped is an acrylic polymer. Such silver-containing agents are known in the art and available on the market, e.g. in SilvaDur AQ Antimicrobial of Rohm and Haas, Switzerland, which contains acrylic polymer(s), silver nitrate, nitric acid and water. In another exemplary embodiment of the invention, the silver cations are trapped in a polymeric matrix. Such silver-containing agents are known in the art and available on the market, e.g. in SILVADUR™ 930 Antimicrobial of Dow Chemical Company, USA, which contains polymer(s), silver cations, ammonia, ethanol and water.

Polyglucosamine (chitosan) has a structure as shown hereinafter, wherein n indicates the number of monomer units as known in the art:

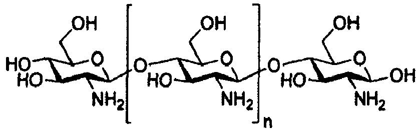

Under the reaction conditions disclosed herein, chitosan can react with -NH groups of silk resulting in covalent bonds as shown below.

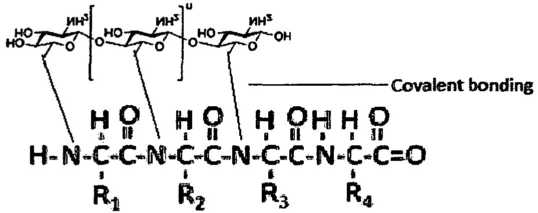

Under the reaction conditions disclosed herein, chitosan can react with functional groups of cellulosic materials resulting in covalent bonds as shown below.

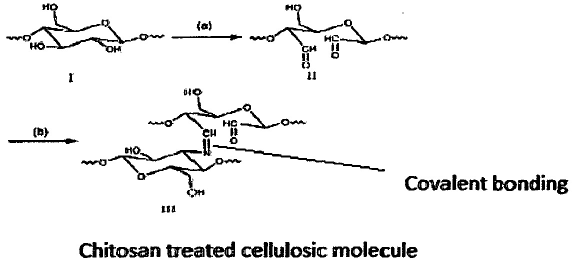

Chitosan is known in the art and commercially available, e.g. in Goyenchem-102 of Go Yen Chemical, Taiwan. It is particularly effective for killing viruses. It cannot easily be bound to synthetic textile materials in a wash-durable or even substantially non-leaching manner.

Polyhexamethylene biguanide (PHMB) has a structure as shown hereinafter, wherein n indicates the number of monomer units as known in the art.

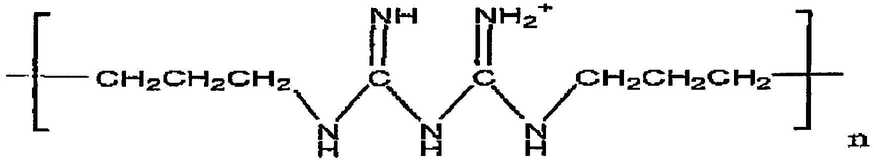

Under the reaction conditions disclosed herein, polyhexamethylene biguanide can react with hydroxyl groups of cellulose to form covalent bonds as shown hereinafter.

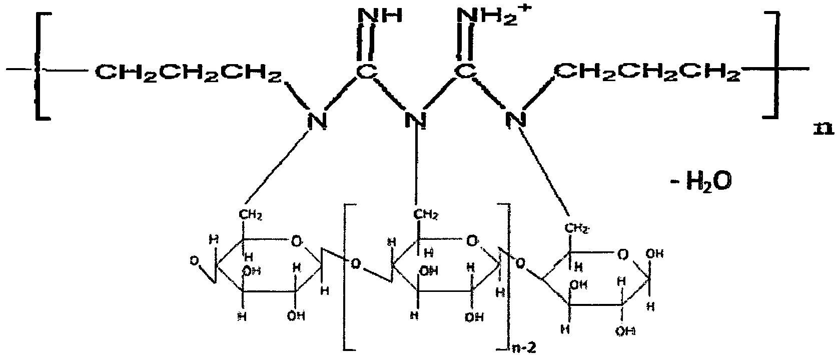

Under the reaction conditions disclosed herein, PHMB can react with carbonyl groups of silk fiber to form covalent bonds as shown hereinafter. 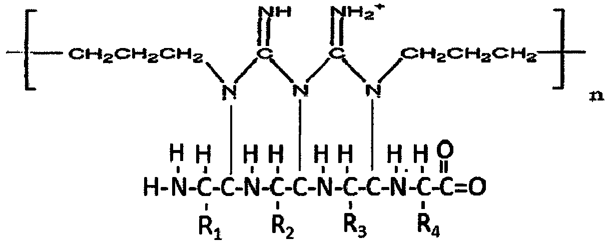 PHMB is known in the art and commercially available, e.g. in Texguard 20 of Swissol Specialties Pvt. Ltd, India. It cannot easily be bound to synthetic textile materials in a wash-durable or even substantially non-leaching manner.

The azole-based compound can be, e.g., thiabendazole, carbendazim or preferably a triazole-based compound. Tests made by the inventors, whose results are not reproduced herein, showed that thiabendazole has similar antimicrobial properties as the triazole-based compounds, and it can be adhered to textile materials in similar ways. However, the antimicrobial performance of triazole-based compounds is considerably higher than that of thiabendazole.

The triazole-based compound is preferably propiconazole. Propiconazole has a structure as shown hereinafter.

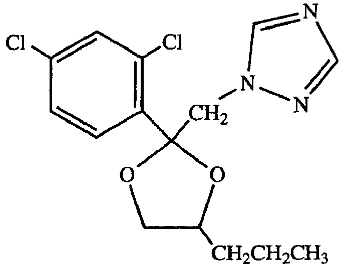

Propiconazole is known in the art and commercially available, e.g. in Biogard PPZ 250 of Beyond Surface Technologies, Switzerland. Propiconazole can be bound to the textile material using a crosslinking agent, in particular a preferably blocked isocyanate compound, which results in urethane bonds, or an acrylate based-product. When using propiconazole, it is preferred to use a crosslinking agent in the liquor, in particular an exhaust liquor. It is even more preferred that the formulation of propiconazole contains the cross linking agent or the cross-linking agent is part of the propiconazole formulation. Furthermore, it is preferred to use propiconazole together with an emulsifier. Propiconazole is particularly effective for killing fungi.

### Combinations of several antimicrobial agents

In preferred embodiments of the invention, at least two, at least three, at least four, or all five of the antimicrobial agents selected from the above-mentioned group consisting of quaternary ammonium organosilane compounds, metal, chitosan, azole-based compounds, and PHMB are adhered to a textile material.

The use of a combination of several antimicrobial agents has the following advantages over the use of a single agent:

First of all, different agents have different antimicrobial effects. Some may work better against bacteria, others against virus, and again other against fungi. Adding a variety of agents increases the spectrum of microbes which can be killed when they come in contact with the antimicrobial textile.

Secondly, the use of a variety of agents can lead to significantly higher killing rates, even for the same organism. It is believed that the higher killing rates are due to synergistic effects between the different agents. The different agents may work synergistically together due to their different killing mechanisms.

Thirdly, the use of different agents may allow binding a higher total amount of agents to the textile. For each of the different agents, there is an inherent limit on the quantity of the agent that can be adhered to the textile in a non-leaching or substantially non-leaching manner. However, even if a textile material has been saturated with and for one agent, there may still be "space" for another agent.

Forth, the use of several agents allows reducing the leaching rates per agent. It is less the total amount of leached substances that will determine the threat to health and environment, but rather the amount per substance. Thus, although the total amount of leached substances may be the same, the leaching values per agent are lower, which is highly beneficial.

Fifth, inherent undesired effects of a substance can be reduced or even counterbalanced by the use of several agents. For example, organosilane is hydrophobic by nature, which is an undesired property for many applications of textiles. For such applications, the concentration of organosilane should be kept at a minimum.

Sixth, some of the preferred agents of the present invention are more expensive than others, e.g. silver cations and chitosan. Reducing the concentrations of these agents and complementing them by other agents allows achieving the antimicrobial performance at substantially lower costs.

These advantages could be shown by the inventors in a series of experiments, some of which are presented in International patent application PCT/EP2016/054245 by the same applicant, and some of which will be presented below.

It is one merit of the invention to have recognized the advantages of using several antimicrobial agents in combination. It is another merit of the invention to have identified several highly effective antimicrobial agents which can be bound to a textile material together, which are relatively inexpensive, and low in toxicity. It is a further merit of the invention to have identified a process, which will be described below, by which many different agents can be applied to a textile material in one and the same liquor application process, be it in one or more application cycles, in a wash-durable or even substantially non-leaching manner. According to the preferred embodiments, wash-durable means that any agents that achieve a property of the textile material are essentially permanently adhered to a textile material, and the property is present even after at least 25 laundry washes in a laundry washing machine at 85±15 °C for 10-15 minutes using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

In some embodiments of the invention, at least one, preferably at least two, more preferably at least three, and most preferably all four selected from the group consisting of metal, azole-based compounds, chitosan, and PHMB are adhered to a textile material. This is the same group as the group of five mentioned above but without quaternary ammonium organosilane. Such a combination is particularly suitable where hydrophilicity is an important property of the treated textile because organosilane renders the textile mildly hydrophobic.

In some embodiments of the invention, at least one, preferably at least two, more preferably at least three, and most preferably all four selected from the group consisting of metal, azole-based compounds, quaternary ammonium organosilane compounds, and PHMB are adhered to a textile material. This is the same group as the group of five mentioned above but without chitosan. Such a combination is particularly suitable where manufacturing costs are an important issue because chitosan is relatively expensive.

In some embodiments of the invention, at least one, preferably at least two, more preferably all three selected from the group consisting of metal, azole-based compounds, and PHMB are adhered to a textile material. This is the same group as the group of five mentioned above but without chitosan and organosilane. Such a combination is particularly suitable where hydrophilicity is an important property of the treated textile and manufacturing costs are an important issue.

In some embodiments of the invention, at least one, preferably at least two, more preferably all three selected from the group consisting of metal, azole-based compounds, and quaternary ammonium organosilane compounds are adhered to a textile material. This is the same group as the group of five mentioned above but without chitosan and PHMB. Such a combination is particularly suitable where the starting textile is substantially made of a synthetic material and comprises no significant amounts of cellulosic material because chitosan and PHMB cannot easily be bound to synthetic textile materials, in particular not in a wash-durable or even substantially non-leaching manner.

In some embodiments of the invention, at azole-based compounds and at least one, preferably at least two, or all least three, selected from the group consisting of metal, PHMB and quaternary ammonium organosilane compounds are adhered to a textile material. The azole-based compounds, in particular propiconazole, are highly efficient against fungi, whereas the other three agents are highly efficient against bacteria. Therefore, azole-based compounds and at least one of the other three agents complement each other well, for manufacturing textiles that are effective against both bacteria and fungi.

### Amounts of antimicrobial agents adhered to the textile material

The ideal percentage of the chemicals present on the textile material has been elaborated in an extensive research and development effort and is in part based on the work discussed in International patent application PCT/EP2016/054245 by the same applicant.

Preferably, the total amount of the one or more antimicrobial agents adhered to the textile material is at most 4.0%, preferably at most 3%, more preferably at most 2.5%, and most preferably at most about 2% on weight fabric of the textile material. These amounts represent the maximum amount of antimicrobial agents the textile can take in, and if higher amounts are applied, leaching of the agents increases significantly. Furthermore, the total amount of the one or more antimicrobial agents adhered to the textile material is preferably at least 0.1%, preferably at least 0.2%, more preferably at least 0.3%, and most preferably at least about 0.4% on weight fabric of the textile material, to ensure high antimicrobial efficiency.

In preferred embodiments, PHMB is adhered to the textile material in an amount of at most 1.5%, preferably at most 1%, more preferably at most 0.8%, particularly at most 0.6%, and most preferably at most about 0.4% on weight fabric of the textile material. Furthermore, the PHMB is adhered to the textile material in an amount of preferably at least 0.1%, more preferably at least 0.2%, even more preferably at least 0.3%, and most preferably at least about 0.4% on weight fabric of the textile material.

In preferred embodiments, metal is adhered to the textile material in an amount of at most 0.1%, preferably at most 0.05%, more preferably at most 0.03%, even more preferably at most 0.02%, and most preferably at most about 0.0085% on weight fabric of the textile material. Furthermore, the metal is adhered to the textile material in an amount of preferably at least 0.0002%, more preferably at least 0.0005%, even more preferably at least 0.001%, and most preferably at least about 0.0017% on weight fabric of the textile material.

In preferred embodiments, azole-based compounds are adhered to the textile material in an amount of at most 2.5%, preferably at most 2.0%, more preferably at most 1.75%, and most preferably at most about 1.25% on weight fabric of the textile material. Furthermore, the azole-based compounds are adhered to the textile material in an amount of preferably at least 0.05%, more preferably at least 0.1%, even more preferably at least 0.15%, and most preferably at least about 0.25% on weight fabric of the textile material.

In preferred embodiments, chitosan is adhered to the textile material in an amount of at most 1.5%, preferably at most 1.0%, more preferably at most 0.7%, in particular at most 0.5%, and most preferably at most about 0.3% on weight fabric of the textile material. Furthermore, chitosan is adhered to the textile material in an amount of preferably at least 0.05%, more preferably at least 0.1%, even more preferably at least 0.2%, and most preferably at least 0.3% on weight fabric of the textile material.

In preferred embodiments, quaternary ammonium organosilane compounds are adhered to the textile material in an amount of at most 2.0%, preferably at most 1.5%, more preferably at most 1.3%, in particular at most 1%, and most preferably at most about 0.72% on weight fabric of the textile material. Furthermore, the quaternary ammonium organosilane compounds are adhered to the textile material in an amount of preferably at least 0.03%, preferably at least 0.05%, more preferably at least 0.10%, and most preferably at least about 0.14% on weight fabric of the textile material. The inventors found that if quaternary ammonium organosilane is applied to synthetic textile materials such as polyester or polyamide, amounts of more than 0.15% decrease the possibility to adhere hydrophilic and/or stain release agents in a subsequent processing step in a wash-durable manner. For cellulosic textiles such as cotton or viscose, the ability to bond hydrophilic and/or stain release agents in a wash-durable manner decreases even at lower amounts. Therefore, the use of quaternary ammonium organosilane is not preferred for cellulosic textiles where hydrophilic and/or stain release agents are to be adhered to the textile as well.

### Antimicrobial efficiency

The one or more antimicrobial agents adhered to the textile material preferably increase the reduction value of the textile material compared to the starting textile material, and/or the treated textile material preferably exhibits a reduction value of Escherichia coli ATCC 25922 and/or Staphylococcus aureus ATCC 6538 and/or Pseudomonas aeroginosa ATCC 15442 and/or Salmonella enterica ATCC 10708 and/or Candida albicans ATCC 10231 and/or Aspergillus niger 16404 measured in accordance with AATCC test method 100-2012 and/or ASTM E2149-10 by/of at least 90% (1 log), preferably at least 99% (2 log), more preferably at least 99.9% (3 log) within 10 minutes of contact time and/or of at least 99% (2 log), preferably at least 99.9% (3 log), more preferably at least 99.99% (4 log) within 1 hour of contact time and/or of at least 99% (2 log), preferably at least 99.9% (3 log), more preferably at least 99.99% (4 log), particularly at least 99.999 (5 log), and most preferably at least 99.9999% (6 log) within 24 hours of contact time.

### Hydrophilic Agents/Stain Release Agents

A great variety of hydrophilic agents/stain release agents can be fixed to a textile by using the process of the invention described below. Like the preferred antimicrobial agents, the hydrophilic agents/stain release agents are preferably non-ionic or cationic, but not anionic.

According to preferred embodiments of the invention, the one or more hydrophilic agents adhered to the textile material comprise at least one selected from the group consisting of carboxylic acid esters, polyester ether copolymers, starch based emulsions, fatty alcohol ethoxylates, and organosilane terpolymers, preferably fatty alcohol ethoxylates or organosilane terpolymers, most preferably organosilane terpolymers.

According to preferred embodiments of the invention, the one or more stain release agents adhered to the textile material comprise at least one selected from the group consisting of fatty alcohol ethoxylates and organosilane terpolymers, preferably organosilane terpolymers. In this case, the one or more or all of the stain release agents can convey hydrophilic properties to the textile as well, which for many applications like sanitary napkins or other hygiene products is particularly advantageous.

Organosilane terpolymer is known in the art and commercially available, e.g. in Hydrosil 8900, of Britacel Silicones Ltd., India. It is based on modified silicone and applicable for both natural (cellulosic) as well as synthetic textiles.

Fatty alcohol ethoxylates are known in the art and commercially available, e.g. in Hydroperm of Archroma, which contains ultra-fine polyester granules and is most suitable for cellulosic textiles, or in Permalose of Croda Chemicals, India, which contains ultra-fine polyurethane granules and is most suitable for synthetic textiles like polyester.

Both organosilane terpolymer and fatty alcohol ethoxylates are in preferred embodiments covalently bonded, or strongly fixed, optionally by using an inbuilt binder, to the textile material such that they can withstand numerous washes.

It is one merit of the invention to have recognized that the usability of many textiles such as sanitary napkins or other hygiene products can be increased by improving the hydrophilic properties of the textiles, which makes the textiles more absorbent. It is another merit of the invention to have recognized that the washability of such textiles can be increased by improving the stain release properties of the textiles. It is another merit of the invention to have identified several highly effective hydrophilic and stain release agents that achieve these properties. It is a further merit of the invention to have identified a process by which hydrophilic/stain release properties and in particular both antimicrobial and hydrophilic/stain release properties can be conveyed to a textile material, be it in one or more liquor application cycles, in a wash-durable or even substantially non-leaching manner. Finally, it is a merit of the invention to have identified combinations and amounts of antimicrobial agents and hydrophilic/stain release agents to be adhered to a textile material such that the hydrophilic/stain release agents do not prevent a satisfactory antimicrobial efficiency of the textile material, and the antimicrobial agents do not prevent satisfactory hydrophilic/stain release properties of the textile material.

### Amounts of hydrophilic agents/stain release agents adhered to the textile material

In preferred embodiments, the hydrophilic and/or stain release agents are adhered to the textile material in an amount of together at most 5%, preferably at most 4%, more preferably at most 3%, even more preferably at most 2%, in particular at most 1.5%, and most preferably at most about 1% on weight fabric of the textile material, and/or in an amount of together at least 0.1%, preferably at least 0.3%, more preferably at least 0.3%, and most preferably at least about 0.4% on weight fabric of the textile material.

Organosilane terpolymers are preferably adhered to the textile material in an amount of at least 0.1%, preferably at least 0.2%, more preferably at least 0.5%, particularly at least 0.7%, and most preferably at least about 1% on weight fabric of the textile material. Fatty alcohol ethoxylates are preferably adhered to the textile material in an amount of at most 3%, preferably at most 2%, more preferably at most 1.5%, even more preferably at most 1%, and most preferably at most about 0.7% on weight fabric of the textile material. The inventors found that when more than 1% o.w.f. of organosilane terpolymers or more than 0.7% (cellulosic textiles) or 0.4% (synthetic textiles) o.w.f. of fatty alcohol ethoxylates are adhered to a textile material, the efficiency of any antimicrobial agents adhered to the textile starts to decrease.

### Efficiency of the hydrophilic agents/stain release agents

In preferred embodiments of the invention, the one or more hydrophilic agents adhered to the textile material reduce the water absorbency time of the starting textile material by at least 20%, preferably at least 40%, more preferably at least 60%, and most preferably at least 80%, preferably when measured in accordance with AATCC test method 79-2014 (Option A). The treated textile material preferably exhibits a water absorbency time of at most 3 seconds, preferably at most 2 seconds, more preferably at most 1 second, and most preferably at most 0.5 seconds, preferably when measured in accordance with AATCC test method 79-2014 (Option A).

In some embodiments, the one or more hydrophilic agents adhered to the textile material preferably do not increase the water repellency of the starting textile material, measured in accordance with AATCC test method 22-2014. The treated textile material preferably exhibits a water repellency rating of at most 50, preferably 0, measured in accordance with AATCC test method 22-2014.

In some embodiments, the one or more hydrophilic agents adhered to the textile material increase the vertical wicking rate of the starting textile material by at least a 10%, preferably at least 20%, and most preferably at least 30%, when tested according to AATCC test method 197-2013. The treated textile material preferably exhibits a vertical wicking rate of at least 0.15 mm/sec, preferably at least 0.20 mm/sec, more preferably at least 0.25 mm/sec, and most preferably at least 0.30 mm/sec, when tested according to AATCC test method 197-2013. The vertical wicking rate is the speed at which liquid travels along or through a textile held vertically.

In some embodiments, the one or more hydrophilic agents adhered to the textile material increase the horizontal wicking rate of the starting textile material by at least 20%, preferably at least 40%, more preferably at least 60% and most preferably at least 100% when tested according to AATCC test method 198-2013. The treated textile material preferably exhibits a horizontal wicking rate of at least 15 mm²/sec, preferably at least 10 mm²/sec, more preferably at least 15 mm²/sec, and most preferably at least 20 mm²/sec, when tested according to AATCC test method 197-2013. The horizontal wicking rate is the change in the area of the liquid with respect to time as the liquid travels through a textile held horizontally.

In some embodiments, the one or more hydrophilic agents adhered to the textile material have the effect that the textile material becomes noticeably more easy to squeeze out than the starting textile material, which is particularly advantageous if the textile is used together with water or other solvents.

In some embodiments, the one or more stain release agents adhered to the textile material increase the stain release rating of the starting textile material by at least one grade, more preferably by at least two grades, particularly at least three grades, and most preferably 4 grades, when tested according to AATCC test method 130-2010. The treated textile material preferably has a stain release rating, measured in accordance with AATCC test method 130-2010, of at least grade 3, preferably at least grade 4, and most preferably grade 5.

In some embodiments, the one or more stain release agents adhered to the textile material increase the stain/oil repellency rating of the starting textile material, measured in accordance with AATCC test method 118-2013, by at most one grade, preferably do not increase the stain/oil repellency. The treated textile material preferably exhibits a stain/oil repellency rating, measured in accordance with AATCC test method 118-2013, of at most grade 1, preferably grade o.

### Starting Textile Material

Generally, any textile material can be used as the starting textile material if it comprises functional groups having the ability to bond one or more antimicrobial agents to the textile material. According to some embodiments of the invention, the starting textile material comprises hydroxyl, peptide and/or carbonyl groups, in particular hydroxyl and/or peptide. These functional groups enable fixing, bonding, attaching or adhering of one or more antimicrobial and/or hydrophilic or stain release agents to the textile material. In exemplary embodiments, the starting textile material comprises peptide and/or hydroxyl groups, in particular hydroxyl groups. According to the preferred embodiments of the invention, the textile material is a cellulosic textile material, a synthetic textile material, or a blend comprising a cellulosic textile material and a synthetic textile material. According to specific embodiments of the invention, the cellulosic textile material comprises cotton, viscose, rayon, linen, hemp, ramie, jute, and combinations (blends) thereof, preferably cotton or viscose or combinations thereof, most preferably viscose.

Examples of embodiments of synthetic textile material include polyester, polyamide (nylon), acrylic polyester, spandex (elastane, Lycra), aramids, modal, sulfar, polylactide (PLA), lyocell, polybutyl tetrachloride (PBT), and combinations (blends) thereof, preferably polyester. Blends of cellulosic and synthetic textile materials comprise between 20% and 80% of cellulosic textile material, preferably between 30% and 75%, more preferably between 50% and 70%, and most preferably about 60%. In specific embodiments the blend of cellulosic textile material and synthetic textile material comprises between 20 and 80% of synthetic textile material, preferably between 25 and 70%, more preferably between 30 and 50%, and most preferably about 40%.

Synthetic textiles are typically stronger and more durable than most textiles made of natural fibers. In addition, it is typically easier to bind chemical substances to a synthetic textile. Counterexamples are the antimicrobial agents PHMB and chitosan, which are difficult to adhere to synthetic textiles. Synthetic textiles can be designed to not wrinkle, and they typically offer a priori stretching and stain release functionality. However, they are typically hydrophobic in nature and hardly biodegradable. Depending on the requirements, these functionalities may also be disadvantageous. Natural textiles are, in turn, fairly biodegradable and hydrophilic in nature. The advantages of synthetic and natural textiles tend to be opposing. Wrinkling, stain release and hydrophobic/hydrophilic properties may be mentioned as examples.

The aforementioned textile materials may be textile material selected from the group consisting of woven, knitted, warp knitted, crocheted, and non-woven fabrics. The textile material is preferably a raised fabric, e.g. a looped fabric (such as a terry fleece or a polar fleece) or a fabric having a peach finishing or a brushed finishing. Wipes with textiles which did not undergo a raising process may leave traces, which may lead to a quick repopulation given the reproduction rate of microbes.

The skilled person understands that several of the components listed above may be combined and thereby form the starting textile material to be finished with process 10 described subsequently.

In general, any starting textile material can be processed with said process 100, wherein the textile material is a fiber, preferably a yarn or a fabric, and most preferably a fabric. In case the textile material is a fabric, it can generally have any specific weight, or fabric weight, such as e.g. 100, 200 or 300 g/m² (GSM)

### Finishing Process

### Process Cycles

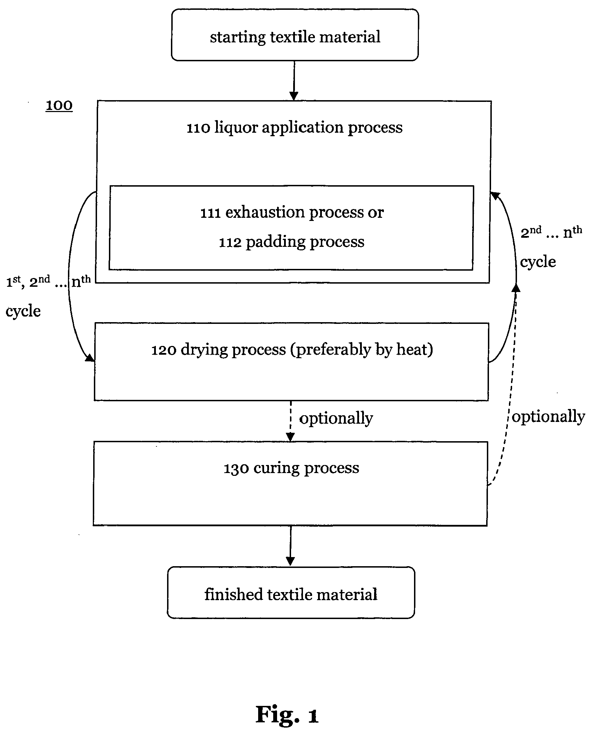

The preferred process of finishing a textile material according to the present invention comprises one or more process cycles 100 of adhering antimicrobial and/or hydrophilic/stain release agents to a textile material. Each process cycle 100, exemplarily shown in Fig. 1, can be divided into at least two process steps, a first process step 110 and a second process step 120. The first process step is a liquor application process. The liquor application process is followed by the second process step 120, in which the textile material is dried, preferably by subjecting it to a heat treatment. Optionally, the step of drying is followed by a third process step 130, in which the textile material is cured. At least the last cycle of the process of finishing a textile material according to the present invention should comprise the third step 130 of curing.

### Liquor Application Process

A liquor is a liquid containing chemicals to be applied to a textile. In the present invention, the liquor comprises one or more antimicrobial agents and/or one or more hydrophilic/stain release agents. A liquor application process is any process by which the textile is brought in contact with the liquor to treat the textile with the chemicals. The liquor application process 110 shown in Fig. 1 comprises preferably an exhaust process 111 or a padding process 112.

Preferably, the liquor has a pH-value of at most 6.9, preferably at most 6.5, more preferably at most 6.3, in particular at most 6.0, and most preferably at most about 5.5, and/or a pH-value of at least 3.0, preferably at least 3.5, more preferably at least 4.0, even more preferably at least 4.5, in particular at least 5.0, and most preferably at least about 5.5.

The liquor contains a solvent, preferably water, and the agents and the solvent preferably form a homogenous mixture, and in particular do not form a slurry or dispersion. The antimicrobial and/or the hydrophilic/stain release agents and preferably all other components in the liquor are preferably dissolved in the liquor, in particular are they not dispersed in the liquor, and/or the liquor is substantially free of solids.

The dynamic viscosity of the liquor at 20 °C and/or 80 °C, in centipoise (cP), is preferably at most 20% higher than the dynamic viscosity of water at 20 °C and/or 80 °C, respectively, preferably at most 10%, more preferably at most 5%, particularly at most 2%, and most preferably at most about 0% higher. The amount of latex in the liquor is preferably at most 10 gpl, preferably at most 5 gpl, more preferably at most 2 gpl, even more preferably at most 1 gpl, and most preferably o.

The amount of cyclodextrin and/or inclusion complexes, in particular inclusion complexes of fiber-reactive cyclodextrin derivatives and antimicrobial agents is preferably at most 10 gpl, more preferably at most 5 gpl, even more preferably at most 2 gpl, particularly at most 1 gpl, and most preferably o. The amount of dye in the liquor is preferably at most 10 gpl, more preferably at most 5 gpl, even more preferably at most 2 gpl, particularly at most 1 gpl, and most preferably o.

### Exhaustion

As is known in the art, during an exhaust process (see e.g. step 111 in Fig. 1), a textile material is brought in contact with a liquor which comprises ingredients which are transferred to the textile material during the exhaust process. This can be achieved by guiding the textile material through a container filled with the liquor. Yarn and woven fabrics are typically treated with exhaust processes. During a common exhaust process, chemicals to be applied to a textile material are dissolved or dispersed in a solvent, e.g. water, according to the required material to liquor ratio, which describes the ratio between the weight of the textile to be treated and the weight of the liquor. For example, if the desired material to liquor ratio is 1:2, there would be 600 kg of liquor for 300 kg of textile material to be exhausted. The textile material is brought in contact with the liquor, for example by immersing it into the liquor, whereby the chemicals preferably contact the fibers and more preferably enter the fibers. For obtaining proper diffusion and penetration of the chemicals in the fiber, a respective liquor temperature and respective exhaustion time are set, such that kinetic and thermodynamic reactions take place as desired. As the textile material and its fibers absorb the chemicals, the concentration thereof in the liquor decreases. As is known in the art, the degree of liquor exhaustion as a function of elapsed time is termed extent of the exhaust process. The percentage of the chemicals initially present in the liquor which is exhausted onto the textile at the end of the process is called exhaustion rate or exhaust rate. Preferably, the exhaustion rate of the exhaust process is at least 90%, preferably at least 95%, more preferably at least 98%. This exhaustion rate allows for reducing costs, as most of the ingredients of the liquor are exhausted by the textile material. It is also more ecological than processes with lower exhaustion rates.

The exhaust process 111 shown in Fig. 1 may be performed by any suitable technique, and on any suitable machine, like a yarn dying machine, a beam machine, a winch machine, a jet-dyeing machine, a continuous dyeing range (CDR), continuous bleaching range (CBR), or a jigger machine. In a jigger machine, an open-width fabric revolves on two main rollers. The fabric passes from one roller through the liquor bath at the bottom of the machine and then onto a driven take-up roller on the other side. When all the fabric has passed through the bath, the direction is reversed. Each passage is called an end. The process typically involves an even number of ends. The liquor bath has one or more guide rollers around which the cloth travels. During the immersion, the desired contact with the process liquor is achieved. When passing through the liquor bath, the fabric picks up an adequate quantity of liquor, excess of which is drained out, but still a good quantity is held in the fabric. During rotation of the rollers, the chemicals contained in the liquor penetrate and diffuse into the fabric. The largest part of the diffusion process takes place not in the liquor bath but when the fabric is on the rollers, since only a very small length of fabric is in the liquor bath at a given time, and the major part is on the rollers. Jigger machines are preferred because they are very economical and because they can be used with a high material to liquor ratio.

The exhaust process 111 shown in Fig. 1 allows for evenly spreading the liquor across the entire cross section of the textile material, such that preferably no spot of the textile material is left untouched by the liquor. As a result, interactions and/or bonds may be created between the textile material and one or more agents at this time. Preferably, most of the one or more agents of the liquor are exhausted evenly onto the entire cross section of the textile material.

In general, more heat on the fabric is better for bonding. Therefore, preferably, the temperature of the liquor during the exhaust process is sufficiently high and the exhaust time is sufficiently long such that the one or more agents in the liquor are substantially uniformly dispersed across the cross section of the textile material as a result of the exhaust process. Thus, the temperature of the liquor should be sufficiently high and the exhaust time should be sufficiently long such that preferably the textile material is well impregnated and the one or more agents are dispersed throughout the entire textile material. Preferably, the exhaust time is sufficiently long and the temperature of the liquor during the exhaust process is sufficiently high such that the textile material can achieve the desired performance after a respective curing process, as will be outlined below.

However, too much heat causes yellowness and weakens the fabric. Therefore, preferably, the temperature of the liquor during the exhaust process is sufficiently low and/or the exhaust time is sufficiently short such that the textile material does not discolor and/or turn yellow and/or its breaking (tensile) strength is not reduced by more than 15%, preferably not more than 10%, more preferably not more than 7%, and most preferably not more than 5%, as a result of the exhaust process. At too high temperatures, too much steam forms, reducing the efficiency of the process. Furthermore, if the temperature of the liquor is too high, turbulences can occur within the liquor bath and the textile material may get harmed.

The term exhaust time when used in the context of the present invention is preferably defined as the period starting when at least part of the entire batch of textile material first comes into contact with the liquor and lasting until the last part of the batch is taken out of the liquor. For a given application, the ideal exhaust time can vary significantly. In case the textile is a fabric, it will depend on the type of machine, the size of the liquor bath, and the length and weight of the fabric. For example, if the ideal exhaust time for a fabric of a length of 1,500 meters is 60 minutes, the ideal exhaust time for a fabric of a length of 3,000 meters may be 100 minutes under otherwise identical conditions. Whenever an exhaust time is specified herein, it refers to the time which is equivalent to the exhaust time of a fabric of 1,500 meters in length and 200 g/m² in weight on a standard jigger machine (e.g. model number Y1100 manufactured by Yamuda) being operated at a standard fabric speed (e.g. 50 meters/minute). For any given textile material and exhaustion machine, the skilled person, using common general knowledge, will be able to determine the exhaust time which is equivalent to an exhaust time specified for the above-mentioned parameters.

The breaking strength may be measured with any suitable technique, and is preferably measured in accordance with ASTM standard D 5035-11 (in case the textile material is a fabric), or in accordance with ASTM standard D 2256/D 2256M-10e1 (in case the textile material is a yarn).

The inventors found that the preferred temperature of the liquor during the exhaust process and the exhaust time is substantially independent of the weight and the type of the textile material, and of the agents in the liquor. This is because the ideal exhaust process parameters are determined by the way textiles, in particular multifilament yarns and fabrics, behave in general.

In the preferred embodiments, the liquor has a temperature of at least 45 °C, preferably of at least 50 °C, more preferably of at least 60 °C, particularly of at least 70 °C, and most preferably of at least about 80 °C. Furthermore, the liquor has a temperature below boiling temperature, preferably of at most 95 °C, more preferably of at most 90 °C, particularly of at most 85 °C, and most preferably of at most about 80 °C. The exhaustion process time is preferably at most 90 min, preferably at most 80 min, more preferably at most 70 min, and most preferably at most about 60 min. Furthermore, the exhaustion process time is preferably at least 45 min, preferably at least 50 min, more preferably at least 55 min, and most preferably at least about 60 min. As shown in International patent application number PCT/EP2016/054245 by the same applicant, when a textile is treated at a temperature of 80 °C for 60 minutes, it expands and opens up, exposing individual fibers so that the agent can reach even the most remote spot, and there is even dispersion of the agents. Accordingly, different textile materials can easily be treated by means of the exhaust process 111 shown in Fig. 1 without having to change parameters of the exhaust process, while still obtaining the best possible results.

Preferably, during the exhaust process 111 shown in Fig. 1, the liquor is stirred. The stirring should be performed at intervals, in other words, the stirring is performed regularly during the exhaust process with interruptions. It will be appreciated that other suitable intervals may preferably be set, depending on the specific application. Ideally, the stirring is performed continuously during the exhaust process. This intermixing of the chemicals in the exhaust bath increases reliability of the exhaust process, as one or more agents are more evenly distributed in the bath and as a result, a product with even quality throughout the entire textile material can be obtained. Preferably, the stirring is performed by means of a circulation pump, which circulates the liquor inside the exhaustion bath and which is typically comprised by a conventional exhaustion machine. The stirrer used by the inventors is a simple mixer, which is similar to but larger than a standard household mixer. The stirrer was added by the inventors to the exhaustion machine they used as it is not provided by conventional exhaustion machines. Most preferably, the liquor is stirred by means of both a circulation pump and a stirrer. Due to this extensive mixing of the liquor, the exhaust process is supported and one or more agents are well dispersed across the cross section of the textile material during the exhaust process. As is known in the art, an exhaust process is typically applied for dyeing cloth, for example. In such applications, typically only a circulation pump is applied for ensuring proper fluid characteristics of the bath, such that a homogeneous dispersion of the dyeing molecules is present in the bath. However, since the one or more agents used in the context of the present invention can be less soluble in water compared to dyeing agents, the utilization of both a stirrer and a circulation pump assures that the agents are not undissolved and do not settle at the bottom of the bath. Instead, due to the combination of both stirring means, the agents are uniformly and homogeneous dispersed throughout the bath.

Accordingly, with exhaust process 111 shown in Fig. 1, agents are substantially uniformly dispersed across the cross section of the textile material, whereby the textile material itself, advantageously, does not yellow and essentially does not lose its breaking strength.

### Padding

Any suitable technique can be utilized for performing a padding process 112 shown in Fig. 1, in which preferably a respective liquor is prepared and fed through a pump to a respective padding mangle. Accordingly, padding process 112 shown in Fig. 1 preferably comprises applications of one or more rolls to obtain optimum wet pickup of the liquor on the textile material. According to a preferred embodiment, the textile material passes through a multiple trough padding mangle, or a continuous dyeing or bleaching range. The liquor may be at room temperature or it may be heated during the padding process.

The appropriate padding mangle pressure is typically predetermined, depending on the quality of the textile material, and it is in general set such that the pick-up rate (or "wet pick-up") of the agents is optimized. The pick-up rate specifies the amount of liquor applied and is defined as a percentage on the weight of the dry untreated textile as follows: % pick-up rate = weight of liquor applied x 100 / weight of dry textile. For example, a pick-up rate of 65% means that 650 grams of liquor are applied to 1 kg of textile. Preferably, a liquor pickup rate of the padding process is at least 30%, preferably at least 40%, more preferably at least 50%, particularly at least 60% or at least 80%, and most preferably at least about 100%, and/or at most 140%, preferably of at most 130%, more preferably of at most 120%, particularly at most 110%, and most preferably of at most about 100%. However, if the textile is already to a certain extent saturated with agents before the padding process is applied, e.g. because another process cycle has been performed previously, it is believed that the effective pick-up rate for the agents is only about 70% of the nominal pick-up rate mentioned above, in the sense that the rest of the agents padded onto the fabric does not become permanently fixed to the fabric.

### Drying

Any suitable technique can be utilized for performing the drying process step 120 shown in Fig. 1. Drying is, however, preferably performed by subjecting the textile material to a heat treatment. The heat treatment is preferred because it is more efficient for various reasons: accelerated manufacturing, i.e. shortened residence time of the textile material in the production chain and lean process management without removal for drying purposes and re-insertion of the textile material in the processing chain may be mentioned as examples. Furthermore, the textile should be dried by a heat treatment before it is washed. This is because the heat treatment will achieve basic bonding of the agents to the textile so that they will not immediately be washed out during the washing process.

After drying process 120 by heat treatment, the textile material should be 99% devoid of moisture. However, when the textile cools down to room temperature, it will have a moisture regain of, e.g., about 7-8% for cotton and of about 4-5% for polyester.

Preferably the drying is conducted at least partially at an ambient temperature of at least 70 °C, preferably at least 100 °C, more preferably at least 110 °C, most preferably at least about 120 °C. Lower temperatures would require longer dwell time, which is disadvantageous because a longer dwell time has a negative impact on the textile in terms of yellowing and also on the strength of the fabric. Furthermore, the drying is preferably conducted at an ambient temperature of at most 160 °C, preferably of at most 140 °C, more preferably of at most 130 °C, and most preferably of at most about 120 °C.

Preferably, the drying time at the temperatures given above is of at least 30 seconds, preferably at least 40 seconds, more preferably at least 50 seconds, and most preferably at least about 60 seconds, per 100 g of fabric weight per m² (in case the textile material is a fabric). Further preferably, the drying is performed over a period of at most 120 seconds, preferably at most 90 seconds, more preferably at most 75 seconds, most preferably at most about 60 seconds, per 100 g of fabric weight per m² (in case the textile material is a fabric). It will be appreciated that the drying times increase with increasing fabric weight (per m²). The skilled person understands that similar drying times apply if the textile material is a yarn, and understands to choose respective drying times which then depend on the yarn diameter.

Drying process 120 is typically conducted by passing the textile material through a stenter or stenter frame (sometimes also referred to as a "tenter") or similar drying machine. By drying the textile material, preferably excess moisture is removed.

### Curing

A curing process in the context of the present invention (see e.g. 130 in Fig. 1) is essentially a heat treatment process applied to a textile material for inducing physical cross-linking. Curing can only be performed once the textile is dry because the temperature of the textile cannot exceed 100 °C until the water in the textile is evaporated.

The curing temperature is preferably sufficiently high and the curing time is preferably sufficiently long such that one or more agents applied to (preferably exhausted and/or padded onto) the textile material are sufficiently strongly fixed or bonded to the textile material. They should preferably be set such that the agents are bound to the textile material and optionally polymerized, become an inherent part of the textile material and provide the desired properties of the textile material in a wash-durable or even non-leaching manner. Depending on the agents and chemicals used, also a large part of the crosslinking of the agents takes place during the curing step. In case the textile material is a fabric, the curing time depends on the weight of the fabric (per m²). However, the inventors found that the preferred curing temperature, which will be detailed below, is substantially independent of the type of the textile material.

Furthermore, the curing temperature is sufficiently low and the curing time is sufficiently short such that the textile material does not discolor and/or turn yellow, and/or its breaking strength is not significantly reduced. Further preferred, the curing temperature is sufficiently low and the curing time is sufficiently short such that the textile material does not melt and/or burn and/or yellow, and/or that the colors of the textile material do not substantially change (discolor) as a result of the curing.

In the preferred embodiments, the curing process is conducted at least partially at an ambient curing temperature of at least 140 °C, preferably at least 160 °C, more preferably at least 170 °C, particularly at least 175 °C, and most preferably at least about 180 °C. Preferably, the curing process is conducted at an ambient temperature of at most 200 °C, more preferably at most 190 °C, even more preferably at most 185 °C, and most preferably at most about 180 °C. Thus, in the most preferred embodiment, the maximum curing temperature is 180 °C, independent from the textile material treated with process 100.

In particular where the textile material is a fabric of less than 350 grams per m², curing takes place at the above stated curing ambient temperatures over a period of preferably at least 30 seconds, preferably at least 40 seconds, more preferably at least 50 seconds, most preferably at least about 60 seconds, and preferably over a period of at most 120 seconds, preferably at most 90 seconds, more preferably at most 80 seconds, particularly at most 70 seconds, most preferably at most about 60 seconds.

Where the textile material is a fabric between 350 and 500 grams per m², curing takes place at the above stated curing ambient temperature over a period of preferably at least 45 seconds, preferably at least 60 seconds, more preferably at least 75 seconds, most preferably at least about 90 seconds, and preferably over a period of at most 180 seconds, preferably at most 160 seconds, more preferably at most 140 seconds, particularly at most 120 seconds, most preferably at most about 90 seconds.

Where the textile material is a fabric of at least 500 grams per m², curing takes place at the above stated curing ambient temperature over a period of preferably at least 60 seconds, preferably at least 75 seconds, more preferably at least 90 seconds, most preferably at least about 120 seconds, and preferably over a period of at most 240 seconds, preferably at most 210 seconds, more preferably at most 180 seconds, particularly at most 150 seconds, most preferably at most about 120 seconds.

The preferred process parameters for curing (at 180 °C during the curing times mentioned above) were obtained in an extensive research and development campaign performed by the inventors and described in International patent application PCT/EP2016/054245 by the same applicant.

The curing process 130 is preferably carried out in a pass through a stenter. Referring to Fig. 1, a drying process 120 following a liquor application process may be directly followed by a curing process 130. In this case, the drying process 120 and the curing process 130 is preferably carried out together with one single pass through the stenter, preferably with a temperature ramp-up and ramp-down procedure as described in International patent application PCT/EP2016/054245 by the same applicant. Furthermore, where the textile material is a fabric, drying and curing of the textile material are performed at the above stated drying/curing ambient temperatures over a period of together at least 45 seconds, preferably at least 50 seconds, more preferably at least 55 seconds, most preferably at least about 60 seconds, per 100 grams of fabric weight per square meter, and preferably at most 75 seconds, preferably at most 70 seconds, more preferably at most 65 seconds, most preferably at most about 60 seconds, per 100 grams of fabric weight per square meter.

### Putting the cycles together

The subsequent sections are dedicated to the description of the preferred and most preferred embodiments based on the above stated elementary building blocks specifying the starting textile materials, composition of liquors as well as processing parameters including concentrations of agents, temperatures and processing times at standard pressure.

Exhaustion is preferred over padding in the first process cycle if the starting textile material is a multifilament yarn or a fabric made out of them, which is preferred for most applications because multifilament textiles are stronger, have a higher surface area, and can be blended. In an exhaust process, the multifilament textile opens up and the fibers are individually exposed to penetration by the agents. Thus, by use of an exhaust process, the agents can diffuse into the fibers and do not occupy the surface space of the fibers to the same extent as it is the case in more superficial liquor application processes like padding or spraying.

The use of an exhaust process in the first process cycle is particularly advantageous in cases where the first process cycle is followed by a further process cycle. The use of an exhaustion process in the first process cycle allows to improve the performance or convey another property by a second process cycle, in particular by a second process cycle in which a padding process is used. E.g., the inventors found in experiments, whose results are not reproduced herein, that the antimicrobial performance and/or the wash-durability thereof can be increased if the textile material is treated with an antimicrobial liquor application process in each of two process cycles. In this case, the first antimicrobial liquor application process is preferably an exhaust process, and the second liquor application process is preferably a padding process. The two antimicrobial cycles can then be followed by one or more cycles in which a hydrophilic/stain release liquor application process is performed.

In contrast, repeated superficial liquor applications like repeated padding applications will not improve performance, or at least not to the same extent, and it will be more difficult to impart another property in the second cycle. Furthermore, the inventors found that leaching is at lowest values only when exhaustion is used in the first process cycle. On the other hand, in the case of non-woven fabrics, padding is preferred because non-woven fabrics can oftentimes not withstand the forces applied by exhaustion machines like jiggers.

For embodiments which make use of more than one processing cycle, padding is preferred for all subsequent cycles, independent of the starting textile material. This is because once a textile has been exhausted with agents in a first process cycle, the interior of the fibers is saturated at least to a certain degree, and space for more agents to be adhered to the textile material will be found primarily on the fiber surface. Furthermore, padding is less time consuming and therefore less costly than exhaustion.

Curing, which consumes large amounts of energy and decreases the breaking strength of the textile considerably, is preferably only performed in the last cycle. This is in general sufficient for bonding even the agents applied in the previous cycles to the textile. Some embodiments may comprise a washing step at the end of one or more or even all cycles, to obtain particularly low leaching values (for a detailed description see International patent application PCT/EP2016/054245 by the same applicant). However, this may not be necessary for most of the preferred applications disclosed herein, such as wipes and other sanitary products. Where washing is carried out after the drying step, in particular without or before curing, the drying should be performed with a heat treatment, preferably at least at the above-specified temperatures for drying. The heat treatment will ensure that there is a basic bonding between the textile and the agents applied in this cycle, which prevents washing out of these agents in the washing step.

As a rule, where both antimicrobial and hydrophilic/stain release agents are applied to the starting textile material, antimicrobial and hydrophilic/stain release agents are, on the one hand, separately applied, i.e. in separate cycles. On the other hand, the antimicrobial agents are applied in one or more cycles prior to the cycle in which hydrophilic/stain release agents are applied, or applied for the first time. The inventors found that it is difficult, if not impossible to apply antimicrobial agents and hydrophilic/stain release agents in the same liquor in a wash-durable manner, or to apply antimicrobial agents in a wash-durable manner after hydrophilic/stain release agents have been applied to the textile. Metal, in particular silver is an exception to this rule, as it may be applied together with hydrophilic/stain release agents in the same liquor, i.e. in the same cycle. However, it should not be applied after the application of hydrophilic/stain release agents, as the pores of the textile would be blocked and bonding would be severely hampered.

A first group of embodiments of the present invention comprises the finishing of starting textile materials by adhering only antimicrobial agents to them but no hydrophilic/stain release agents, in one or more cycles of the finishing process of the present invention. A second group of embodiments comprises the finishing of starting textile materials by adhering only hydrophilic/stain release agents to them but no antimicrobial agents, in one or more cycles of the finishing process of the present invention. However, the most preferred group of embodiments of the present invention comprises the application of both antimicrobial agents and hydrophilic/stain release agents, in two separate cycles.

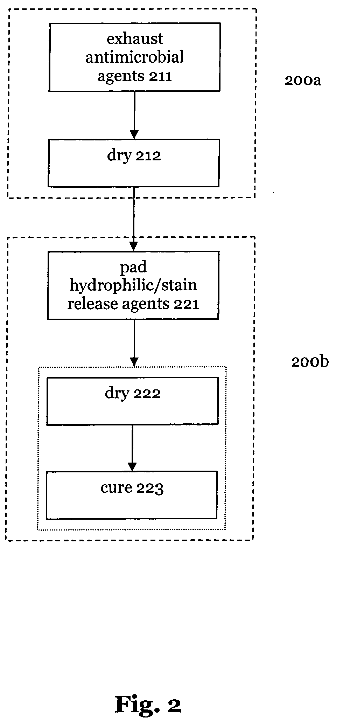

The preferred process of adhering both antimicrobial agents and hydrophilic/stain release agents to a textile material will now be described with reference to Fig. 2. In a first cycle 200a, the starting textile material, preferably a woven fabric, is subjected to an exhaustion process 211, wherein the liquor comprises one or more antimicrobial agents dissolved in water, with the pH-value adjusted as specified above. The selection and the concentration of the antimicrobial agents in the liquor are such that the exhaustion process 211 results in a textile material to which the agents as specified above are adhered in the combinations and the amounts as specified above. Exhaustion 211 is performed at standard pressure with an exhaustion temperature of 80 °C for 60 minutes while the liquor is stirred. The first process cycle 200a is finished by drying 212 as described above, at 120 °C.

In a second cycle 200b, the textile material is subjected to a padding process 221, wherein the liquor comprises hydrophilic/stain release agents dissolved in water, with the pH-value again adjusted as specified above. The selection and the concentration of the hydrophilic/stain agents in the liquor are such that the padding process 221 results in a textile material to which the agents are adhered in the combinations and the amounts as specified above. Padding 221 is performed at both standard pressure and temperature, with a liquor pickup rate of 100%. The second process cycle is finished by drying 222 followed by curing 223 at 180 °C, wherein drying and curing are performed in one single pass through a stenter, which provides the finished textile material.

It will be appreciated that one or more additional process steps or cycles may be introduced between the individual process steps or cycles of process 100 of Fig. 1. Furthermore, one or more additional process steps or cycles may be performed prior to or after performing process 100 of Fig. 1. For example, before the start of process 100 with the liquor application process 110, the textile material should be tested, washed and/or cleaned. Preferably, the fabric is first tested and if necessary washed or cleaned, so that the fabric is free from all chemical contaminants that would hinder the application of the chemistry to the textile. In a particularly preferred embodiment, one or more of the following steps may be performed prior to conducting process 100 of Fig. 1: Testing the textile material at laboratory scale to verify and confirm that it meets respective selection criteria, batching and stitching together of individual textile pieces on a frame, inspecting the textile material thoroughly for defects, ensuring that the fabric is free from any chemical contaminants.

The textile material may be dyed prior to performing process 100. In some embodiments, the textile material is manufactured to be multifunctional. After having performed process 100, i.e. after the antimicrobial and/or hydrophilic/stain release treatment, a respective multifunctional treatment is performed. With such a multifunctional treatment, the textile material may be provided e.g. with water-and-oil-repellent properties, as will be discussed below, and/or other properties. It is also possible to conduct a multifunctional treatment in a padding process 112, wherein the padding liquor contains the respective functional agents, in addition to antimicrobial or hydrophilic/stain release agents.

It will be appreciated that different machines may be utilized in case the textile material is a yarn. For example, the exhaustion process may be performed with a pressurized yarn dyeing machine, and the yarn may then be treated with a hydro extractor for removing excess moisture. The drying and curing of the yarn may take place in a Radio Frequency RF Dryer and curing machine. The dwell times thereby depend on the yarn diameter, wherein the temperatures mentioned above still apply.

### EXAMPLES

A series of different textile fabrics and materials were finished and tested according to the test protocols stated herein. The finishing process and the test results as well as the references to the underlying test protocols are summarized in the tables of Fig. 11 to Fig. XX. All finished textile fabrics and materials were treated with antimicrobial agents.

The finishing processes are summarized on the upper side of each table under the section "application process". Fabrics were produced under laboratory conditions closely simulating the finishing processes described below.

### Chemicals

Chemicals used for the examples and mentioned in the tables comprise the following:
"Texguard 20" of Swissol Specialties Pvt. Ltd, India A: a solution containing 20% PHMB;
"Biogard PPZ" (=Biogard PPZ 250) of Beyond Surface Technologies, Switzerland: a solution containing 25% of propiconazole;
"Silvadur 930" of Dow Chemical Company, USA: a solution containing 0.17% of silver cations trapped in a matrix;
"AEM 5772" of Aegis, USA: a solution containing 72% of quaternary ammonium organosilane compounds;
"Chitosan 102" (=Goyenchem-102) of Go Yen Chemical, Taiwan: a solution containing 15% of chitosan;
"Hydrosil" (= Hydrosil 8900) of Britacel Silicones Ltd., India: a solution containing 30% organosilane terpolymer;
"Permalose" of Croda Chemicals, India: a solution containing 12% of fatty alcohol ethoxylates.

Recipes are stated in the figures. Percentages refer to the weight of the chemicals relative to the weight of the textile material ("o.w.f.") if not otherwise stated. The percentages of active agents which were used can be calculated with the concentration of actives in the chemicals as specified above. E.g., 3% Texguard 20 means that 20% x 3% = 0.6% o.w.f. of PHMB were used. The expression "gpl" refers to the amount of a chemical in a liquor. Again, the amount of actives can be calculated with the concentration of actives in the chemicals as specified above. E.g., 50 gpl Hydrosil means that 30% x 50 gpl = 15 gpl of organosilane terpolymer were comprised in a liquor. Other units are explicitly stated in each table.

### Test protocols

The underlying test protocols are generally publically available from the American Association of Textile Chemists and Colorist (AATCC) and the American Society for Testing and Materials (ASTM).

All microbiological tests were performed with cultures of the bacteria *Escherichia coli* American Type Culture Collection (ATCC) 25922, *Staphylococcus aureus* ATCC 6538, *Pseudomonas aeruginosa* ATCC 15442, *Salmonella enterica* ATCC 10708, and of the fungi *Candida albicansATCC* 10231, and *Aspergillus Niger* ATCC 10231.

### Working Examples

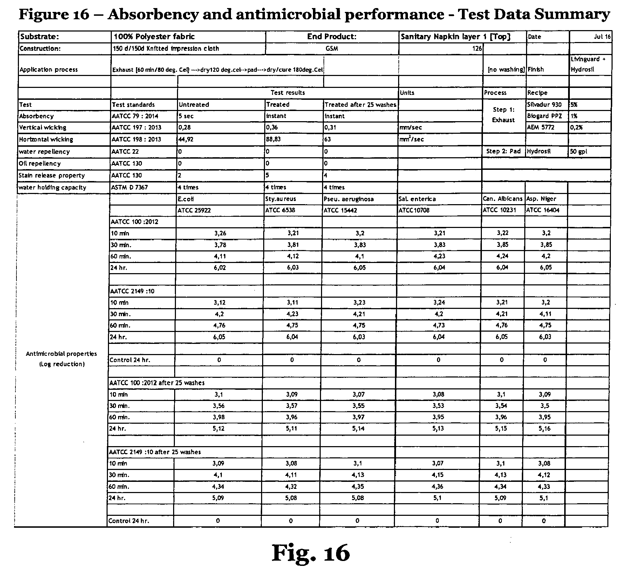

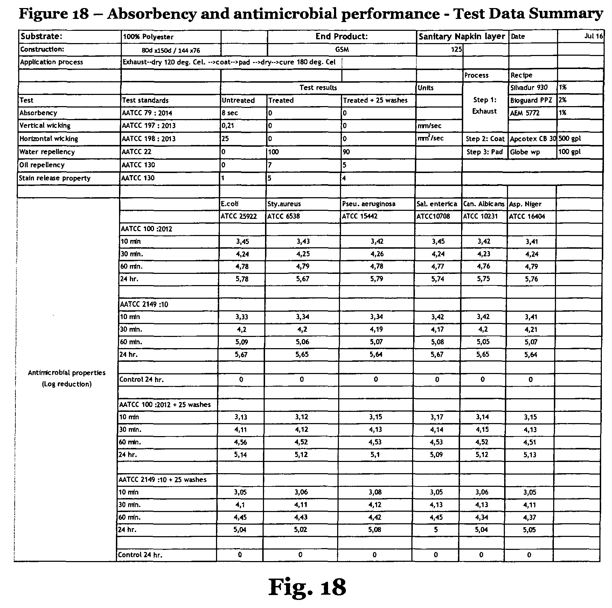

Fig. 16 to 18 summarize the starting textile materials, the finishing processes and recipes used to produce different Working Examples according to preferred embodiments of the invention.

### Working Example 1 - Transportation layer for sanitary napkin

Working Example 1 will now be described with reference to the table in Fig. 16. This example can be used as a transportation layer in a sanitary napkin as will be described below.

The starting textile of the Working Example 1 was a 100% polyester fabric, construction 150d / 150d knitted impression, width 150 cm, fabric weight 126 g/m².

In a first process cycle, the fabric was exhausted and then dried. In a second process cycle, the fabric was padded, then dried again and cured.

In the exhaustion process, 300 kg (approx. 1.500 meters) of starting textile material were loaded on a jigger machine (Yamuna, model number Y1100). Into about 600 liters of water (material to liquor ratio of about 1:2), 15 kg (5% of the weight of the textile material) of Silvadur 930, resulting in an amount of 0.0085% silver o.w.f., 3 kg (1% of the weight of the textile material) of Biogard PPZ, resulting in an amount of 0.25% propiconazole o.w.f., 0.6 kg (0,2% of the weight of the textile material) of AEM 5772, resulting in an amount of 0.14% quaternary ammonium organosilane o.w.f. The pH of the liquor was adjusted with 0.03 gpl of citric acid and maintained between pH 5 and pH 6, preferably at pH 5.5. The temperature of the liquor was set to 80 °C.

The jigger machine was started and run at a speed of 50 m/s, and the run was continued for the next 60 minutes (2 ends, with a break of less than 30 seconds between the ends). The liquor was constantly stirred with a stirrer at a speed of 300 rpm throughout the exhaustion process. The exhaustion rate was about 98%. Following this, the process bath was drained and the textile material was immediately transported to a stenter machine for drying. Thus, the exhaust time was 60 minutes.

The textile was dried by passing it through the stenter, which had 8 chambers and a length of 24 meters, at a speed of 20 centimeters per second. The maximum temperature of 120 °C was applied in all 8 chambers, i.e. during 120 seconds.

Following this first process cycle, a second process cycle with a padding process was performed to apply a hydrophilic/stain release agent: To about 400 liters of water, 20 kg (50 gpl) of Hydrosil were added, resulting in a concentration of 15 gpl organosilane terpolymer in the liquor. During the padding process, the liquor had a temperature of 30 °C. The padding mangle pressure was 2 bar. The nominal pick-up rate was 100%. Estimating that, after the first process cycle, the effective pick-up rate in a padding process of subsequent process cycles is about 70% of the nominal pick-up rate, the amount of organosilane terpolymer adhered to the textile material was about 1.05% o.w.f.

The textile material was then dried and cured for in total 2 minutes in a single pass through the stenter, at a maximum temperature of 180 °C. The maximum curing temperature was applied for 60 seconds (in 4 of the 8 chambers of the stenter).

Different tests regarding hydrophilicity, stain release capability, and antimicrobial efficiency were performed on the untreated starting textile material and on the finished textile of Working Example 1, the results of which are summarized in the table of Fig. 11.

With regard to hydrophilicity, the treated fabric exhibits essentially instant water absorbency, while the untreated fabric exhibits a water absorbency time of 5 seconds. Both were measured in accordance with AATCC test method 79-2014 (Option A). The vertical wicking rate of the treated textile increased slightly, from 0.28 to 0.36 mm/s, measured in accordance with AATCC test method 197-2013. However, the horizontal wicking rate increased significantly from 44,92 to 88.83 mm²/s, measured in accordance with AATCC test method 198-2013. The water repellency rating measured in accordance with AATCC test method 22-2014 was 0 both before and after finishing the textile. After 25 laundry washes (washes as described above), the hydrophilicity had slightly decreased. For example, the horizontal wicking rate had decreased to 63 mm²/s. However, water absorbency time was still instantaneous. The wash-durability of the increased hydrophilicity imparted by the finishing process is therefore satisfactory.

Both the starting textile material and the finished textile had an oil repellency rating of grade 0 when measured in accordance with AATCC test method 130-2013, which means that both absorb oily stains well. However, the finishing of the textile had the effect that the stain release capability increased from the low grade of 2 to the highest grade of 5 as measured in accordance with AATCC test method 130-2010. After 25 laundry washes, the stain release capability had slightly decreased, to grade 4. The wash-durability of the improved stain release properties imparted by the finishing process is therefore satisfactory.

While the starting textile material did not exhibit any measurable antimicrobial properties, the finished textiles were highly antimicrobial, and the imparted antimicrobial properties proved to be sufficiently wash-durable. The results following the AATCC 100-2012 and the AATCC 2149:10 test methods of the unwashed textiles showed that the treated fabric is capable of reducing all of the above-mentioned organisms by more than 99,9% (3 log) within 10 minutes of contact time. With increasing contact time (up to 24 hours), microbial reduction values increased up to more than 99,9999% (6 log) for all microbial cultures. After 25 washes as described above, the treated fabric still exhibited microbial reduction values of more than 3 log for all organisms within 10 minutes of contact time, and more than 5 log within 24 hours of contact time.

### Working Example 2 - Absorption layer for sanitary napkin

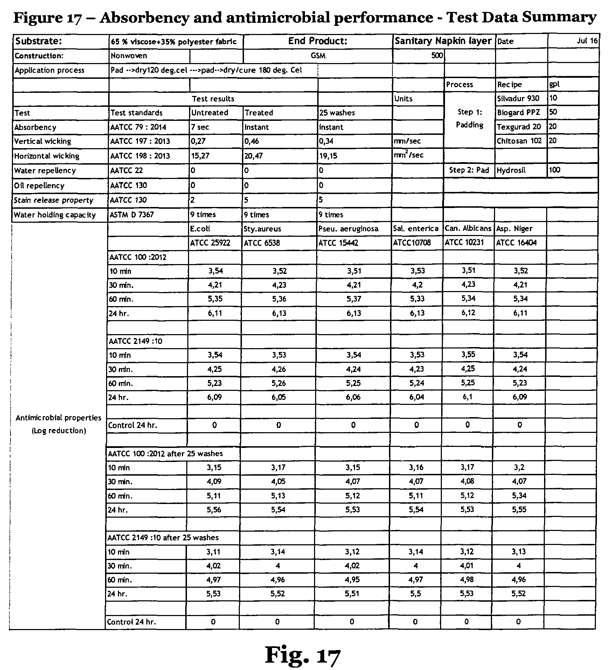

Working Example 2 will now be described with reference to the table in Fig. 17. This example can be used as an absorption layer in a sanitary napkin as will be described below.

The starting textile of the Working Example 2 was a 65% viscose and 35% polyester fabric, non-woven construction, width of 150 cm, fabric weight 500 g/m².

In a first process cycle, the fabric was padded with antimicrobial agents and then dried. In a second process cycle, the fabric was padded with hydrophilic/stain release agents, then dried again and cured.

In the first process cycle, on 400 meters (300 kg) of starting textile material, a padding process was performed: To about 400 liters of water, 4 kg (10 gpl) of Silvadur 930 were added, resulting in 0.0017 gpl of silver cations, 20 kg (50 gpl) of Biogard PPZ, resulting in 12,5 gpl of propiconazole, 8 kg (20 gpl) of Texguard 20, resulting in 4 gpl of PHMB, and 8 kg (20 gpl) of Chitosan 102, resulting in 3 gpl of chitosan. During the padding process, the liquor had a temperature of 30 °C. The padding mangle pressure was 2 bar. The nominal pick-up rate was 100%, and it is estimated that the effective pick-up rate corresponds roughly to the nominal pick-up rate.

The textile was dried by passing it through the stenter, which had 8 chambers and a length of 24 meters, at a speed of 20 centimeters per second. The maximum temperature of 120 °C was applied in all 8 chambers, i.e. during 300 seconds.

Following this first process cycle, a second process cycle with a padding process was performed to apply a hydrophilic/stain release agent: To about 400 liters of water, 20 kg (50 gpl) of Hydrosil were added, resulting in a concentration of 30 gpl organosilane terpolymer in the liquor. During the padding process, the liquor had a temperature of 30 °C. The padding mangle pressure was 2 bar. The nominal pick-up rate was 100%.

Estimating that, after the first process cycle, the effective pick-up rate in a padding process of subsequent process cycles is about 70% of the nominal pick-up rate, the amount of organosilane terpolymer adhered to the textile material was about 2.1% o.w.f.

The textile material was then dried and cured for in total 5 minutes in a single pass through the stenter, at a maximum temperature of 180 °C. The maximum curing temperature was applied for 60 seconds (in 4 of the 8 chambers of the stenter).

The different performance tests of Working Example 2 are summarized in the table of Fig. 12.

With regard to hydrophilicity, the treated fabric exhibits essentially instant water absorbency, while the untreated fabric exhibits a water absorbency time of 7 seconds. The water holding capacity, measured with ASTM test method D 7367 was 9 times the weight of the fabric both before and after treatment, which showed that the hydrophilic agent does not increase the water holding capacity. The vertical wicking rate of the treated textile increased from 0.27 to 0.46 mm/s, and the horizontal wicking rate increased from 15,27 to 20,47 mm²/s. The water repellency rating was 0 both before and after finishing the textile. After 25 laundry washes, the horizontal wicking rate had decreased to 19,15 mm²/s; water absorbency time was still instantaneous.

Both the starting textile material and the finished textile had an oil repellency rating of grade o. The finishing of the textile had the effect that the stain release rating increased from grade 2 to the highest grade (5). After 25 laundry washes, the stain release capability remained at grade 5.

While the starting textile material did not exhibit any measurable antimicrobial properties, the finished textiles were highly antimicrobial, and the imparted antimicrobial properties proved to be highly wash-durable. The unwashed textiles showed that the treated fabric is capable of reducing all tested organisms by more than 99,9% (3 log) within 10 minutes of contact time. With increasing contact time (up to 24 hours), microbial reduction values increased up to more than 99,999% (6 log) for most microbial cultures. After 25 washes, the treated fabric still exhibits microbial reduction values of more than 3 log for all organisms within 10 minutes of contact time, and more than 5 log within 24 hours of contact time.

### Working Example 3 - Water repellent layer for sanitary napkin

Working Example 3 will now be described with reference to the table in Fig. 17.

The starting textile of the Working Example 3 was a 100% polyester fabric, construction 80d x 150d / 144 x 76, width 150 cm, fabric weight 125 g/m². Such a fabric may be used, for instance, for a water repellent layer for a sanitary napkin.

In a first process cycle, the fabric was exhausted with antimicrobial agents and then dried. In a second process cycle, the fabric was coated with synthetic rubbe, then padded with water repellency agents, then dried again and cured.

In the exhaustion process, 1,500 meters (281.25 kg) of starting textile material were loaded on the jigger machine. Into about 600 liters of water, 2,81 kg (1% of the weight of the textile material) of Silvadur 930, resulting in an amount of 0.0017% silver o.w.f., 5,62 kg (2% of the weight of the textile material) of Biogard PPZ, resulting in an amount of 0.5% propiconazole o.w.f., 2,81 kg (1% of the weight of the textile material) of AEM 5772, resulting in an amount of 0.72% quaternary ammonium organosilane o.w.f. The pH of the liquor was again adjusted at pH 5.5. The temperature of the liquor was set to 80 °C. The exhaustion rate was about 98%; the exhaust time was 60 minutes.

The textile was dried by passing it through the stenter, which had 8 chambers and a length of 24 meters, at a speed of 20 centimeters per second. The maximum temperature of 120 °C was applied in all 8 chambers, i.e. during 120 seconds.

Following this first process cycle, a second process cycle with a coating process was performed to apply 50% o.w.f. of synthetic rubber to the textile material, by screen or knife coating, wherein a liquor with 500 gpl of Apcotex CB300 of Apcotex Industries Limited, a chemical which contains 50% of synthetic rubber. Then, a padding process was performed, wherein the liquor contained 100 gpl of Globe of Globechem Imports, India, a chemical which contains 20% of polyurethane (PU).

The textile material was then dried and cured for in total 2 minutes in a single pass through the stenter, at a maximum temperature of 180 °C. The maximum curing temperature was applied for 60 seconds (in 4 of the 8 chambers of the stenter).

As can be seen in Fig. 13, the treated fabric did not absorb water, while the untreated fabric exhibited a water absorbency time of 8 seconds. The water repellency rating measured in accordance with AATCC test method 22-2014 was 0 both before and increased to 100 after finishing the textile. After 25 laundry washes, the absorbency remained at 0, and the water repellency rating dropped slightly to grade 90. The wash-durability of the water repellency imparted by the finishing process is therefore satisfactory.

The starting textile material had an oil repellency rating of grade 0 (i.e., it did not repel oil) while in the finished textile, the oil repellency grade had increased to grad 7 out of 10 when measured in accordance with AATCC test method 130-2013, which means that the treated fabric repels oily stains well. The finishing of the textile had also the effect that the stain release rating increased from the grade 1 to the highest grade (5) as measured in accordance with AATCC test method 130-2010. After 25 laundry washes, the oil repellency decreased slightly to grade 5 and the stain release capability decreased slightly to grade 4. The wash-durability of the improved water repellency properties imparted by the finishing process is therefore satisfactory.

While the starting textile material did not exhibit any measurable antimicrobial properties, the finished textiles were highly antimicrobial, and the imparted antimicrobial properties proved to be wash-durable.

### Example 2: Disinfecting textile for application in diapers for incontinence

First, select a textile material comprising 100% cotton or 100% viscose or a blend of cotton and polyester or a blend of viscose and polyester.

Spray technique is used for application.

Chemicals used: 0.2 g/l silver chloride in aluminosilicate carrier base, 5 g/l polyhexamethylene biguanide, 10 g/l propiconozole, and 0.03% citric acid for adjusting pH-value between 5 and 6.

All the chemicals are dissolved in water and fed into the drum of a spray gun. The textile material is then sprayed at room temperature. Following this, the material is dried with a hot air gun at 180 °C for 2 minutes.

### Working Example 4: Disinfecting textile for use as a hand sanitizer

First, select a textile material comprising of 100% cotton or 100% viscose or a blend of cotton and polyester or a blend of viscose and polyester. Then prepare chemicals in a liquor, and feed into bath of exhaust machine. Fabric is dried at 120 °C using a stenter frame. Subsequent the fabric is put through a padding mangle at room temperature with chemicals and then cured at 180 °C. The fabric is then cut into required pieces and used as a napkin or handkerchief with hand sanitizing properties.

Chemicals used in exhaust: 0.2% owf silver chloride in aluminosilicate carrier base, 1% owf of polyhexamethylene biguanide, 0.8% owf of polyglucosamine and 0.03% citric acid for adjusting pH-value between 5 and 6, along with water (OWF means on weight of fabric). Chemicals used in padding : 50 gpl of organosilane terpolymers, 50 gpl of carbendazime complex and 50 gpl of a cross linker based on acrylates.

### Working Example 5. Disinfecting and stain release bed pads

A bed pad can be produced by proceeding in the same manner as described above in Working Example 2.

### Experimental Examples

The inventors of the present invention performed comprehensive further experiments to determine the effect of different recipes and finishing process parameters. The finishing processes were in general as described above in the context of Working Example 1. Where differences exist, these will be discussed below.

Most Experimental Examples were produced with two different starting textile materials: The first textile was a 100% cotton poplin (or popline), a strong and tightly woven fabric, with count 40s warp and 40s weft, construction 144 x 98, width 150 cm, fabric weight 135 g/m². Such a fabric may be used, for instance, for handkerchiefs. The second textile was a 100% polyester chiffon, a lightweight woven fabric with construction 75D x 60D / 150 x 100, width 150 cm, fabric weight 110 g/m². Like the first textile, such a fabric may be used, for instance, for handkerchiefs.

### Experimental Examples 1: Different combinations of antimicrobial agents

With Experimental Examples 1, the effect of the application of different numbers of agents to the starting textile materials was studied. The agents applied to the cotton material were taken from the group of silver, PHMB, and propiconazole, which are preferred antimicrobial agents for treating cellulosic textile materials. The agents applied to the polyester material were taken from the group of silver, propiconazole, and quaternary ammonium organosilane compounds, which is a group of preferred antimicrobial agents for treating synthetic textile materials.

The textiles were treated with (1) each of the agents from the respective group individually, (2) all three tuple combinations of agents from the respective groups, and (3) all three agents from the respective groups together. The total amount of the chemicals in all experiments was 6% o.w.f. Thus, where one single agent was applied to the textile, 6% o.w.f. of the respective chemical was used, where a combination of two agents was applied, 3% o.w.f. of each of the respective two chemicals was used, and where a combination of three agents was applied, 2% o.w.f. of each of the respective three chemicals was used.

The finishing process was as described above in the context of Working Example 1, but since no hydrophilic/stain release agent was applied, there was only one process cycle. The fabric was exhausted and then dried and cured in one single pass through the stenter, with the process parameters as described above.

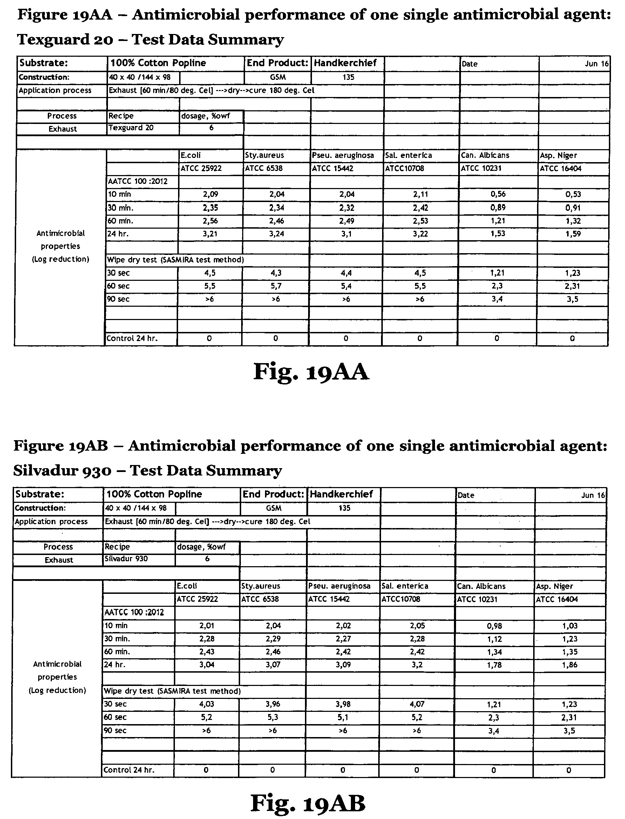

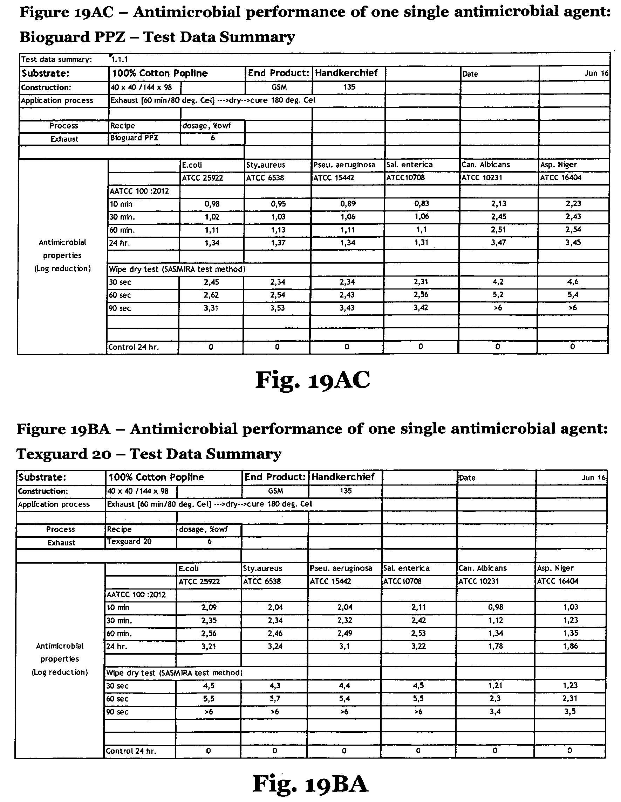

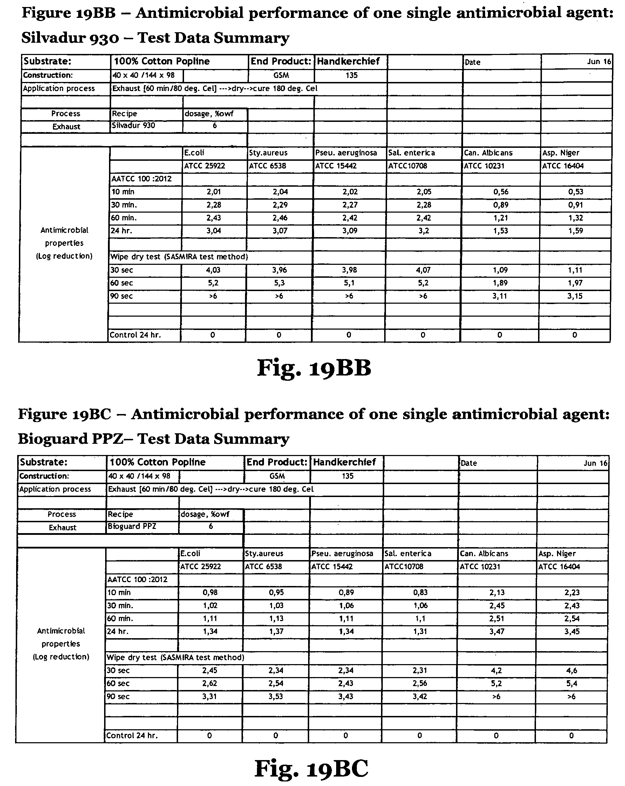

Fig. 19AA to 19AC show the antimicrobial performance of the cotton textiles treated with one single agent. The results indicate that PHMB and silver applied individually are about equally efficient for killing bacteria (*Escherichia coli, Staphylococcus aureus, Pseudomonas aeruginosa,* and *Salmonella enterica).* However, they perform poorly when it comes to killing fungi (*Candida albicans* and *Aspergillus Niger).* On the other hand, propiconazole is efficient for killing fungi, but not efficient for killing bacteria.

Fig. 19BA to 19BC show the antimicrobial performance of the polyester textiles treated with one single agent. Similar to the results for silver and PHMB on cotton, the results for polyester indicate that silver and quaternary ammonium organosilane compounds applied individually are about equally efficient in killing bacteria, but not efficient for killing fungi. However, unlike propiconazole adhered to cotton, propiconazole adhered to polyester is about as efficient against bacteria as silver and quaternary ammonium organosilane adhered to polyester. Furthermore, like propiconazole adhered to cotton, propiconazole adhered to polyester works well against fungi. The overall killing rates of the treated cotton materials were about the same as the killing rates of the treated polyester materials.

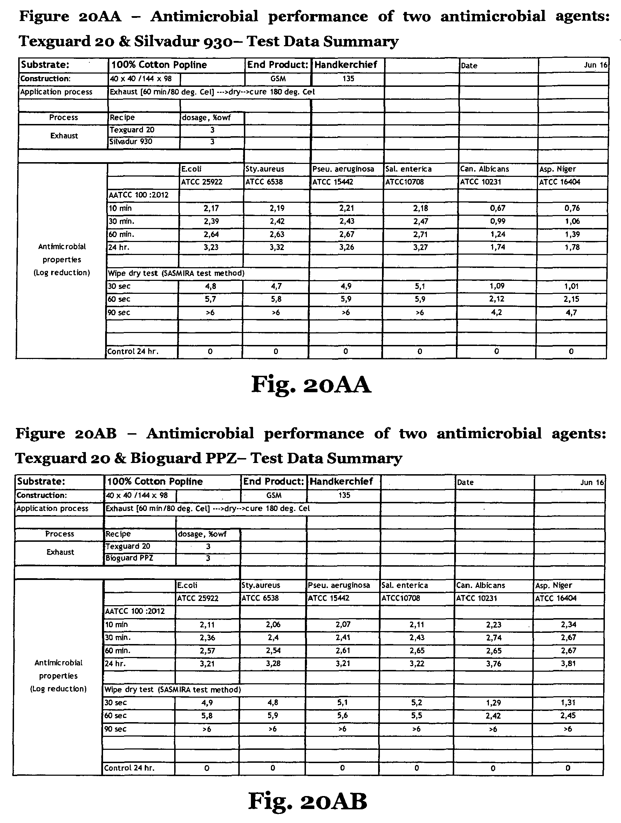

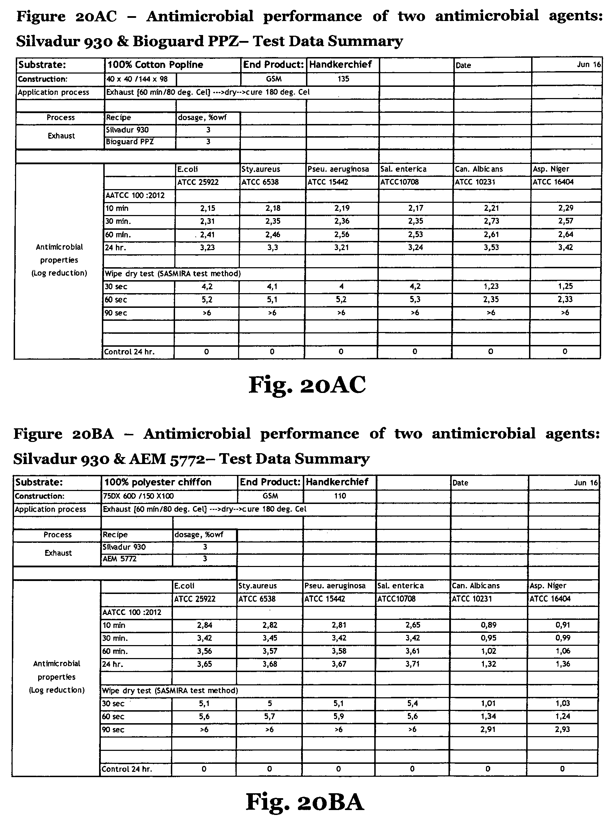

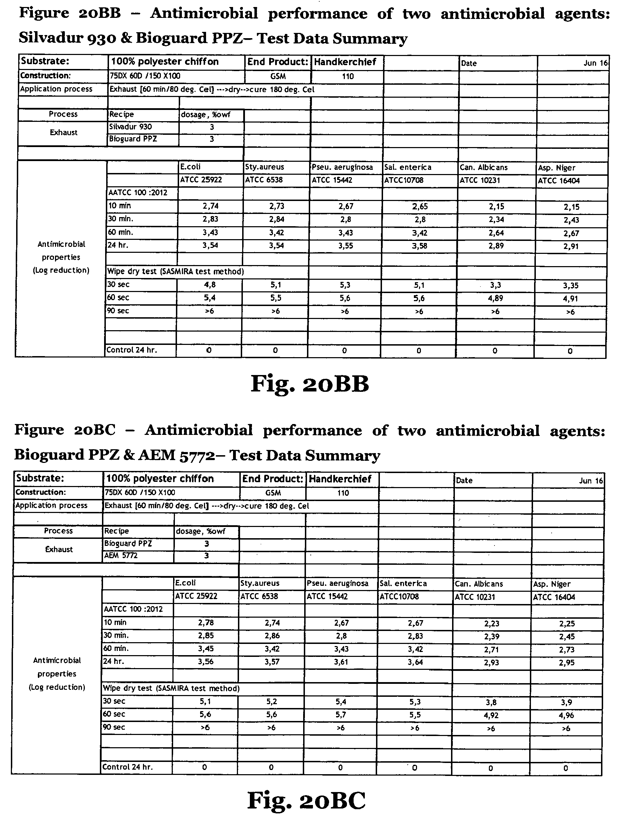

Fig. 20AA to 20AC show the antimicrobial performance of the cotton textiles treated with combinations of two agents. The textile which was not treated with propiconazole (i.e. the textile treated with silver and PHMB) performed poorly against fungi. On the other hand, the textiles treated with propiconazole and either silver or PHMB performed well against both bacteria and fungi. The overall performance of the textiles treated with combinations of two agents was slightly higher than the performance of the textiles treated with one single agent.

Fig. 20BA to 20BC show the antimicrobial performance of the polyester textiles treated with combinations of two agents. Again, the textile which was not treated with propiconazole (i.e. the textile treated with silver and quaternary ammonium organosilane compounds) performed poorly against fungi. On the other hand, the textiles treated with propiconazole and either silver or quaternary ammonium organosilane compounds performed well against both bacteria and fungi. The overall performance of the textiles treated with combinations of two agents also here was slightly higher than the performance of the textiles treated with one single agent.

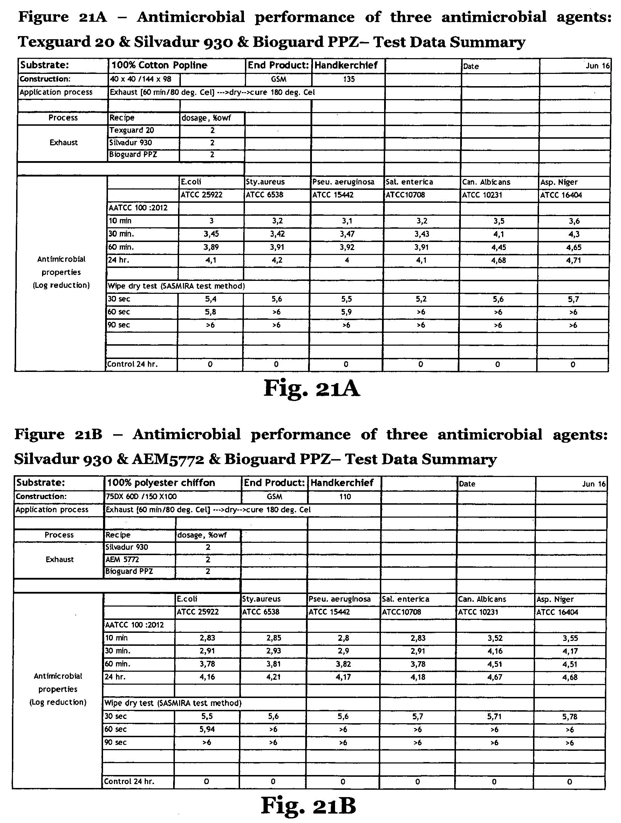

Fig. 21A and 21B show the antimicrobial performance of the cotton and polyester textiles, respectively, treated with combinations of all three agents from the above-specified groups. Overall performance is again higher than the performance of the textiles treated only with two agents.

From Experimental Examples 1, it can be concluded that all antimicrobial agents identified as preferred by the inventors are about equally powerful against bacteria. This was also found in earlier experiments conducted by the inventors and discussed in International patent application PCT/EP2016/054245 by the same applicant. An exception to this rule is propiconazole (or other azole-based agents), which performs poorly against bacteria when adhered to cotton (or other cellulosic textile materials). The inventors believe that propiconazole gets more efficiently attached to synthetic textiles like polyester than to cellulosic textiles like cotton because polyester is thermoplastic polymer. Furthermore, only propiconazole (or other azole-based agents) or combinations comprising propiconazole show a good performance against fungi. Cotton (or other cellulosic textile materials) and polyester (or other synthetic textile materials) are generally equally suitable unless only propiconazole (or another azole-based agent) is applied, in which case polyester performs better. The experiments show a synergistic killing effect for the same organism with a growing number of agents. Although the effect is not dramatic, it is well visible.

### Experimental Examples 2: Effect of hydrophilic/stain release agents

The purpose of Experimental Examples 2 was to study the effect of the hydrophilic/stain release agents, in particular in combination with antimicrobial agents.

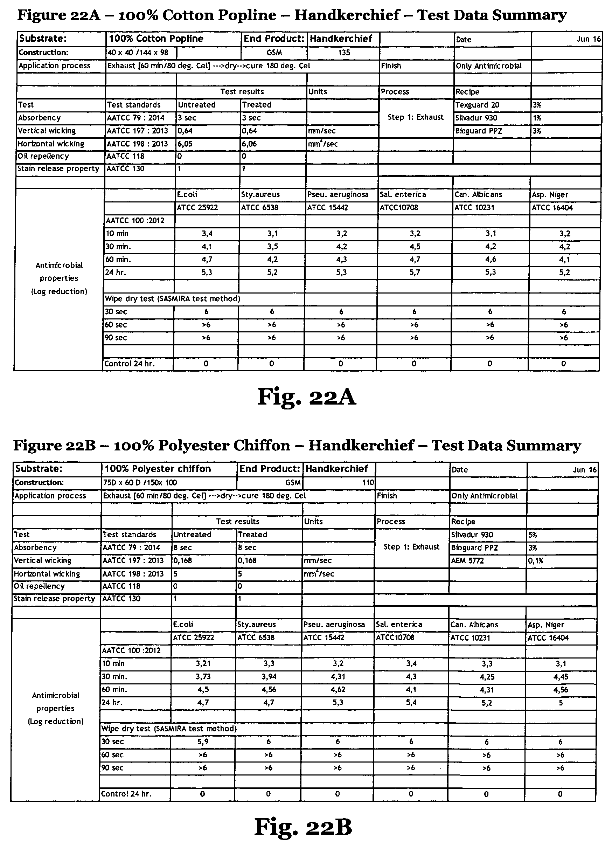

Fig. 22A and 22B show the data of Experimental Examples 2a, where only antimicrobial agents were applied to the textile materials. The finishing process was the same as for Experimental Examples 1, but the antimicrobial recipes were more preferred recipes for cotton and polyester, respectively.

As can be seen in Fig. 22A and 22B, the hydrophilic/hydrophobic properties of the starting textile materials were not affected by the treatment with antimicrobial agents. The same is true for the stain release rating. It can therefore be concluded that any change in hydrophilicity and stain release rating observed above for the Working Examples can be attributed to the treatment with Hydrosil in the second process cycle, which contains the hydrophilic/stain release agent organosilane terpolymer.

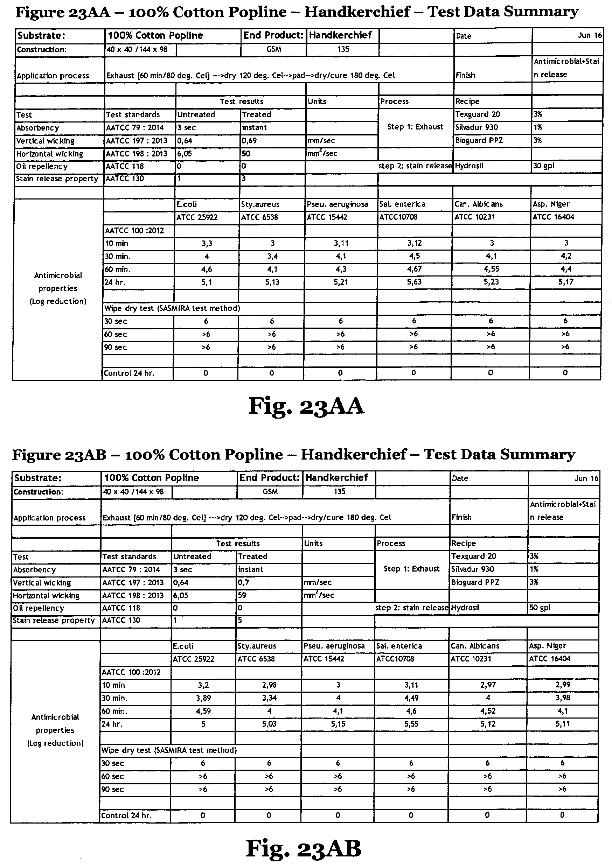

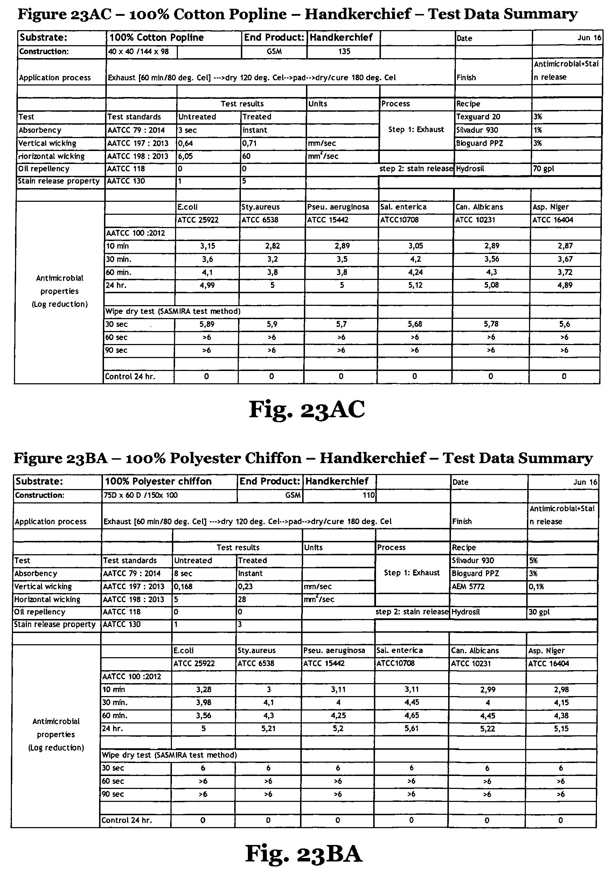

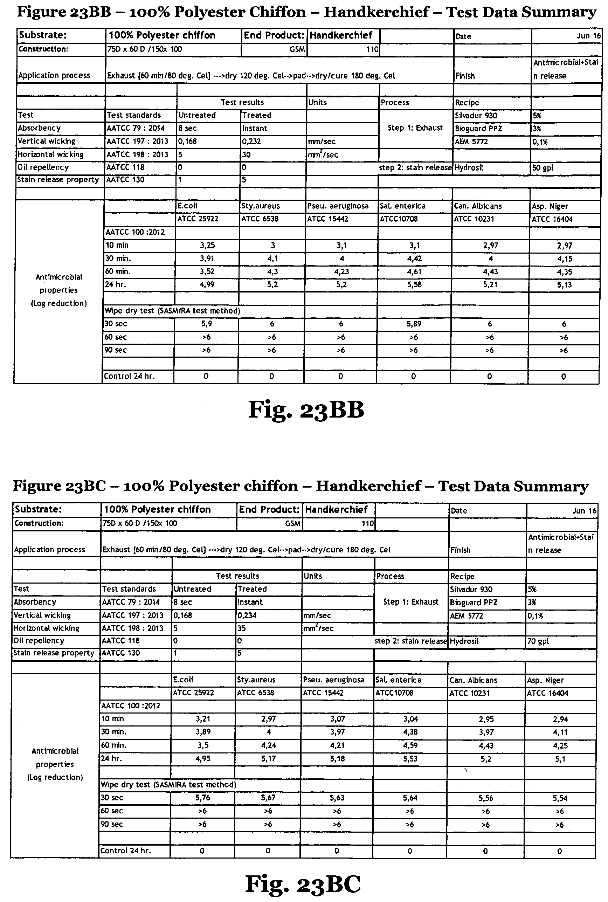

Fig. 23AA to 23AC (cotton) and Fig. 23BA to 23BC (polyester) show the data of the textile materials of Experimental Examples 2b, which were treated with the same antimicrobial process as the Experimental Examples 2a of Fig. 22A and 22B. However, these textiles were treated with Hydrosil in a second process cycle as described in the context of Working Examples 1 and 2. Varying amounts of Hydrosil were added to the liquor of the padding process: 30 gpl (Fig. 23AA and 23BA), 50 gpl (like in Working Example 1, Fig. 23AB and 23BB), and 70 gpl (Fig. 23AC and 23BC).

The test results show that Hydrosil improves absorbency time and horizontal wicking significantly, as has already been observed in the context of the Working Examples. Vertical wicking is only marginally (cotton) or slightly (polyester) improved. Between an application of 30 gpl of Hydrosil in the padding liquor and 50 gpl, the stain release rating improves from grade 3 to grade 5. No significant changes can be observed in hydrophilicity and stain release capability when increasing the Hydrosil concentration in the liquor from 50 gpl to 70 gpl. The antimicrobial efficiency decreases slightly with increasing amounts of Hydrosil.

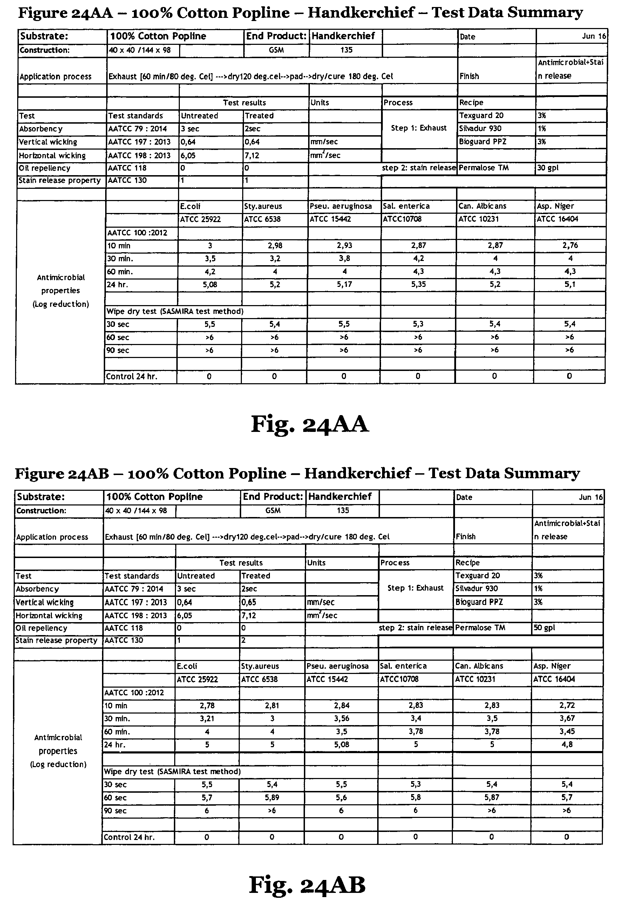

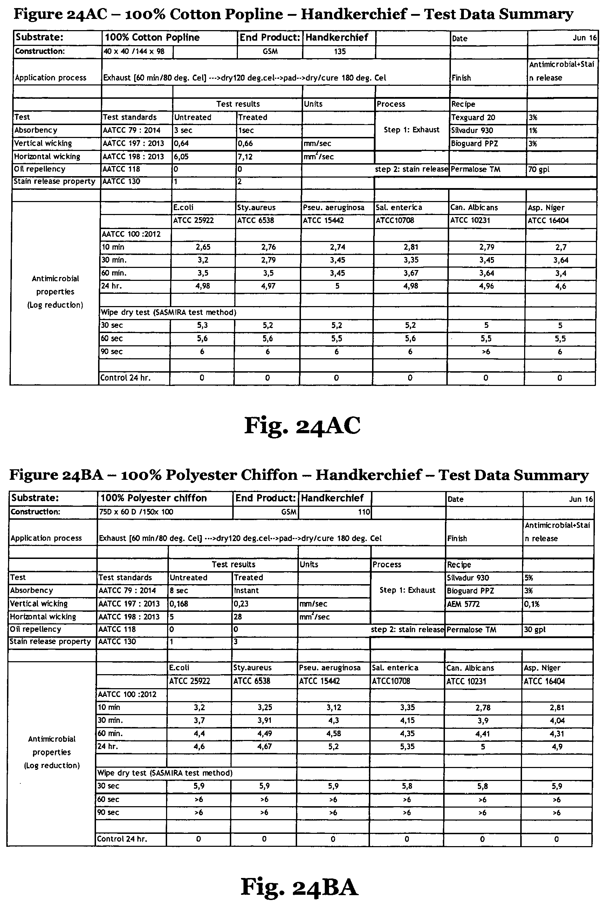

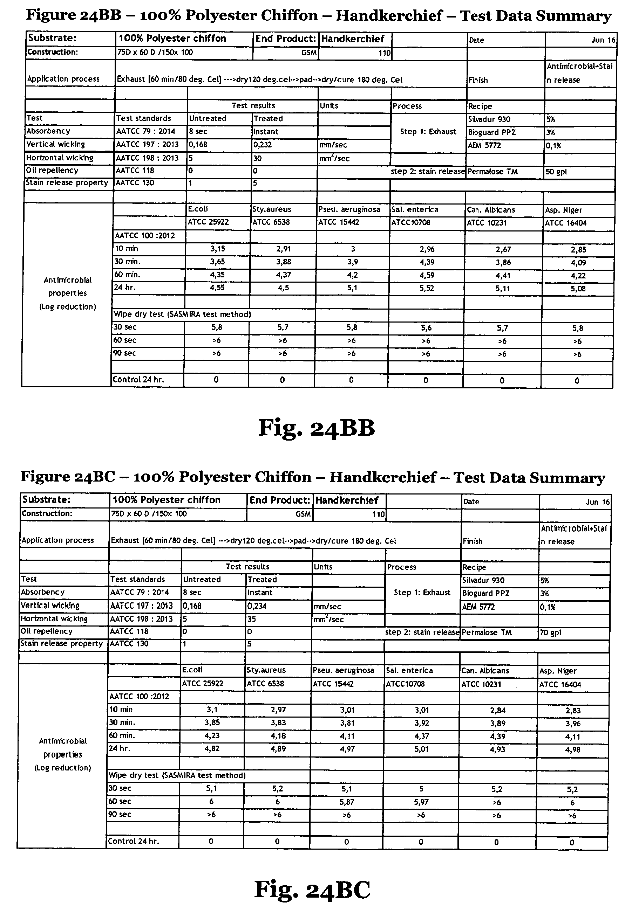

Fig. 24AA to 24AC (cotton) and Fig. 24BA to 24BC (polyester) show the data of the textile materials of Experimental Examples 2c, which were treated with the same antimicrobial process as the Experimental Examples 2a and 2b. However, in the second process cycle, Permalose was applied in the padding process of the second process cycle instead of Hydrosil.

The treatment with Permalose increases the hydrophilic properties of cotton only slightly, which is not unexpected given that it is optimized for synthetic materials. Also the stain release rating of cotton is only improved to grade 2 (for 50 and 70 gpl). The antimicrobial efficiency decreases a little faster with increasing amounts of Permalose than with increasing amounts of Hydrosil. On the other hand, on polyester (Fig. 24BA to 24BC), Permalose improves hydrophilicity and stain release capability equally well as Hydrosil, but again the antimicrobial efficiency decreases a little faster with increasing amounts of Permalose than with increasing amounts of Hydrosil.

From Experimental Examples 2, it can be concluded that it is not the antimicrobial agents used herein but only the hydrophilic/stain release agents which affect hydrophilicity and stain release capability. Furthermore, Permalose is preferred only for polyester or other synthetic textile materials. An optimum concentration of both Hydrosil and Permalose in a padding process with a pick-up rate of 100% seems to be 50 gpl for many applications, which results in actives adhered to the textile material in an amount of about 1% o.w.f. (organosilane terpolymer/Hydrosil) and about 0.4% o.w.f. (fatty alcohol ethoxylates/Permalose).

As mentioned above, a chemical which is equivalent to Permalose but optimized for cellulosic textiles is Hydroperm of Archroma. It comprises 20% of fatty alcohol ethoxylates. The inventors found in experiments, whose results are not reproduced herein, that also for Hydroperm, a concentration of 50 gpl in the padding liquor brings about the best results at a pick-up rate of 100%. With an estimated effective pick-up rate of 70%, the amount of fatty alcohol ethoxylates adhered to the textile is then about 0.7% o.w.f

### Experimental Examples 3: Variations in the finishing process

In previous research efforts related to processes of conveying antimicrobial properties to textiles, the inventors had already found out that for the exhaustion process, a temperature of the exhaust liquor of 80 °C and an exhaust time of 60 min is ideal in terms of antimicrobial efficiency and wash-durability thereof, see International patent application PCT/EP2016/054245 by the same applicant. Furthermore, application of an exhaustion process with these parameters leads to higher antimicrobial efficiency and wash-durability than application of a padding process. Finally, the ideal curing temperature had been determined to be 180 °C. Experimental Examples 3 were manufactured to confirm that these earlier findings hold true also when a hydrophilic/stain release agent is applied to the textile, subsequent to the application of the antimicrobial agents.

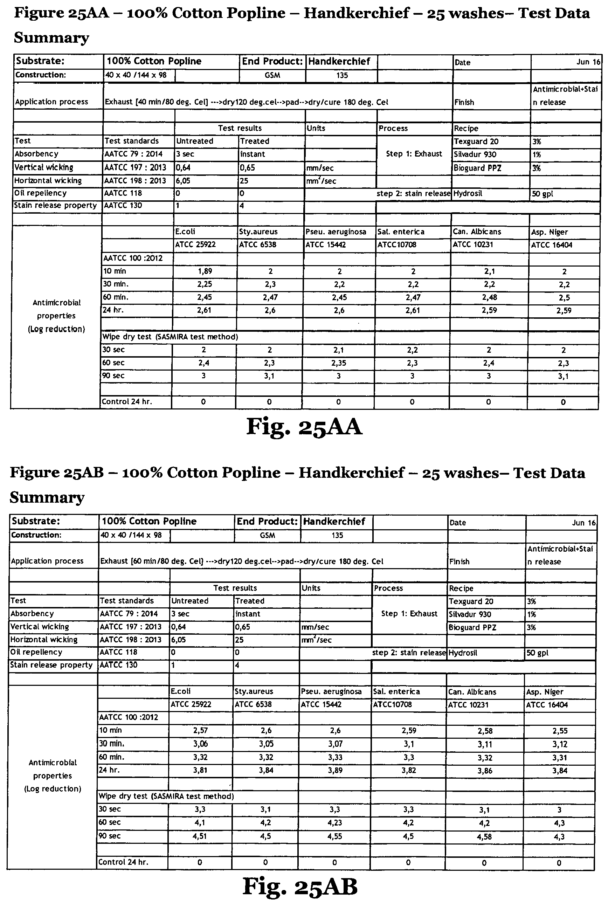

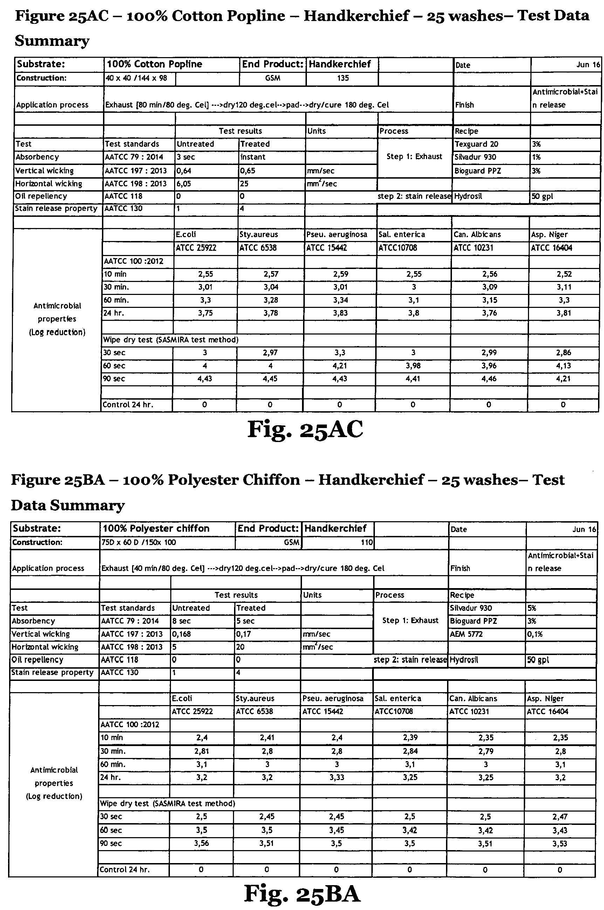

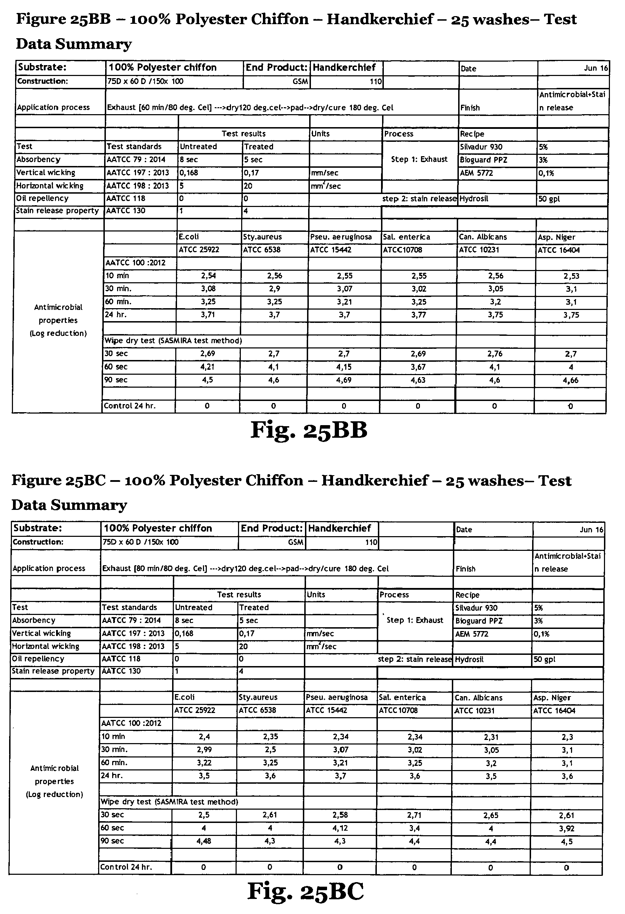

Fig. 25AA to 25AC (cotton) and Fig. 25BA to 25BC (polyester) show the data of the textile materials of Experimental Examples 3a. They were manufactured by finishing the starting textile material with the same antimicrobial exhaustion process cycle followed by a hydrophilic/stain release padding process cycle, and with the same recipes, as described above for the Working Examples. However, the exhaust time was varied between 40/60/80 minutes.

The performance tests were made on the finished textile after 25 laundry washes. As could be expected, the variation in exhaust time in the antimicrobial process cycle had no impact on the hydrophilic and stain release properties. However, the tests confirmed that the antimicrobial performance after 25 washes for the samples treated with an exhaust time of 60 minutes is considerably higher (about 1 log) than that of the samples treated for 40 minutes, and slightly higher than that of the samples treated for 80 minutes.

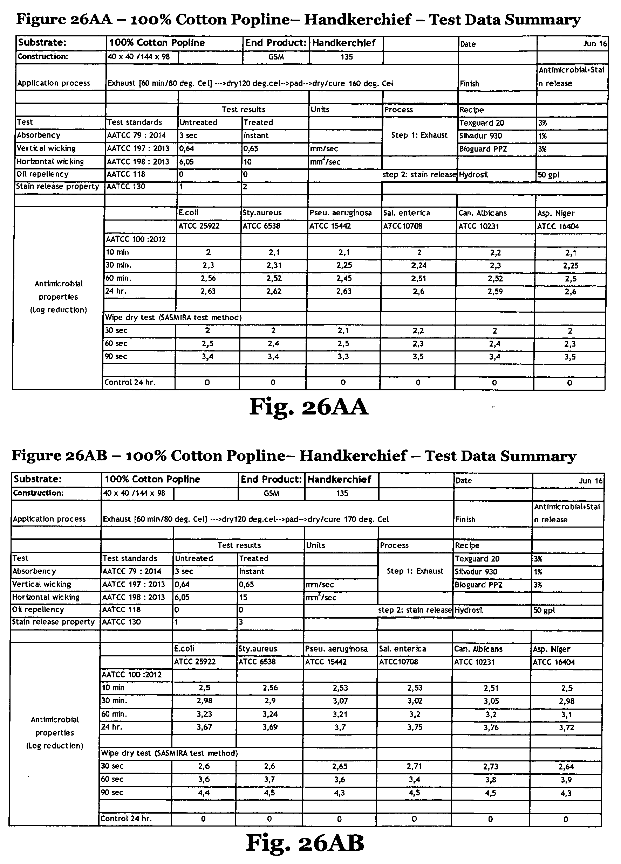

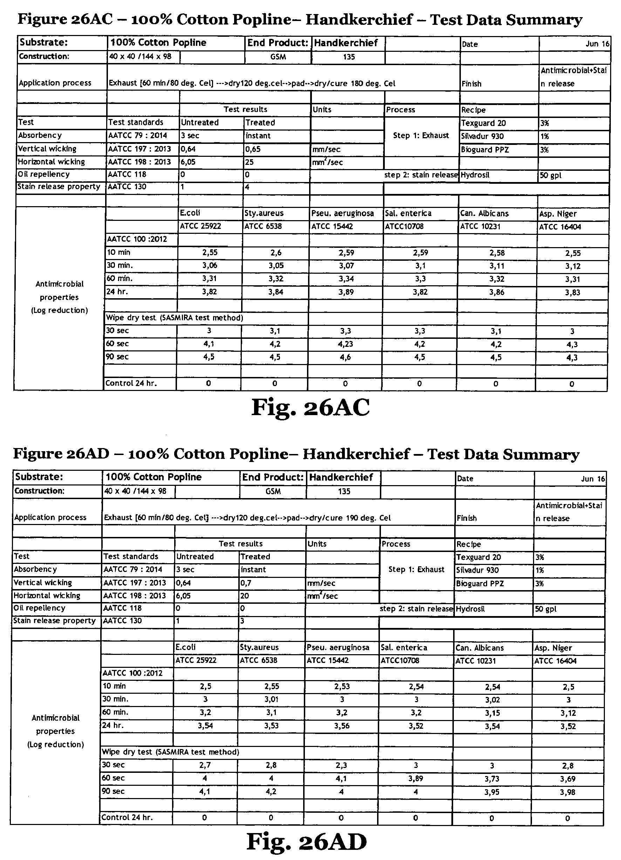

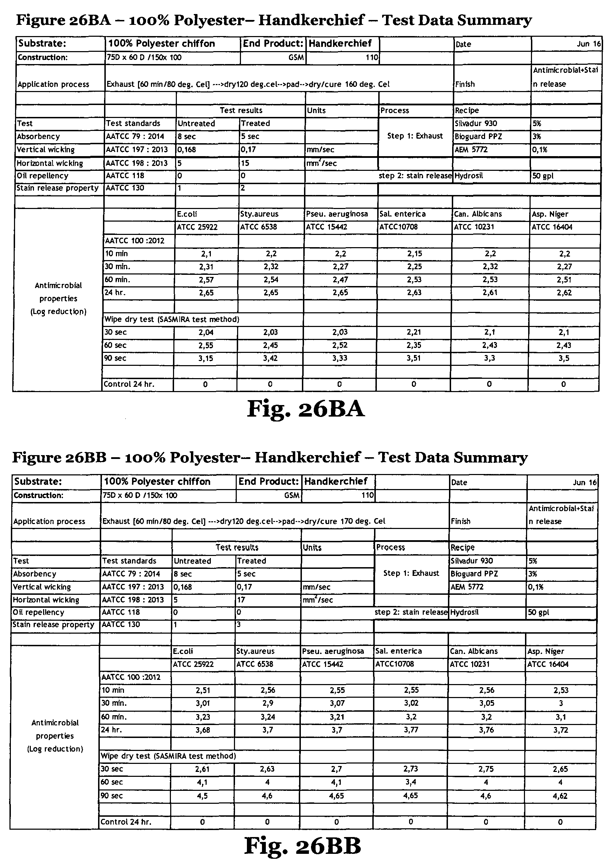

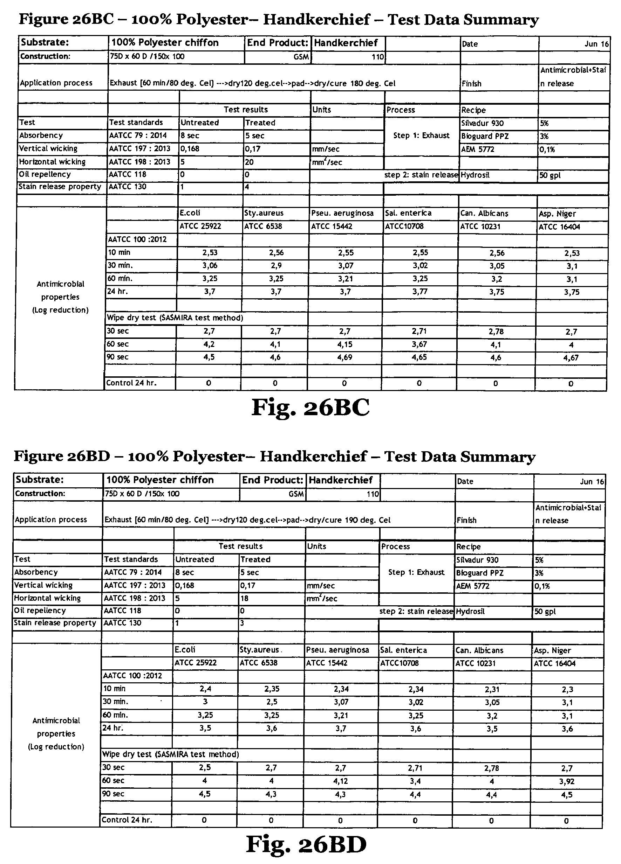

Fig. 26AA to 26AD (cotton) and Fig. 26BA to 26BD (polyester) show the data of the textile materials of Experimental Examples 3b. They were finished with the same process as described above for Working Example 1, but the curing time (at the end of the second process cycle) was varied between 160/170/180/190 minutes.

The performance tests were again made on the finished textile after 25 laundry washes. The hydrophilic properties peaked for a curing temperature of 180 °C. This could be observed in the horizontal wicking rate, which increased for the cotton textile material from 10 mm²/sec (curing temperature of 160 °C) to 25 mm²/sec (curing temperature of 180 °C), and then declined again to 20 mm²/sec (curing temperature of 190 °C). Similar results could be observed for the polyester textile material. However, the absorption time was the same for all curing temperatures, namely instantaneous for cotton and 5 seconds for polyester.

A similar observation could be made for the stain release capability, which for both cotton and polyester peaked at grade 4 for the curing temperature of 180 °C. For the lower curing temperatures of 160 °C and 170 °C, the stain release ratings were grade 2 and grade 3, respectively. For the curing temperatures of 190 °C, the stain release rating started to decline again, to grade 3.

Finally, also the antimicrobial performance peaked for both cotton and polyester at the curing temperature of 180 °C. A considerable performance increase could be observed when the curing temperatures went from 160 to 170 °C, and a slight increase when the curing temperature went from 170 to 180 °C. When the curing temperature was further increased to 190 °C, the performance started to decline again.

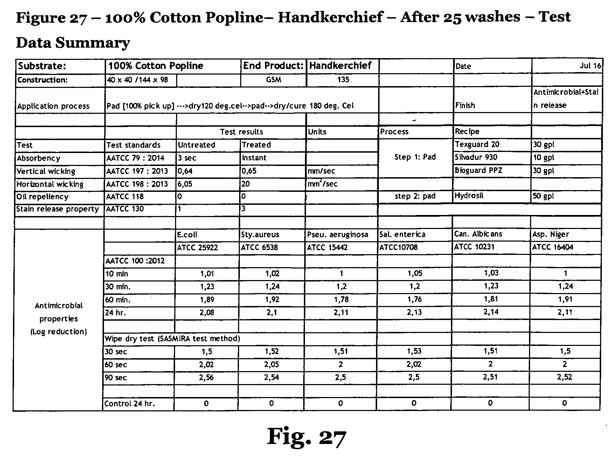

Fig. 27 shows the data of the textile material of Experimental Example 3c, which was finished using a padding process in the first (the antimicrobial) cycle, instead of an exhaust process. The concentrations of the antimicrobial chemicals in the padding liquor were chosen such that the amount of antimicrobial agents adhered to the finished textile material were the same as for the examples finished with an exhaust process. This experiment was only made with cotton, not with polyester.

The performance tests were again made on the finished textile after 25 laundry washes. Interestingly, hydrophilicity and the stain release capability were somewhat lower than in the examples finished with an exhaust process in the antimicrobial cycle. For example, the vertical wicking rate was only 20 mm²/sec instead of 25, and the stain release rating was only grade 3 instead of grade 4. It is believed that if the antimicrobial agents are padded in the first cycle, they occupy more space on the fiber surface than when they are exhausted, leaving less space for the hydrophilic/stain release agents to be adhered in the padding process of the subsequent cycle.

More importantly, the antimicrobial performance of Experimental Example 3c was much lower (by about 2 log) than that of the examples where an exhaustion process was used for applying the antimicrobial agents.

From Experimental Example 3c, it can be concluded that applying the antimicrobials with an exhaustion process leads to much better antimicrobial performance and/or wash-durability of the antimicrobial properties than the application of a padding process. It even increases the hydrophilic and stain release properties conveyed in a subsequent padding process, and/or the wash-durability thereof. Experimental Example 3a confirmed that even when hydrophilic/stain release agents are applied to a textile which had been treated with antimicrobials in an exhaust process, an exhaust time of 60 minutes is ideal for antimicrobial performance and/or wash-durability thereof. Finally, Experimental Example 3b showed that a curing time of 180 °C is ideal not only for antimicrobial performance but also for hydrophilic and stain release capability, and/or wash-durability thereof.

### EXAMPLES FOR THE USE OF THE FINISHED TEXTILE MATERIAL

### Hygiene and other products

Embodiments of the textile materials according to the invention can be used, e.g., as a hygiene product. The hygiene product comprises at least one layer, preferably at least two layers, more preferably three layers or at least three layers. The one or more layers can comprise, consist, or be formed by a textile material. The textile material can convey stain release capability, antimicrobial efficiency, water holding capacity, water absorption time, stain/oil repellency, or water repellency, in particular stain release, absorbing, and/or antimicrobial properties. In exemplary embodiments, the hygiene product comprises one layer, preferably formed by a textile material, and at least a second layer formed by a textile material conveying stain release, absorbing and/or antimicrobial properties. In further embodiments, the hygiene product comprises a further layer of a textile material. The layer, the second layer and/or the further layer of the textile material may comprise or consist of a synthetic fabric or a natural fabric or a blend of both, with or without the addition of a stretch yarn, in particular lycra or spandex. In exemplary embodiments, the textile material comprises polyester, preferably consists of polyester. In other exemplary embodiments, the textile material comprises a blend of polyester and cotton, preferably consists of a blend of polyester and cotton, e.g. about 65% polyester and about 35% cotton.

In exemplary embodiments of the hygiene product, at least one of the surfaces of the further layer is finished with a water-and-oil repellant, optionally including polyurethane (PU). In other exemplary embodiments, the further layer is formed by a textile material which is a blend of polyester and cotton, e.g. about 65% polyester and about 35% cotton.

In further exemplary embodiments, the hygiene product comprises three layers, wherein two layers are formed by a textile material conveying stain release, absorbing and/or antimicrobial properties, and the third layer is impermeable to liquids, e.g. the third layer is formed by a textile finished with a water-and-oil repellant. The layers of a hygiene product may be connected by ultra-sound welding, stitching, gluing or by gluing using fusible interlinings.

The hygiene product can be a sanitary napkin. Further exemplary hygiene products are panty or underwear liners, tampons, nappies, diapers, diaper liners, adult diapers, panties or underwear, bras, or nursing pads. Other exemplary embodiments are a towel, kitchen towel, napkin, handkerchief, face mask, floor cleaning mop, wipe, or textile used in wound care. As an exemplary embodiment, the hygiene product may be used as a hand sanitizer.

In a preferred embodiment, a hygiene product comprises a textile material as described above. The textile material can form a layer. The hygiene product can comprise at least a second layer formed by a textile material as described above. The hygiene product can comprise a further layer of a textile material. The further layer can comprise or consists of a synthetic fabric or a natural fabric or a blend of both, with or without the addition of a stretch yarn, in particular lycra or spandex. At least one of the surfaces of the further layer may be finished with a water- and oil-repellant, optionally including polyurethane (PU), preferably enhanced with perfluoroalkylethylacrylate copolymer with C6 or and C8 carbon chain. The hygiene product may be one selected from the group consisting of sanitary napkins, sanitary lungots or lungots, panty or underwear liners, tampons, nappies, diapers, diaper liners, adult diapers, panties or underwear, bras, bed pads, or nursing pads. Further, the hygiene product may be a towel, napkin, handkerchief, face mask, floor cleaning mop, wipe, or textile used in wound care, comprising or consisting of a textile material as described above.

The hygiene product of the invention provides the advantage that due to its stain release properties it can be washed easily at mild conditions, e.g. with water, optionally using some mild detergent, by hand or in a washing machine at low temperatures, e.g. 30°C or 40°C.

The invention will be further described by the following example, which illustrates preferred embodiments without limiting the scope of the invention.

### Sanitary napkin

A preferred embodiment of the invention is a sanitary napkin.

### Layers of the sanitary napkin

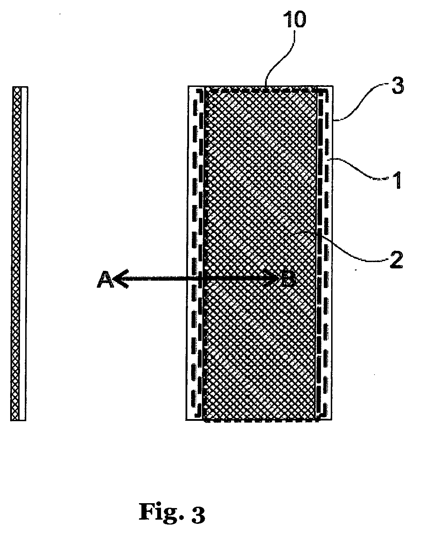

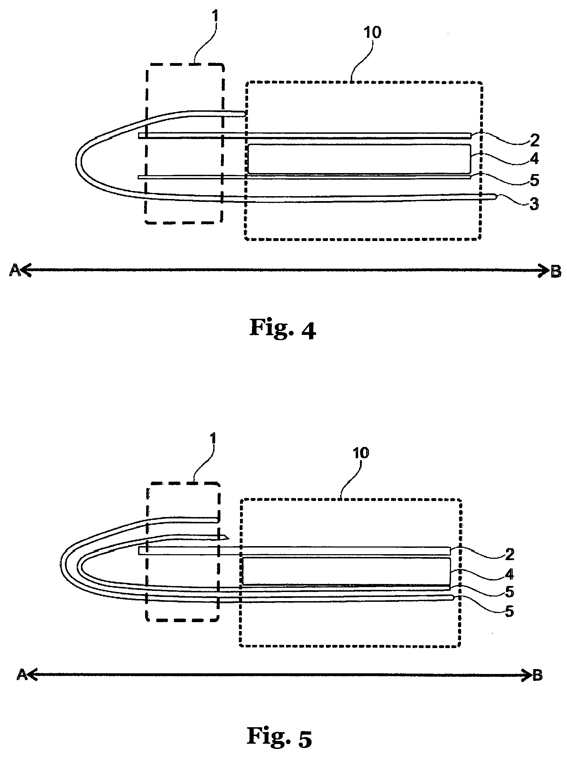

Referring to Fig. 3 and 4, the sanitary napkin comprises one or more dispersion layers (2, 4) comprising or consisting of textile material. In a preferred embodiment of the sanitary napkin, the one or more dispersion layers in conjunction have fluid dispersion properties such that if 1 ml of water is dropped onto the dispersion layers, the water is dispersed within 1 sec in a dispersion area having an average diameter of at least 3 cm, preferably at least 4 cm, more preferably at least 4.5 cm, and most preferably at least 5 cm.

The sanitary napkin also comprises one or more water repellent layers 3, 5, preferably having a water repellency rating of at least 70, preferably at least 80, more preferably at least 90, measured in accordance with AATCC test method 22-2014.

### Transportation layer (first layer)

The one or more dispersion layers can comprise a transportation layer 2, a preferred embodiment of which has already been described above as Working Example 1. This transportation layer 2 can be the layer which lies against the skin of the user when the sanitary napkin is worn by the user. In this case, the transportation layer 2 forms the inner (upper) layer of the sanitary napkin.

As its name implies, the main purpose of the transportation layer is to allow for quick transportation of fluids to the layer(s) below the transportation layer, and to provide a layer which is kept relatively dry. This can be achieved with a textile material with at least 10, preferably at least 15, more preferably at least 25, particularly at least 30, and most preferably at least about 40 holes, per cm². The textile material can have at most 625, preferably at most 300, more preferably at most 100, and most preferably at most about 40 holes, per cm². The size of the holes may be at least 0.02 mm, preferably at least 0.04 mm, more preferably at least 0.06 mm, in average diameter. The size of the holes can be at most 2.0 mm, preferably at most 1.5 mm, more preferably at most 1.0 mm, and most preferably at most 0.08 mm, in average diameter. Such a textile material may be an impression fabric, a knitted, warp knitted, and/or a multifilament fabric. Preferably, the knitted or warp knitted fabric is a knit pique fabric.

The starting textile material of the transportation layer 2 may comprise at least 95%, preferably at least 97%, more preferably at least 99% of a synthetic fiber, such as polyamide (nylon), or preferably polyester. Synthetic textile materials are preferred, *inter alia,* because they can be welded with ultrasound.

In exemplary embodiments, the textile material of the transportation layer 2 has a weight of at least 50 GSM (grams per square meter), preferably at least 80 GSM, more preferably at least 100 GSM, particularly at least 120 GSM, and most preferably at least about 125 GSM. The textile material can have a weight of at most 250 GSM, preferably at most 200 GSM, more preferably at least 170 GSM, particularly at least 150 and most preferably at most about 125 GSM.

Advantageously, the textile material is equipped with antimicrobial agents. For example, an azole-based compound is adhered to the textile material in an amount of at least 0.05%, preferably at least 0.10%, more preferably at least 0.15%, particularly at least 0.20%, and most preferably at least about 0.25%, and/or in an amount of at most 2.0%, preferably at most 1.5%, more preferably at most 1.0%, particularly at most 0.5%, most preferably at most about 0.25% on weight fabric of the textile material; and/or metal is adhered to the textile material in an amount of at least 0.0005%, preferably at least 0.001%, more preferably at least 0.002%, particularly at least 0.005%, most preferably at least about 0.0085%, and/or in an amount of at most 0.1%, preferably at most 0.05%, more preferably at most 0.03%, particularly at most 0.02%, most preferably at most about 0.085% on weight fabric of the textile material; and/or a quaternary ammonium organosilane compound is adhered to the textile material in an amount of at least 0.01%, preferably at least 0.02%, more preferably at least 0.05%, particularly at least 0.10%, and most preferably at least about 0.14%, and/or in an amount of at most 1.0%, preferably at most 0.75%, more preferably at most 0.5%, particularly at most 0.25%, most preferably at most about 0.14% on weight fabric of the textile material.

Advantageously, hydrophilic and/or stain release agents can be adhered to the textile material, with the types and amounts of agents selected as described in the sections above, to accelerate the distribution of fluid in the layer, and to increase its washability. The antimicrobial agents and/or the hydrophilic agents can be applied to the textile material by the process as described above.

### Main Absorption layer(s) (second layer(s))

The one or more dispersion layers can also comprise one or more main absorption layers 4, a preferred embodiment of which has already been described above as Working Example 2. The one or more main absorption layers 4 can be located between the transportation layer 2 and the one or more water repellent layers 3, 5.

As its name implies, the main purpose of the absorption layer(s) is to store as much fluid as possible. The inventors found that it is hardly possibly to increase the water holding capacity of a textile with a re-usable hydrophilic agent by a significant amount. Already the starting textile material of the absorption layer(s) should therefore exhibit a fluid holding capacity of preferably at least 5 times its weight, more preferably at least 6 times its weight, even more preferably at least 7 times its weight, and most preferably at least 9 times its weight, when tested according to ASTM D7367-14.

The inventors found that such a fluid holding capacity can be achieved, e.g., by a polar or preferably terry fleece fabric, or a heat set or heat bonded, spun bonded, melt blown, or preferably needle punch non-woven fabric. The non-woven textile material has preferably fibers with an average diameter of at most 10 microns, preferably at most 6 microns, more preferably at most 3 microns, even more preferably at most 2 microns, and most preferably at most 1.5 microns. The inventors found that the smaller the diameter of the fibers, the larger is the water holding capacity of the textile material.

The textile material may comprise at least 30%, preferably at least 45%, more preferably at least 60%, most preferably at least about 65% viscose. The textile material may comprise at most 100%, preferably at most 75%, more preferably at most 70%, most preferably at most about 65% viscose. The textile material may comprise at least 15%, preferably at least 25%, more preferably at least 30%, most preferably at least about 35% of a synthetic fiber, such as polyamide (nylon) or preferably polyester. The textile material may comprise at most 100%, preferably at most 65%, more preferably at most 40%, most preferably at most about 35% of a synthetic fiber, such as polyamide (nylon) or preferably, polyester.

During a menstrual cycle, the average quantity of total flow is of approximately 120 ml. On the heaviest days, the quantity of the flow can be up to 45 ml, to which a margin has to be added for other possible fluids such as urine. In a preferred embodiment, the sanitary napkin has then an absorption capacity in the range of 70-80 ml. Based on a fluid holding capacity of 5 to 9 times the weight of the textile, and on the assumption that the transportation layer 2 will also absorb parts of the fluid, the total weight of the one or more main absorption layers 4 is at least 1 gram, preferably at least 2 grams, more preferably at least 2.5 grams, most preferably at least 3 grams, and/or at most 20 grams, preferably at most 15 grams, more preferably at most 10 grams, even more preferably at most 8 grams, and most preferably at most 6 grams.

Given the dimensions of the sanitary napkin as outlined below, in an exemplary embodiment, one or more of the absorption layers 4 is advantageously made of a fabric of at least 50 GSM, preferably at least 100 GSM, more preferably at least 200, and most preferably at least 300 GSM, and/or of at most 700 GSM, preferably at most 500 GSM, more preferably at most 400, and most preferably at most 300 GSM.

In an exemplary embodiment where the sanitary *napkin* comprises two main absorption layers 4, one absorption layer is a fabric of at least 50 GSM, preferably at least 100 GSM, more preferably at least 200, and most preferably at least 300 GSM, and/or of at most 500 GSM, preferably at most 400 GSM, more preferably at most 350, and most preferably at most 300 GSM. The second absorption layer is a fabric of at least 50 GSM, preferably at least 100 GSM, more preferably at least 150, and most preferably at least 200 GSM, and/or of at most 500 GSM, preferably at most 400 GSM, more preferably at most 300 GSM, most preferably at most 200 GSM.

Advantageously, the textile material is equipped with antimicrobial agents. For example, an azole-based compound is adhered to the textile material in an amount of at least 0.10%, preferably at least 0.20%, more preferably at least 0.50%, particularly at least 1.0%, and most preferably at least about 1.25%, and/or in an amount of at most 3.0%, preferably at most 2.5%, more preferably at most 2.0%, particularly at most 1.75%, most preferably at most about 1.25% on weight fabric of the textile material; and/or a metal is adhered to the textile material in an amount of at least 0.0001%, preferably at least 0.0002%, more preferably at least 0.0005%, particularly at least 0.0010%, most preferably at least about 0.0017%, and/or in an amount of at most 0.05%, preferably at most 0.02%, more preferably at most 0.01%, particularly at most 0.005%, most preferably at most about 0.0017% on weight fabric of the textile material; and/or polyhexamethylene biguanide is adhered to the textile material in an amount of at least 0.05%, preferably at least 0.1%, more preferably at least 0.2%, particularly at least 0.3%, and most preferably at least about 0.4%, and/or in an amount of at most 2.0%, preferably at most 1.5%, more preferably at most 1.0%, particularly at most 0.8%, most preferably at most about 0.4% on weight fabric of the textile material; and/or polyglucosamine is adhered to the textile material in an amount of the at least 0.05%, preferably at least 0.10%, more preferably at least 0.15%, particularly at least 0.20%, and most preferably at least about 0.3%, and/or in an amount of at most 2.0%, preferably at most 1.5%, more preferably at most 1.0%, particularly at most 0.6%, most preferably at most about 0.3% on weight fabric of the textile material. Quaternary ammonium organosilane is less preferred for treating the main absorption layer due to its hydrophobic properties.

Advantageously, hydrophilic and/or stain release agents can be adhered to the textile material, with the types and amounts of agents selected as described in the sections above, to accelerate the absorption of fluid in the layer, and to increase its washability. The antimicrobial agents and/or the hydrophilic agents can be applied to the textile material by the process as described above.

### Water repellant layer (fourth layer)

Water repellant layer 3 is an outer layer preventing leakage of liquids or fluids. The material suitable as water repellant layer is not subject to any restrictions and can be any textile or non-textile material known in the art for that purpose. In a preferred embodiment, water repellent layer 3 is or comprises a textile material produced using the above-described process, an example of which has already been described above as Working Example 3.

In preferred embodiments, the textile material of the water repellant layer 3 is woven and and/or a microfiber, to give stability to the sanitary napkin. At least 95%, preferably at least 97%, more preferably at least 99%, of the textile material may consist of polyester, nylon, polyamide, polypropylene, or any other synthetic textile substrate. Synthetic textile materials are preferred, because they can be welded with ultrasound, they have a higher breaking strength, and they are easier to be made water-repellent than natural textiles.

The textile material may have a weight of at least 50 GSM (grams per square meter), preferably at least 80 GSM, more preferably at least 110 GSM, and most preferably at least about 125 GSM. The textile material may have a weight of at most 250 GSM, preferably at most 200 GSM, more preferably at least 150 GSM, and most preferably at most about 125 GSM.

Advantageously, the textile material is equipped with antimicrobial agents. For example, an azole-based compound is adhered to the textile material in an amount of at least 0.1%, preferably at least 0.2%, more preferably at least 0.3%, particularly at least 0.4%, and most preferably at least about 0.5%, and/or in an amount of at most 2.0%, preferably at most 1.5%, more preferably at most 1.0%, particularly at most 0.75%, most preferably at most about 0.50% on weight fabric of the textile material; and/or a metal is adhered to the textile material in an amount of at least 0.0001%, preferably at least 0.0002%, more preferably at least 0.0005%, particularly at least 0.0010%, most preferably at least about 0.0017%, and/or in an amount of at most 0.05%, preferably at most 0.02%, more preferably at most 0.01%, particularly at most 0.005%, most preferably at most about 0.0017% on weight fabric of the textile material; and/or a quaternary ammonium organosilane compound is adhered to the textile material in an amount of at least 0.1%, preferably at least 0.2%, more preferably at least 0.3%, particularly at least 0.4%, and most preferably at least about 0.72%, and/or in an amount of at most 2.0%, preferably at most 1.5%, more preferably at most 1.3%, particularly at most 1.0%, most preferably at most about 0.72% on weight fabric of the textile material.

The textile material of the water repellent layer 3 has preferably a water repellency rating of at least 80, preferably at least 90, more preferably 100, measured in accordance with the AATCC test method 22-2014. Furthermore, the textile material of the water repellant layer should exhibit a stain/oil repellency rating, measured in accordance with AATCC test method 118-2013, of at least grade 4, preferably at least grade 5, more preferably at least grade 6, particularly at least grade 6, and most preferably at least grade 7.

Such repellency ratings can be achieved by adhering one or more water repellent agents like C6- or C8-fluorocarbon and/or polyurethane (PU) to the textile material. The total amount of the one or more water repellency agents adhered to the textile material can be at least 0.2%, preferably at least 0.5%, more preferably at least 1.0%, particularly at least 1.5%, and most preferably at least about 2.0%, and/or at most 5.0%, preferably at most 4.0%, more preferably at most 3.0%, particularly at most 2.5%, most preferably at most about 2.0% on weight fabric of the textile material.

The antimicrobial agents and/or water repellency agents can be applied to the textile material by the process as described above, in which the hydrophilic/stain release liquor application process is replaced by a water repellency liquor application process like an exhaustion or preferably a padding process, wherein the liquor comprises the one or more water repellency agents.

### Water impermeable layer (third layer)

The inventors found that even where such a woven fabric is treated with a composition as described above to make it water repellent, blood stains remain on the fabric after use which cannot easily be washed out. Furthermore, since it is difficult to make a woven fabric water impermeable, and not just water repellent, some leakage may occur during the use. Therefore, in a preferred embodiment of the sanitary napkin, a water impermeable layer 5 is placed between the dispersion layers and the outer layer. This water impermeable layer 5 may comprise a substrate such as bonded, preferably spun bonded non-woven textile fabric or paper, the substrate being coated by a water impermeable film. This water impermeable film 5 can be made of or comprise plastic, preferably polyurethane (PU). The water impermeable film can be air permeable, which increases the wearing comfort, but is more expensive. The water impermeable film can be located on the inner (upper) side of the water impermeable layer 5, with the substrate being located on the outer (lower) side of the water impermeable layer 5, or the water impermeable film can be sandwiched between two layers of substrate. In the latter case, the inner substrate layer of the sandwich may be brown so that blood stains which cannot be washed out are less visible. In exemplary embodiments of sanitary napkin, the weight of the water impermeable layer 5 is at least 20 GSM, preferably at least 30 GSM, more preferably at least 40 GSM, most preferably at least 50 GSM, and/or at most 150 GSM, preferably at most 100 GSM, more preferably at most 75 GSM, most preferably at most 50 GSM.

### Seam

The sanitary napkin can have a seam 1 which is located at one or more edges of the sanitary napkin, where one or more layers of the sanitary napkin are directly or indirectly attached together. The layers may be attached together by stitching, gluing, or preferably by ultrasonic welding. The seam 1 can have a width of at least 2 millimeters, preferably at least 4 millimeters, more preferably at least 6 millimeters, even more preferably at least 8 millimeters, most preferably at least 1 centimeter, and/or of at most 2 centimeters, preferably at most 1.5 centimeters, more preferably at most 1.3 centimeters, most preferably at most 1 centimeter.

In a preferred embodiment of the sanitary napkin, the seam 1 is located on one or preferably both longitudinal edges of the sanitary napkin, more preferably on all edges of the sanitary napkin. The layers attached together by the seam 1 can be at least the inner 2 and/or the outer layer 3, so that all layers of the sanitary napkin are held together. The water impermeable layer 5 may be among the layers attached together by the seam, in order to fix the impermeable layer 5 in place and to ensure that no fluid can trickle through to the outer layer even in the area of the seam. The one or more or preferably all main absorption layers 4 are preferably not among the layers attached together by the seam 1, in order to avoid leakage by capillarity through the seam 1 and also to ease the attachment at the level of the seam 1, especially when the seam 1 is made by ultrasonic welding.

In an exemplary embodiment, where the seam 1 is located at least on both longitudinal edges of the sanitary napkin, in order to ensure the stability of the one or more absorption layers 4, and to make the best use of the space, the width of one or more or preferably all of the absorption layers 4 is substantially equal to the respective distance between the inner edges of the seams 1 on the longitudinal sides, along at least 50%, preferably at least 75%, more preferably at least 90%, most preferably 100% of the of the length of the respective absorption layer. Substantially equal here means plus/minus 2 centimeters, preferably plus/minus 1.5 centimeters, more preferably plus/minus 1 centimeter, even more preferably plus/minus 5 millimeters, and most preferably plus/minus 3 millimeters.

When realized by stitching, the seam 1 of the sanitary napkin may not present the best protection against leaks. Indeed, the needle creates holes through which blood can leak, especially when an impermeable layer 5 as described above is used. Moreover, leaks can also result from the stitching thread conducting fluids on the outer side of the sanitary napkin. When the seam 1 of the sanitary napkin is made with glue, the seam may loosen after some wash cycles, as the glue can come out of the seam 1. Moreover, as it comes out, the glue can make the edges of the sanitary napkin abrasive. Hence, in the preferred embodiments, the seam is made by ultrasonic welding. A preferred sonotrode for such welding is described below.

In an exemplary embodiment, the parameters of the ultrasonic welding are the following:

| | |
|---|---|
| Frequency of ultrasonic | Minimum 19.8 Khz |
| | Maximum 20.15 Khz |
| Air pressure | 2-5 kg / cm² |
| Welding time | Minimum 1 sec. |
| | Maximum 2.5 sec. |

### Shape of the sanitary napkin and fixing means

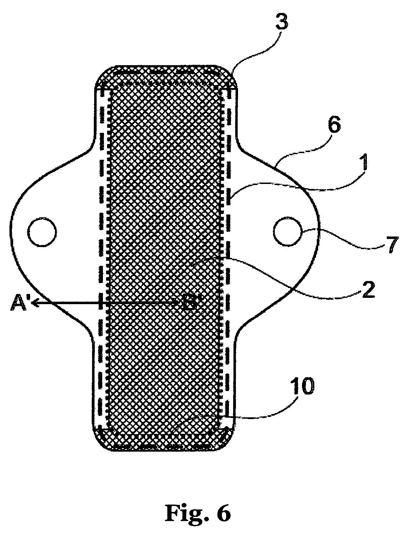

The sanitary napkin can have a functional zone 10 as delimited by the dotted line in Fig. 3 and Fig. 6., through which it absorbs fluids. In an exemplary embodiment of the sanitary napkin, the functional zone has a maximum and/or minimum width of at least 3 cm, preferably at least 4 cm, most preferably at least 6 cm, and/or a maximum and/or minimum width of at most 20, preferably at most 15 cm, more preferably at most 10 cm, most preferably at most 7.5 cm. The functional zone can have a maximum and/or minimum length of at least 10 cm, preferably at least 15 cm, more preferably at least 20 cm, most preferably at least 23 cm, and/or of at most 50 cm, preferably at most 40 cm, more preferably at most 35cm, most preferably at most 30 cm.

In a preferred embodiment of the sanitary napkin, the water repellent layer 3 is folded on one or preferably both longitudinal edges of the sanitary napkin such that the water repellent layer 3 covers the one or more dispersion layers in an area of the seam, thereby preventing leakage on the edges of the sanitary napkin, in particular through the transportation layer 2. Also the water impermeable layer 5 can be folded on one or preferably both longitudinal edges of the sanitary napkin such that the water impermeable layer covers the one or more dispersion layers in an area of the seam 1, to further improve the protection against leakage on the edges, as shown in Fig. 5.

The sanitary napkin can comprise fixing means to attach the sanitary napkin to a textile, in particular an undergarment, or to the user.

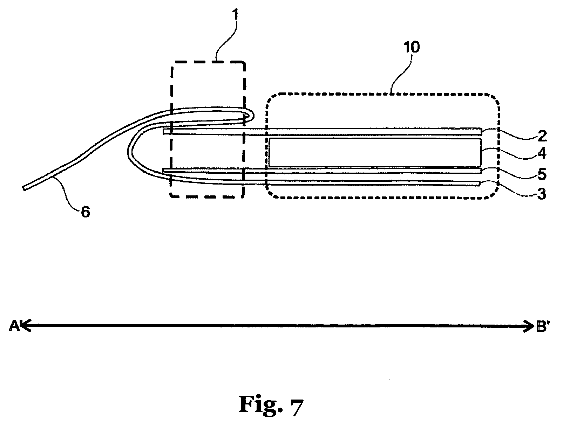

In one embodiment as shown in Fig. 6 and Fig. 7, the fixing means are wings 6 which can be wrapped around the undergarment of the user and attached to each other, preferably by a snap fastener 7. These wings 6 can be an extension of the outer layer 3, and/or can be part of the outer layer. Where the outer layer is folded on the longitudinal edges to cover the dispersion layers in an area of the seam, the wings are obtained by folding the outer layer once more and on itself, at the level of the seam 1.

This kind of fixing means is particularly suitable where the sanitary napkin is a pad. Such a pad can have a total minimum and/or maximum length of at least 10 cm, preferably at least 20 cm, more preferably at least 25 cm, most preferably at least 28 cm, and of at most 60 cm, preferably at most 40 cm, more preferably at most 35 cm, most preferably at most 31,5 cm. The minimum and/or maximum width of the pad can be of at least 4 cm, preferably at least 6 cm, more preferably at least 8 cm, most preferably at least 8,7 cm, and/or of at most 20 cm, preferably at most 12 cm, more preferably at most 9 cm, most preferably at most 8,7 cm. The fixing means (e.g. the wings) are not considered to be part of the sanitary napkin for determining the width and/or length of the sanitary napkin. The functional zone of this pad can have a minimum and/or maximum width of at least 3 cm, preferably at least 5 cm, more preferably at least 6 cm, most preferably at least 7 cm, and/or a of at most 20 cm, preferably at most 15 cm, more preferably at most 10 cm, most preferably at most 7 cm. The functional zone of this pad can have a minimum and/or maximum length of at least 10 cm, preferably at least 20 cm, more preferably at least 25 cm, most preferably at least 27 cm, and/or of at most 60 cm, preferably at most 40 cm, more preferably at most 35 cm, most preferably at most 30 cm.

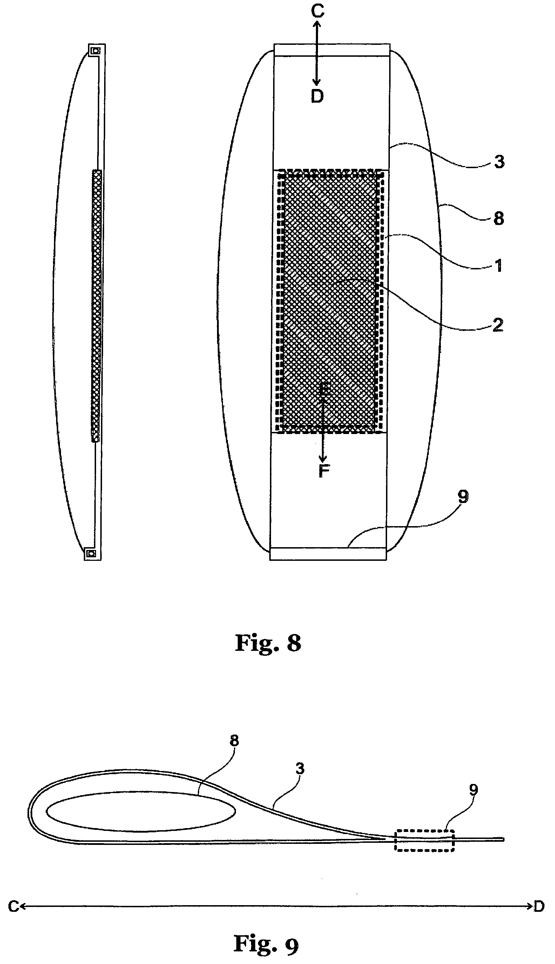

In another embodiment of the sanitary napkin, shown in Fig. 8 and 9, the fixing means are one or more ribbons 8 by which the sanitary napkin can be fixed to the user, for example by stretching them around the waist of the user. Preferably, the ribbons 8 are made of an elastic material, more preferably rubber. They can be attached at each of the opposing edges of the longitudinal ends of the sanitary napkin, preferably at an extension of the outer layer 3. Preferably, the outer layer 3 of the sanitary napkin is folded at one or more preferably both longitudinal ends, and attached to itself 9, leaving a passing through which the one or more ribbons are disposed as shown in Fig. 9.

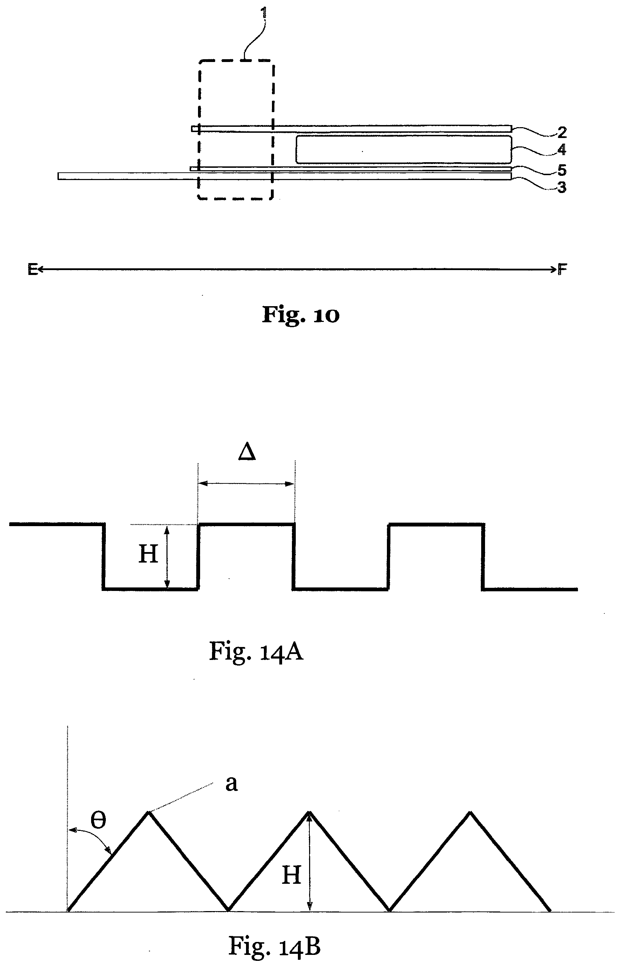

This kind of fixing means is particularly suitable for sanitary napkins as shown in Fig. 8 and Fig. 10. Such a sanitary napkin can have a minimum and/or maximum length of at least 20 cm, preferably at least 40 cm, more preferably at least 50 cm, most preferably at least 56 cm, and/or of at 100 most cm, preferably at most 80 cm, more preferably at most 70 cm, most preferably at most 61 cm. The minimum and/or maximum width of such a sanitary napkin may be of at least 4 cm, preferably at least 5 cm, more preferably at least 7 cm, most preferably at least 8 cm, and/or of at most 25 cm, preferably at most 15 cm, more preferably at most 10 cm, most preferably at most 9 cm. The fixing means (e.g. the ribbons) are not considered to be part of the sanitary napkin for determining the width and/or length of the sanitary napkin. The functional zone of such a sanitary napkin can have a minimum and/or maximum width of at least 3 cm, preferably at least 4 cm, more preferably at least 5 cm, most preferably at least 6 cm, and/or of at most 24 cm, preferably at most 14 cm, more preferably at most 9 cm, most preferably at most 7 cm. The minimum and/or maximum length of the functional zone can be of at least 10 cm, preferably at least 15 cm, more preferably at least 20 cm, most preferably at least 23 cm, and/or of at most 60 cm, preferably at most 40 cm, more preferably at most 35 cm, most preferably at most 30 cm.

### Sonotrode:

The inventors developed a sonotrode for use in the process of attaching the various layers of the sanitary napkin together at the level of the seam, by ultrasonic welding.

Such a sonotrode comprises protrusions which emit the energy of the sonotrode to the textile. The protrusions are typically arranged in repeating patterns, e.g. in lines, or preferably in grids formed by lines. In preferred embodiments of the sanitary napkin, several layers have to be welded together, which requires a high level of energy. When using prior art sonotrodes, some of the layers may melt during the binding and become brittle, resulting again in an increased risk of leaks at the level of the seam. The inventors found that this problem can be reduced or solved by reducing the distance between the lines formed by the protrusions, thereby increasing the amount of protrusions per unit of area. This distance, being calculated between the centers of the tops of two consecutive protrusions, can be of at least of 1 mm, preferably of at least 1,5 mm, more preferably of at least 1,8 mm and most preferably of at least of 2mm, and/or of at most of 4 mm, preferably of at most 3 mm, more preferably of at most 2.5 mm and most preferably of at most of 2mm.

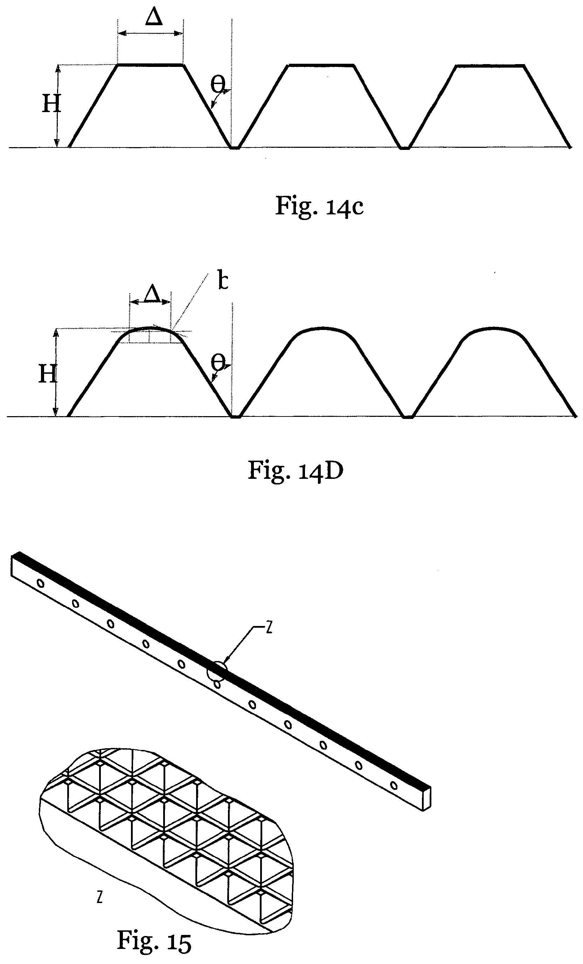

Furthermore, since the protrusions of the prior art sonotrodes have a relatively large width as shown on Fig. 14A, they cannot deliver the required amounts of energy. The attachment of a multilayer seam can thus prove difficult and result in weakness in the seam, with in an increased risk of leaks at the level of the seam. For this reason, and to get a perfect weld with the right amount of overlap, in an embodiment of the invention, one or all the sides of the protrusions form with the vertical direction an angle θ of at least 35°, preferably at least 40°, more preferably at least 45° and most preferably of at least 48.2°, and/or of at most 60°, preferably of at most 55°, more preferably of at most 50° and most preferably of at most 48.2°.

When the sonotrodes have protrusions with such side angles, as shown in Fig. 14B, the tops of the protrusion may be pointed or sharp, and even be reduced to a point such as point a. With such pointed or sharp protrusions, the ultrasonic wave is cut and does not diffuse evenly around the protrusion resulting in failures to properly attach the various layers of the sanitary napkins. Again, some of the layers may melt during the binding and become brittle. For this reasons, in a preferred embodiment of the sonotrode, the tops of the protrusions have a width Δ of at least 0.1 mm, preferably at least 0.2 mm, more preferably at least 0.25 mm and most preferably of at least 0.3 mm, and/or of at most 0.5 mm, preferably at most 0.4 mm, more preferably at most 0.35 mm and most preferably of at most 0.3 mm.

In an embodiment of the sonotrode, the top of protrusions is rounded (Fig. 14C), and the width Δ is then the distance between two points b, where the slope of the sides of the protrusion reaches an angle with the horizontal direction of at most 25°, preferably of at most 20°, more preferably at most 15° and most preferably of at most 10°. small.

In an embodiment of the sonotrode the protrusions have a height H of at most 1,5 mm, preferably at most 1,2 mm, more preferably of at most 0,9 mm, most preferably of at most 0,7 mm, and / or of at least 0,3 mm, preferably at least 0,5 mm, more preferably of at least 0,6 mm, most preferably of at least 0,7 mm.

The sonotrode can be, e.g., a flat blade (Fig. 15) or a roller, on the surface of which the protrusions are formed.

## Claims

1. A textile material, to which one or more stain release agents and one or more antimicrobial agents are adhered, wherein
the textile material exhibits a reduction value of *Escherichia coli* ATCC 25922 and/or *Staphylococcus aureus* ATCC 6538 and/or *Pseudomonas aeroginosa* ATCC 15442 and/or *Salmonella enterica* ATCC 10708 and/or *Candida albicans* ATCC 10231 and/or *Aspergillus niger* 16404 measured in accordance with AATCC test method 100-2012 and/or ASTM E2149-10 by/of at least 90% (1 log), preferably at least 99% (2 log), more preferably at least 99.9% (3 log), and most preferably at least 99.99% (4 log), within 1 hour of contact time, preferably within 15 minutes of contact time and/or of at least 99% (2 log), preferably at least 99.9% (3 log), more preferably at least 99.99% (4 log), particularly at least 99.999 (5 log);
the textile material has a stain release rating, measured in accordance with AATCC test method 130-2010, of at least grade 3, preferably at least grade 4, and most preferably grade 5;
the textile material exhibits a water absorbency time of at most 3 seconds, preferably at most 2 seconds, more preferably at most 1 second, and most preferably at most 0.5 seconds, preferably when measured in accordance with AATCC test method 79-2014 (Option A); and
the antimicrobial efficiency, the stain release rating, and the absorbency time are achieved even after at least 25 laundry washes in a laundry washing machine, preferably at 85±15 °C for 10-15 minutes, preferably using Tergitol 15-S-9 of Dow Chemicals, non-antimicrobial, non-ionic and non-chlorine containing laundry detergent, preferably followed by a standard rinse cycle and preferably dried at 62-96 °C for 20-30 minutes.

2. The textile material of any claim 1, wherein the one or more antimicrobial agents comprise
- at least one, preferably at least two, more preferably at least three, even more preferably at least four, or all five selected from the group consisting of a quaternary ammonium organosilane compound, metal, an azole-based compound, polyglucosamine, and polyhexamethylene biguanide; or
- at least one, preferably at least two, more preferably at least three, and most preferably all four selected from the group consisting of metal, an azole-based compound, polyglucosamine, and polyhexamethylene biguanide; or
- at least one, preferably at least two, more preferably all three selected from the group consisting of metal, an azole-based compound, and a quaternary ammonium organosilane compound; or
- at least metal and at least one selected from the group consisting of an azole-based compound, a quaternary ammonium organosilane compound, polyglucosamine, and polyhexamethylene biguanide; or at least an azole-based compound and at least one selected from the group consisting of a quaternary ammonium organosilane compound, polyglucosamine, and polyhexamethylene biguanide; or at least a quaternary ammonium organosilane compound and at least one selected from the group consisting of polyglucosamine and polyhexamethylene biguanide; or at least polyglucosamine and polyhexamethylene biguanide.

3. The textile material of any one of the preceding claims, wherein the one or more stain release agents adhered to the textile material comprise at least one selected from the group consisting of fatty alcohol ethoxylates, and organosilane terpolymers, preferably organosilane terpolymers.

4. The textile material of any one of the preceding claims, obtainable by a process comprising the steps of:
- treating the textile material using an antimicrobial liquor application process like an exhaustion or padding process, wherein the liquor comprises one or more antimicrobial agents;
- drying the textile material;
- treating the textile material using a hydrophilic/stain release liquor application process like an exhaustion or preferably a padding process, wherein the liquor comprises one or more hydrophilic agents and/or stain release agents; and
- subjecting the treated textile material to a heat treatment.

5. The textile material of claim 4, wherein the heat treatment comprises curing of the textile material, wherein the curing is conducted at least partially at a curing ambient temperature of at least 160 °C, more preferably at least 170 °C, particularly at least 175 °C, and most preferably at least about 180 °C.

6. A sanitary napkin comprising one or more dispersion layers comprising or consisting of textile material according to any one of claim 1 to 5, and
a water repellent layer, having a water repellency rating of at least 70, preferably at least 80, more preferably at least 90, most preferably 100, measured in accordance with AATCC test method 22-2014.

7. The sanitary napkin of any one of claim 6, wherein a water impermeable layer is placed between the dispersion layers and the outer layer of the sanitary napkin.

8. The sanitary napkin of 185a, wherein the water impermeable layer comprises a substrate such as bonded, preferably spun bonded non-woven textile fabric or paper, the substrate being coated by a water impermeable film.

9. The sanitary napkin of claim 193ab, wherein the sanitary napkin has a seam which is located at one or more edges of the sanitary napkin, where one or more layers of the sanitary napkin are directly or indirectly attached together by ultrasonic welding.

10. The sanitary napkin of claim 9, when dependent from claim 7, wherein the water impermeable layer is among the layers attached together by the seam.

11. The sanitary napkin of any one of claims 9 or 10, wherein the one or more dispersion layers comprise one or more main absorption layers which are not among the layers attached together by the seam.

12. The sanitary napkin of any of the claims 9 to 11, wherein the water repellent layer is folded on one or preferably both longitudinal edges of the sanitary napkin such that the water repellent layer covers the one or more dispersion layers in an area of the seam.

13. The sanitary napkin of any one of claims 6 to 12, wherein the sanitary napkin comprises wings which can be attached to each other, preferably by a snap fastener, to attach the sanitary napkin to a textile, in particular an undergarment, and the wings are an extension of the outer layer.

14. The sanitary napkin of claim 13, wherein wings are part of the outer layer, and obtained by folding the outer layer on itself at least twice at the level of the seam.

15. The sanitary napkin of any one of claims 6 to 12, wherein the sanitary napkin comprises one or more ribbons for fixing the sanitary napkin to the waist of the user, which are attached at the edge of at least one or preferably both longitudinal ends of the sanitary napkin, preferably at the outer layer thereof.
